(19) Europäisches Patentamt / European Patent Office / Office européen des brevets

(11) **EP 2 857 844 B1**

(12) **EUROPÄISCHE PATENTSCHRIFT**

(45) Veröffentlichungstag und Bekanntmachung des Hinweises auf die Patenterteilung:
**01.12.2021  Patentblatt 2021/48**

(51) Int Cl.:
***G01N 35/00*** *(2006.01)*

(21) Anmeldenummer: **13004814.3**

(22) Anmeldetag: **07.10.2013**

(54) **Laborgerät, System und Verfahren zur gerätegesteuerten Behandlung mindestens einer Laborprobe unter Verwendung mindestens eines Verbrauchsartikels**

Laboratory device, system and method for device-controlled treatment of at least one laboratory sample using at least one consumable item

Appareil de laboratoire, système et procédé de traitement commandé par un appareil d'au moins un échantillon de laboratoire en utilisant au moins un article de consommation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**08.04.2015  Patentblatt 2015/15**

(73) Patentinhaber: **Eppendorf AG**
**22339 Hamburg (DE)**

(72) Erfinder:
  • **Goemann-Thoss, Wolfgang**
    **22339 Hamburg (DE)**
  • **Wente, Wolf**
    **22339 Hamburg (DE)**
  • **Thieme, Andreas**
    **22339 Hamburg (DE)**
  • **Frerichs, Jan-Gerd**
    **22339 Hamburg (DE)**
  • **Markau, Christiane**
    **22339 Hamburg (DE)**
  • **Hacker, Jan-Hendrik**
    **22339 Hamburg (DE)**

(74) Vertreter: **Ricker, Mathias**
**Wallinger Ricker Schlotter Tostmann**
**Patent- und Rechtsanwälte Partnerschaft mbB**
**Zweibrückenstrasse 5-7**
**80331 München (DE)**

(56) Entgegenhaltungen:
JP-A- 2006 164 295      US-A1- 2005 014 285
US-A1- 2007 255 756      US-A1- 2011 246 215
US-A1- 2013 159 135

• **MICHAEL FRANKLIN ET AL: "Data in your face", SIGMOD RECORD, ACM, NEW YORK, NY, US, vol. 27, no. 2, 1 June 1998 (1998-06-01), pages 516-519, XP058358456, ISSN: 0163-5808, DOI: 10.1145/276305.276360**
• **Anonymous: "Push Architecture", , 9 August 2011 (2011-08-09), pages 1-3, XP055742889, Retrieved from the Internet: URL:https://www.openmobilealliance.org/release/Push/V2_2-20110809-A/OMA-AD-Push-V2_2 -20110809-A.pdf [retrieved on 2020-10-22]**
• **Anonymous: "Push Technology", , 14 January 2020 (2020-01-14), XP055742892, Retrieved from the Internet: URL:https://www.techopedia.com/definition/5732/push-technology [retrieved on 2020-10-22]**

**Beschreibung**

Die Erfindung bezieht sich auf ein Laborgerät, ein System und ein Verfahren zur gerätegesteuerten Behandlung mindestens einer Laborprobe unter Verwendung mindestens eines Verbrauchsartikels.

Laborgeräte werden verwendet, um in chemischen, biologischen, biochemischen, medizinischen oder forensischen Laboratorien Laborproben, insbesondere flüssige Laborproben, mit hoher Effizienz zu bearbeiten. Solche Laborgeräte automatisieren Behandlungsschritte zumindest teilweise, die sonst manuell durchgeführt werden müssten, und steigern auf diese Weise die Geschwindigkeit, Präzision und Zuverlässigkeit dieser Behandlungen. Eine Behandlung von zumeist flüssigen Laborproben kann darauf gerichtet sein, diese Laborproben, insbesondere deren Zusammensetzung, physikalisch, chemisch, biochemisch oder auf andere Weise zu ändern oder zu untersuchen.

Die genannten Laborgeräte weisen eine oder mehrere Behandlungseinrichtungen zur gerätegesteuerten Behandlung der mindestens einen Laborprobe unter Verwendung mindestens eines Verbrauchsartikels auf. Sie weisen oft eine Programmsteuerung auf, mittels der ein Benutzer des Laborgeräts die durchzuführende Behandlung durch Einstellen der gewünschten Programmparameter festlegen kann und dadurch den Ablauf einer Behandlung beeinflussen kann. Zum Erreichen des Behandlungserfolgs gibt es oft mehrere Wege. Es können unterschiedliche Planungen bzw. Abläufe, die z.B. durch unterschiedliche Benutzer vorgenommen werden, zu denselben Ergebnissen, also zu derselben Behandlung führen. Insbesondere kann je nach Planung eine unterschiedliche Menge eines oder mehrerer Verbrauchsartikel zum Einsatz kommen. Solche Verbrauchsartikel können Probenbehältnisse sein, Transportbehältnisse, wie z.B. Kapillaren, Pipettenspitzen oder Dispenserspitzen, Reinigungsmittel, usw. Die Einstellung der Programmparameter erfolgt über eine Bedieneinheit des Laborgeräts, welche die Ein- und Ausgabe von Informationen, insbesondere von Werten der Programmparameter ermöglicht. Die Behandlung wird zumindest teilweise automatisiert durchgeführt. Nach Ablauf der Behandlung kann der Benutzer die behandelte Probe weiterverwenden und das Laborgerät steht für eine weitere Verwendung zur Verfügung. Der nachfolgende Benutzer, der die nächste Behandlung durchführt, wird das Laborgerät in ähnlicher Weise verwenden. Die für die Durchführung erforderlichen Verbrauchsartikel, insbesondere Probenbehälter oder Transportbehältnisse werden meist vom Bedienpersonal manuell an die dazu vorgesehenen Vorratspositionen im Labor oder am Laborgerät verteilt. Eine Störung des Arbeitsflusses in einem Labor entsteht regelmäßig dann, wenn die zur Auffüllung der Vorratspositionen erforderliche Menge an Verbrauchsartikel unerwartet im Labor nicht mehr verfügbar ist. Oft wird in Laboren zusätzliches Bedienpersonal eingesetzt, um die Grundversorgung mit Verbrauchsartikeln zu verantworten, die bei der Arbeit mit den Laborgeräten verbraucht werden. Über derartige Strategien hinaus beschreitet die vorliegende Erfindung einen anderen Weg zur Verbesserung der Situation.

Das Dokument US 2011/246215 beschreibt ein Nachschubzentrum für Verbrauchsmaterial verwendende medizinische Geräte, insbesondere Analysatoren, mit einem Empfänger zum Empfang um auf Verbrauschmaterial bezogene Betriebsinformationen von den Analysatorsysteme zu empfangen, mit einer Datenbank, um diese Betriebsinformationen aufzuzeichnen, einen Bestandsrechner um eine verbleibende Bestandsmenge des Verbrauchsmaterials zu berechnen und in der Datenbank zu speichern, und einen Auftragsgenerator, um dem Benutzer eines Analysators in Abhängigkeit von der Bestandsmenge eine Bestellung vorzuschlagen.

Das Dokument US 2013/159135 beschreibt ein Verfahren zur automatisierten Laborinventarverwaltung, um eine Bestellung für einen Laborartikel zu generieren, die Bestellung an einen Remotecomputer zu senden, um die Bestellung an einen Lieferanten zu übermitteln, und um eine als Antwort auf die Bestellung generierte Versandbenachrichtigung zu erhalten, um ferner Artikelinformationen zu empfangen, die in einem RFID-Tag eines markierten Artikels gespeichert sind, der am Lieferort eingegangen ist, und die Artikelinformationen anhand der Versandvorankündigung zu überprüfen, um sicherzustellen, dass der markierte Artikel mit dem bestellten Laborartikel übereinstimmt.

Das Dokument US 2005/014285 beschreibt ein Verfahren zum automatischen Verarbeiten der biologischen Flüssigkeiten eines Patienten und zur erneuten Zufuhr von Reagenzien, die an Bord eines automatischen klinischen Analysegeräts inventarisiert wurden, indem ein gemitteltes Assay-Anforderungsmuster auf dem Analysegerät für Assays über eine Sequenz spezifisch definierter Zeiträume mit den an Bord des Analysegeräts inventarisierten Reagenzien verglichen wird, um zu bestimmen, welche Reagenzien erschöpft sind vor dem nächsten nach der Abfolge spezifisch definierter Zeiträume und entsprechendem Nachfüllen der Reagenzien.

Das Dokument JP 2006/164295 beschreibt eine Betriebsverwaltungsmethode für ein Analysesystem, das in der Lage ist, den Betriebszustand einer Analyse eines Benutzerziels ohne manuelle Hilfe zu erfassen und einem Benutzer Feedbackinformationen zu geben, indem dieses Analysesystem mit einem Analysegerät an ein Informationsterminal angeschlossen ist, um den Betrieb des Analysesystems zu verwalten, wobei Daten zur Anzahl der Tests für jedes Analyseelement angezeigt werden, wobei Reagenzien sowie die Betriebszeit des Analysators vom Analysator empfangen und gespeichert werden, wobei die jeweils gespeicherten Daten für jedes Analyseelement akkumuliert werden, und eine Betriebseffizienz für jedes Analyseelement auf der Grundlage des Akkumulationsergebnisses berechnet wird, um die Betriebseffizienz des Analysesystems maximal aufrechtzuerhalten.

Aufgabe der vorliegenden Erfindung ist es, ein Laborgerät, ein System und ein Verfahren zur gerätegesteuerten Behandlung mindestens einer Laborprobe unter Verwendung mindestens eines Verbrauchsartikels bereitzustellen, mit dem sich der Arbeitsfluss in einem Labor verbessern lässt.

Die Erfindung löst diese Aufgabe insbesondere durch das Laborgerät gemäß Anspruch 1, das System gemäß Anspruch **8** und das Verfahren gemäß Anspruch **11.** Bevorzugte Ausgestaltungen sind insbesondere Gegenstände der Unteransprüche.

Die Erfindung sieht vor, die Informationen über die Verbrauchsartikel, insbesondere deren Verwendung und/oder Verbrauch, bereits bei der Verwendung der Verbrauchsartikel automatisch zu erfassen und die entsprechenden Daten als erste Daten für die Weiterbearbeitung bei einer weiteren Datenverarbeitungseinrichtung zur Verfügung zu stellen. Diese weitere Datenverarbeitungseinrichtung kann Bestandteil des erfindungsgemäßen Systems sein oder kann insbesondere eine entfernt angeordnete Datenverarbeitungseinrichtung sein, die insbesondere von der Kommunikationseinrichtung des Laborgeräts z.B. über eine das Internet nutzende Datenfernverbindung kontaktiert wird. Bei einem System mit einem Verbund aus mehreren solcher Laborgeräte können Verbrauchsdaten zentral gesammelt werden, z.B. auf einem Server. Durch diese erfindungsgemäßen Lösungen lassen sich Verbrauchsdaten gewinnen, mit denen sich gemäß einer bevorzugten Ausgestaltung der Erfindung ein automatisches Bestell- und/oder Nachschubsystem für Verbrauchsartikel bereitstellen lässt.

Die **"ersten Daten"** können Informationen über die Menge und/oder die Anzahl der Verbrauchsartikel sein, die bei der durchgeführten Behandlung verbraucht werden oder die nach einer bestimmten Zeit oder in einem bestimmten Zeitraum, insbesondere nach Abschluss der Behandlung, verbraucht wurden. Die ersten Daten enthalten die Mengenangabe vorzugsweise jeweils mit Bezug auf die Art des verbrauchten Verbrauchsartikels. Durch die Bezeichnung der Art des Verbrauchsartikels soll vorzugsweise eindeutig die Information gegeben sein, mit der sich der Verbrauchsartikel wiederbeschaffen lässt.

Die ersten Daten enthalten die Mengenangabe vorzugsweise jeweils benutzerabhängig. Insbesondere in diesem Fall ist es bevorzugt, dass das Laborgerät, oder das System, eine Zugriffssteuerung aufweist, mit der ein Benutzer identifizierbar ist.

Ein **Verbrauchsartikel** ist ein Artikel, der nach einmaligem Gebrauch entsorgt werden muss, da entweder die Reinigung und Wiederverwertbarkeit nicht möglich ist oder nicht ökonomisch wäre, oder weil der Verbrauchsartikel nach Gebrauch in einer Behandlung zur Verwendung bei einer weiteren Behandlung unbrauchbar geworden ist. Ein Verbrauchsartikel kann aus Kunststoff gefertigt sein oder Kunststoff aufweisen. Ein Verbrauchsartikel ist vorzugsweise ein Probenbehältnis, auch bezeichnet als Probenbehälter Ein Probenbehältnis kann ein Einzelbehältnis sein, z.B. ein Probenröhrchen, beispielsweise verschieden volumige PCR Gefäße (0,2 mL oder 0,5 mL), verschieden volumige Eppendorf Gefäße (1,5 mL, 0,5 mL, 2 ml; 5 mL), verschieden volumige Falcon®-Tubes oder eine Probenplatte, oder ein Mehrfachbehältnis, z.B. eine Mikrotiterplatte (PCR Platte) PCR-Tube Strips, Zellkultur-Mikroplatte oder Zellkultur-Multiwellplatten oder auch eine Aufbewahrungsbox (Karton) für Probenröhrchen, die in Tiefkühlgeräten üblich sind. Ein Verbrauchsartikel ist vorzugsweise ein Transportbehältnis, auch bezeichnet als Transportbehälter. Ein Transportbehältnis kann z.B. eine Kapillare, Küvette, Pipettenspitze oder Dispenserspitze sein. Ein Verbrauchsartikel ist auch ein Einwegbehälter eines Bioreaktors (Fermentors), der auch in verschiedenen Volumina üblich ist. Ein Verbrauchsartikel kann auch ein Verschluss für ein Einzelbehältnis oder Mehrfachbehältnis sein, beispielsweise eine Verschlussmatte, Deckel für eine Platte (Mikrotiter, PCR; Zellkultur- Mikro - und Multiwellplatten) oder Cap Strips (verbundene Kappen für PCR Tube Strips). Verbrauchsartikel werden verpackt gelagert, wobei eine Mehrzahl ($\leq$ 10) bzw. Vielzahl (> 10) an Verbrauchsartikeln in einer einzigen Verpackung (vorbestimmte Menge) enthalten sind. Da im üblichen Laborbetrieb verschiedene Typen Verbrauchsartikeln eingesetzt werden, müssen alle diese immer vorrätig gehalten werden. Diese Bevorratung von verschiedenen Verbrauchsartikeln erfordert daher einen hohen Raumbedarf, da ein Verbrauchsartikel nicht in der Einzahl sondern üblicherweise in der Mehrzahl oder Vielzahl gelagert werden muss.

Die Steuereinrichtung des Laborgeräts ist vorzugsweise dazu eingerichtet, bei mindestens einer durch eine Behandlungseinrichtung durchgeführten Behandlung mindestens eine erste, von dieser Behandlung abhängige Information in ersten Daten zu erfassen, insbesondere durch einen Zähl-, Mess- oder Berechnungsvorgang. Im Falle eines Pipettiervorgangs mittels einer oder mehrerer Pipettenspitzen, Dispenserspitzen oder Kapillaren, die an einem Pipettier-Werkzeugkopf eines Laborgeräts, insbesondere Laborautomaten, befestigt sein können, kann insbesondere jeder Abwurf dieser Verbrauchsartikel erfasst, gezählt und/oder die entsprechende Zahl gespeichert werden. Ferner kann das Laborgerät einen Sensor aufweisen, mit dem ein Abnutzungswert eines Verbrauchsartikels gemessen werden kann. Bei einer transparenten Küvette könnte als Abnutzungswert insbesondere die Transmission durch die leere Küvette gemessen werden. Falls für einen Verbrauchsartikel ein Erfahrungswert für die Anzahl der maximal möglichen Verwendungen des Verbrauchsartikels besteht, kann, insbesondere als Abnutzungswert, die Anzahl der Verwendungen erfasst werden.

Die mittels der Kommunikationseinrichtung herstellbare Datenverbindung kann insbesondere eine Datenfernverbindung sein, die insbesondere über ein weltweites Netzwerk, insbesondere über das Internet hergestellt wird. Die Datenverarbeitungseinrichtung kann eine externe Datenverarbeitungseinrichtung sein, die insbesondere außerhalb des Labors angeordnet ist, in dem das Laborgerät positioniert ist.

Eine -insbesondere externe- **Datenverarbeitungseinrichtung** kann insbesondere ein Computer oder Mikroprozessor sein oder kann einen Computer oder Mikroprozessor aufweisen. Die Datenverarbeitungseinrichtung kann insbesondere ein Server sein. Ein Server ist insbesondere ein Computer, dessen Hardware vorzugsweise auf Serveranwendungen abgestimmt ist. Vorzugsweise weist das erfindungsgemäße System mindestens eine Datenverarbeitungseinrichtung auf, die auch als systemzugehörige externe Datenverarbeitungseinrichtung bezeichnet wird. Diese ist insbesondere nicht Bestandteil eines Laborgeräts.

Eine -insbesondere externe- Datenverarbeitungseinrichtung weist jeweils vorzugsweise auf: mindestens eine Speichereinrichtung zum flüchtigen oder dauerhaften Speichern von Daten; mindestens eine Steuereinrichtung; mindestens eine Kommunikationseinrichtung; mindestens eine Schnittstelleneinrichtung.

Die Steuereinrichtung ist insbesondere eine elektronische Steuereinrichtung. Eine **Steuereinrichtung** weist im Rahmen der vorliegenden Erfindung generell insbesondere eine Datenverarbeitungseinrichtung, insbesondere eine Recheneinheit (CPU) zum Verarbeiten von Daten und/oder einen Mikroprozessor auf. Eine Recheneinheit ist vorzugsweise auch zum Steuern der externen Datenverarbeitungseinrichtung und/oder des Behandlungsprozesses und/oder der individuellen Behandlungen eingerichtet.

Eine **Kommunikationseinrichtung** ist vorzugsweise eingerichtet für das Senden und/oder Empfangen von Daten, insbesondere den Datenaustausch, über eine durch die Kommunikationseinrichtung bereitgestellte Datenverbindung, insbesondere eine Datenfernverbindung mit einem entfernten Gerät. Insbesondere wird das bezüglich eines Laborgeräts entfernt angeordnete Gerät auch bezeichnet als "Remote-Gerät" oder externes Gerät. Insbesondere wird eine Datenverarbeitungseinrichtung, die nicht Bestandteil eine Laborgeräts ist, auch als externe Datenverarbeitungseinrichtung bezeichnet. Die Datenverbindung, insbesondere Datenfernverbindung, kann über ein beschränktes (insbesondere ein Intranet) oder weltweites (insbesondere das Internet) Netzwerk aus Computern aufgebaut werden. Die Datenverbindung, insbesondere Datenfernverbindung, kann auch über eine Funkverbindung aufgebaut werden. Die Datenverbindung, insbesondere Datenfernverbindung, kann insbesondere über eine Mobilfunkverbindung aufgebaut werden.

Eine -insbesondere externe- Datenverarbeitungseinrichtung weist vorzugsweise mindestens eine Speichereinrichtung auf, in der erste und/oder zweite Daten speicherbar sind.

Das Laborgerät, insbesondere das System, weist vorzugsweise mindestens eine Benutzerschnittstelleneinrichtung auf.

Vorzugsweise ist die Datenverarbeitungseinrichtung des Laborgeräts oder des Systems ein Computer oder Mikroprozessor oder weist einen Computer oder Mikroprozessor auf. Die Datenverarbeitungseinrichtung kann ein Server sein. Die Datenverarbeitungseinrichtung kann eine **Reservierungsdatenbank** aufweisen. Diese kann einen Kalender in Form von Kalenderdaten aufweisen, in dem Einträge der zeitlichen Nutzung eines oder mehrerer Laborgeräten mit einer bestimmten zeitlichen Genauigkeit eingetragen werden können, z.B. mit minutengenauer Belegung. Vorzugsweise ist das System dazu eingerichtet, die Verwendung eines, mehrerer oder aller Laborgeräte des Systems mittels der Reservierungsdatenbank zu verwalten. Die Einträge in der Reservierungsdatenbank können demnach einerseits durch den Computer erfolgt sein oder andererseits durch einen oder mehrere Benutzer eingetragen worden sein.

Vorzugsweise weist ein Laborgerät, oder das System, insbesondere eine **Planungseinrichtung** zur Planung eines zu erwartenden Verbrauchs an Verbrauchsartikeln auf. Die Planungseinrichtung ist vorzugsweise durch die Datenverarbeitungseinrichtung des Systems oder des Laborgeräts realisiert. Dazu ist vorzugsweise ein System, oder ein Laborgerät, insbesondere eine - insbesondere systemzugehörige externe- Datenverarbeitungseinrichtung, dazu eingerichtet, unter Berücksichtigung der in der Reservierungsdatenbank gespeicherten Reservierungsdaten, insbesondere der enthaltenen Information(en) über die zeitliche Reservierung eines oder mehrerer Laborgeräte, eine Menge an Verbrauchsartikeln zu bestimmen. Dabei kann insbesondere die bereits geplante Behandlung, insbesondere die Art der Behandlung berücksichtigt werden, für die das Gerät reserviert wurde. Der Verbrauch kann insbesondere für jede mögliche Art der Behandlung an einem Laborgerät verschieden sein. Vorzugsweise ist das Laborgerät oder das System dazu eingerichtet, den Reservierungsdaten für ein Laborgerät pro Zeitraum und/oder pro Behandlung eine Menge an Verbrauchsmaterial eindeutig zuzuordnen oder abzuschätzen. Diese Menge an Verbrauchsartikeln kann berechnet sein oder aus Tabellen abgeleitet sein, in denen die den Berechnungen entsprechenden Werte abgelegt sind. Die ermittelte Menge an Verbrauchsartikeln kann mit einer oder mehreren Vorratszahlen verglichen werden, die die Menge an jeweils bevorrateten Verbrauchsartikeln repräsentieren. Daraus kann das Laborgerät oder das System ermitteln, welcher zusätzliche Bedarf an Verbrauchsartikeln für den betrachteten Zeitraum besteht. Der Bedarf an Verbrauchsartikeln kann als "erste Daten" über eine Datenfernverbindung an eine externe Datenverarbeitungseinrichtung übersandt werden. Die externe Datenverarbeitungseinrichtung kann einem Anbieter für die Verbrauchsartikel zugeordnet sein. Der Anbieter kann anhand der ersten Daten eine Lieferung oder ein Angebot für die Menge an benötigtem Verbrauchsmaterial planen. Dadurch wird der Arbeitsfluss in einem Labor weiter verbessert.

Vorzugsweise ist die Planungseinrichtung dazu ausgebildet, Planungsdaten zu speichern und auszuwerten, mittels deren Verwendung die Planung eines zu erwartenden Verbrauchs an Verbrauchsartikeln durchgeführt werden kann. Vorzugsweise ist die Planungseinrichtung dazu ausgebildet, Informationen über durchgeführte Behandlung als erste Daten zu sammeln, und insbesondere mindestens eine statistische Größe in Abhängigkeit von den ersten Daten zu ermitteln, insbesondere zu berechnen. Diese statistische Größe kann eine Summe der verbrauchten Mengen mindestens eines Verbrauchsartikels sein, kann ein Durchschnittswert sein, kann insbesondere eine solche Summe bezogen auf eine Art der Behandlung, bezogen auf ein Laborgerät oder eine bestimmte Art von Laborgerät, bezogen auf ein erfindungsgemäßes System, bezogen auf einen Benutzer, bezogen auf einen Zeitraum, insbesondere abhängig von Wochentagen, Arbeitstagen, und/oder Feiertagen und/oder bezogen auf mindestens eine weitere Bezugsgröße oder Information sein.

Vorzugsweise ist die Planungseinrichtung dazu ausgebildet, Planungsdaten zu speichern und auszuwerten, mittels deren Verwendung die Planung eines zu erwartenden Verbrauchs an Verbrauchsartikeln unter zusätzlicher Berücksichtigung von Reservierungsdaten erfolgen kann. Durch Verwendung einer statistischen Größe, die in Abhängigkeit von einer Bezugsgröße ermittelt wurde, kann in Kombination mit Reservierungsdaten, die ebenfalls in Abhängigkeit von der Bezugsgröße stehen, ein zu erwartender Verbrauch an Verbrauchsartikeln ermittelt werden. Es ist z.B. möglich, den aus Planungsdaten bekannten Durchschnittsverbrauch für eine bestimmte Behandlung, z.B. eine Probenverdünnungsreihe bei mindestens einem Laborautomaten, mit den Reservierungsdaten zu kombinieren, welche die Reservierung des mindestens einen Laborautomaten für einen bestimmten Zeitraum angeben, z.B. eine Woche. Die Planungseinrichtung kann daraus den erwarteten Verbrauch der mit dieser Art der Behandlung assoziierten Verbrauchsartikel für eine Woche ermitteln, und kann diesen Wert als "erste Daten" bereitstellen, insbesondere an eine externe Datenverarbeitungseinrichtung übersenden. In einem anderen Beispiel kann die Planungseinrichtung für einen bestimmten Benutzer die erwarteten Verbrauchswerte mit Bezug auf einen zukünftigen Zeitraum ermitteln, indem Planungsdaten verwendet werden, die historische, gespeicherte Angaben über verbrauchte Mengen an Verbrauchsartikeln durch Behandlungen des bestimmten Benutzers enthalten, vorzugsweise in Abhängigkeit von der Art der Behandlung. Der zukünftige Zeitraum kann auf Reservierungsdaten bezogen sein, also in Abhängigkeit von tatsächlich von einem bestimmten Benutzer geplanten Behandlungen erfolgen. Die Planungseinrichtung kann aber auch aus statistischen, historischen Verbrauchswerten dieses Benutzers einen Erwartungswert für den Verbrauch an Verbrauchsartikeln dieses Benutzers in der Zukunft ermitteln, insbesondere ohne Reservierungsdaten zu berücksichtigen.

Vorzugsweise ist die Planungseinrichtung dazu eingerichtet, die von einem oder mehreren Laborgeräten bereitgestellten ersten Daten zu sammeln, insbesondere zu speichern, die insbesondere bei einer oder mehreren Behandlungen von Laborproben von dem einen oder den mehreren Laborgeräten erfasst wurden.

Vorzugsweise ist die ist die Planungseinrichtung dazu eingerichtet, aus den gesammelten ersten Daten mindestens eine erste Menge X eines Verbrauchsmaterials Y zu bestimmen. Die Planungseinrichtung ist vorzugsweise dazu eingerichtet, dritte Daten zu speichern, die vorbestimmte Informationen über eine zweite Menge, diese Menge bezeichnet mit "N", des Verbrauchsmaterials Y zu speichern. Vorzugsweise ist die Planungseinrichtung dazu eingerichtet, die Menge X mit der Menge N zu vergleichen, insbesondere, um einen Bedarf an Verbrauchsartikeln zu bestimmen. Vorzugsweise ist die Kommunikationseinrichtung dazu eingerichtet, die Information über die gespeicherte Menge "N" als diese wenigstens einigen ersten Daten an die - insbesondere zweite- externe Datenverarbeitungseinrichtung zu übermitteln, insbesondere wenn der Vergleich der Menge X mit der Menge N ergibt, dass für den Verbrauchsartikel "Y" ein Bedarf der Menge "N" besteht, insbesondere wenn X >= N ist oder wenn bestimmt wird, dass zu einem bestimmten Zeitpunkt an X >= N zu erwarten ist. Dabei kann insbesondere auch der gemäß einem Reservierungszeitplan zu erwartende Verbrauch des Verbrauchsartikels "Y" berücksichtigt werden, wie bereits beschrieben. Die genannte bevorzugte Gestaltung der Planungseinrichtung ist besonders vorteilhaft, wenn Verbrauchsartikel in vorbestimmten Mengen zur Verwendung mit dem Laborgerät oder dem System gelagert werden und/oder zur Verwendung mit dem Laborgerät oder dem System bestellbar sind, wobei diese vorbestimmte Menge insbesondere der Menge N entspricht, die in der Planungseinrichtung gespeichert sind.

Vorzugsweise ist eine Datenverarbeitungseinrichtung des Laborgeräts dazu eingerichtet, Benutzerdaten zu empfangen, die ein Benutzer, insbesondere mithilfe einer externen Datenverarbeitungseinrichtung, insbesondere über eine Kommunikationseinrichtung dieser externen Datenverarbeitungseinrichtung oder über eine Schnittstelleneinrichtung dieser externen Datenverarbeitungseinrichtung an die empfangende Datenverarbeitungseinrichtung des Laborgeräts überträgt.

Es ist bevorzugt, dass das Laborgerät die bereitgestellten und/oder erzeugten ersten und/oder zweiten Daten, an die -insbesondere externe- Datenverarbeitungseinrichtung, insbesondere den Server, **direkt** überträgt, also über eine direkte drahtlose oder drahtgebundene Datenverbindung, insbesondere Datenfernverbindung, an die - insbesondere externe- Datenverarbeitungseinrichtung, insbesondere den Server überträgt. Es ist auch bevorzugt, dass das Laborgerät die bereitgestellten und/oder erzeugten ersten Daten **indirekt,** also mittelbar, an die -insbesondere externe- Datenverarbeitungseinrichtung, insbesondere den Server, überträgt, also über eine mittelbare drahtlose oder drahtgebundene Datenverbindung, insbesondere Datenfernverbindung, an die -insbesondere externe- Datenverarbeitungseinrichtung, insbesondere den Server, überträgt. Dabei kann mindestens eine weitere Datenverarbeitungseinrichtung zwischengeschaltet sein, das die ersten und/oder zweiten Daten empfängt und weitersendet. Diese weitere Datenverarbeitungseinrichtung kann ein Laborgerät bzw. dessen Steuereinrichtung sein, oder eine andere Datenverarbeitungseinrichtung, insbesondere Server.

Die externe Datenverarbeitungseinrichtung kann dazu eingerichtet sein, aus den empfangenen ersten Daten die zweiten Daten zu erzeugen. Das erfindungsgemäße System, oder ein erfindungsgemäßes Laborgerät, und/oder vorzugsweise die externe Datenverarbeitungseinrichtung kann insbesondere dazu eingerichtet sein, einen Bestellvorgang für Verbrauchsartikel in Abhängigkeit von den ersten Daten zu beginnen. Dazu kann die externe Datenverarbeitungseinrichtung zur Bestätigung des Empfangs der ersten Daten mindestens zweite Daten vorzugsweise an das erfindungsgemäße System, oder Laborgerät senden, welche die ersten Daten übersandt hat und welches nun diese zweiten Daten empfängt.

Jeder Benutzer kann über dieselbe **Benutzerschnittstelleneinrichtung** in eine erste Datenverbindung mit der Zugriffssteuerung treten, oder mehrere Benutzer können über unterschiedliche Benutzerschnittstelleneinrichtungen in eine erste Datenverbindung mit der Zugriffssteuerung treten. Eine Benutzerschnittstelleneinrichtung kann Bestandteil der Zugriffssteuerung sein. Eine Zugriffssteuerung kann Bestandteil der Benutzerschnittstelleneinrichtung sein. Eine Benutzerschnittstelleneinrichtung kann Bestandteil eines Laborgeräts sein. Eine Benutzerschnittstelleneinrichtung weist jeweils vorzugsweise auf: eine Steuereinrichtung für eine Benutzerschnittstelleneinrichtung; eine Kommunikationseinrichtung zur Herstellung einer Datenverbindung mit einem Laborgerät über eine Schnittstelleneinrichtung desselben; eine Eingabeeinrichtung zur Erfassung von Benutzereingaben eines Benutzers; eine Ausgabeeinrichtung, insbesondere eine Anzeige und/oder ein Display, zur Ausgabe von Informationen an den Benutzer. Dabei ist die Steuereinrichtung der Benutzerschnittstelleneinrichtung vorzugsweise dazu eingerichtet, über die Datenverbindung Daten mit dem Laborgerät auszutauschen, die aus den Benutzereingaben gewonnen wurden, und die im erfindungsgemäßen Laborgerät bewirken, dass dem Benutzer am erfindungsgemäßen Laborgerät Berechtigungen und/oder Zugriffsrechte erteilt werden, wobei vorzugsweise ein gleichzeitiges Angemeldetsein und/oder der gleichzeitige Zugriff eines ersten und wenigstens eines zweiten Benutzers am erfindungsgemäßen Laborgerät über die Schnittstelleneinrichtung mit jeweils zugewiesenen Zugriffsrechten auf Funktionen des Laborgeräts, steuerbar ist.

**Als benutzerabhängige Vorgehensweise** wird bezeichnet, dass in Abhängigkeit von einem Benutzer, insbesondere einer Klasse, Gruppe oder Rolle des Benutzers, die unter verschiedenen Aspekten unterschieden werden kann, oder benutzerindividuell vorgegangen wird. Eine benutzerabhängige Anzeige in der Benutzeroberfläche bedeutet insbesondere, dass eine bestimmte Benutzeroberfläche, insbesondere Abfragemaske, eingesetzt wird, die diesem Benutzer zugeordnet ist.

Die Benutzerklasse oder **Rolle** kann über eine Datenbank ermittelt werden. Für ein Individuum kann sich die Benutzerklasse durch seine fachliche Qualifikation, seine berufliche Stellung im Unternehmen oder durch die Zuordnung nach einem anderen Kriterium ergeben. Das Kriterium kann auch an eine Messung gebunden sein, die durch mindestens einen Sensor durchgeführt wird, der mit der Steuereinrichtung signalverbunden ist und der Bestandteil des Laborautomaten sein kann. Diese Messung kann insbesondere einen personengebundenen Messparameter bestimmen, insbesondere einen Körperparameter der Person ermitteln. Durch diese Information ist es insbesondere möglich, das Laborgerät an das Körpermaß anzupassen, z.B. die Körperhöhe der Person, um z.B. automatisch eine Einstellung des Laborgeräts automatisch an dieses Körpermaß anzupassen. Das kann zu einer verbesserten Ergonomie führen. Der Sensor kann auch ein Mikrofon aufweisen, mit dem die Sprache des Benutzers erfasst, insbesondere aufgezeichnet wird. Die Sprachdaten können für die Erstellung einer Protokolldatei verwendet werden und/oder ausgewertet werden. Vorzugsweise weist die Konfigurationssteuerung, insbesondere deren Steuereinrichtung eine Spracherkennungseinrichtung auf. Die vom Sensor erfasste Sprache kann mittels der Spracherkennungseinrichtung analysiert und ausgewertet werden, wobei Sprachdaten ermittelt werden. Die Sprachdaten können verwendet werden, um ein Steuerungskommando zu erfassen, mit dem die Steuerung des Laborgeräts und/oder der Konfigurationssteuerung beeinflusst werden kann. Die Sprachdaten können insbesondere ausgewertet werden, um die Sprache des Benutzers zu ermitteln. In Abhängigkeit von der Sprache kann die Benutzeroberfläche angepasst werden, indem die auf der Benutzeroberfläche angezeigten Texte und/der die über einen Lautsprecher des Laborgeräts ausgegebene Sprache der durch die Sprachanalyse ermittelten Sprache angepasst wird.

Eine **Schnittstelleneinrichtung** dient der Verbindung von zwei Einrichtungen, die jeweils Signale, insbesondere Informationen, insbesondere Daten, verarbeiten können, insbesondere senden und/oder empfangen können. Eine Schnittstelleneinrichtung kann mindestens eine Hardwareschnittstelle beinhalten und/oder mindestens eine Softwareschnittstelle.

**Hardwareschnittstellen** sind insbesondere Schnittstellen zwischen elektrisch arbeitenden Einheiten, gemäß dem üblichen Verständnis in der Elektrotechnik und Elektronik. Vorliegend bezeichnet der Begriff "Hardwareschnittstelle" insbesondere auch die Verbindungskomponenten zwischen wenigstens zwei elektrisch arbeitenden Einheiten selbst, also insbesondere alle Bestandteile, die diese Verbindung ermöglichen, z.B. integrierte Schaltkreise, Elektronik und Leitungen, über die zwischen den wenigstens zwei elektrisch arbeitenden Einheiten elektrische Signale versandt werden. Diese zwei elektrisch arbeitenden Einheiten können insbesondere ein Laborgerät und eine externe Datenverarbeitungseinrichtung sein, oder zwei Laborgeräte, oder zwei elektrisch arbeitenden Einheiten innerhalb eines Laborgeräts. Eine Hardwareschnittstelle muss nicht, aber kann eine lösbare Verbindungseinrichtung zum Lösen und/oder Wiederherstellen dieser Verbindung aufweisen, insbesondere mindestens einen Stecker.

**Softwareschnittstellen,** insbesondere softwareseitige Datenschnittstellen, sind insbesondere logische Berührungspunkte in einem Informationsverwaltungssystem, insbesondere Softwaresystem: Sie ermöglichen und regeln den Austausch von Kommandos und Daten zwischen verschiedenen Prozessen und Komponenten. Softwareschnittstellen können nur zur Kommunikation benutzte, datenorientierte Schnittstellen sein. In diesem Fall enthält die Softwareschnittstelle lediglich die Informationen, die zwischen beteiligten Systemteilen ausgetauscht werden.

Die vorliegend genannten bevorzugten Gestaltungen eines erfindungsgemäßen Laborgeräts gelten auch für ein Laborgerät, das Bestandteil des erfindungsgemäßen Systems ist.

Erfindungsgemäß ist die Kommunikationseinrichtung des Laborgeräts, insbesondere auch jene der systemzugehörigen externen Datenverarbeitungseinrichtung dazu eingerichtet, eine Kommunikationsanfrage zu empfangen, die von einer -insbesondere zweitenexternen Datenverarbeitungseinrichtung an die Kommunikationseinrichtung gesendet wurde. Eine Kommunikation für einen Datenaustausch kann also einerseits vom Laborgerät und/oder vom erfindungsgemäßen System gestartet, d.h. initiiert werden, wird erfindungsgemäß jedenfalls aber durch eine -insbesondere zweite- externe Datenverarbeitungseinrichtung initiiert, die insbesondere nicht Bestandteil eines Laborgeräts oder des Systems ist. Ähnliche Techniken werden im Bereich der Mobilfunktechnologie als "push technology" bezeichnet. "Push", oder "server push" beschreibt insbesondere eine Internet-basierte Art der Kommunikation zwischen Datenverarbeitungseinrichtungen, bei denen die Anfrage ("request") nach einer vorbestimmten Transaktion durch einen zentralen Server oder den Publizierer von Information initiiert wird. Gewöhnlich liegt -nicht zuletzt aufgrund der Grundrechte auf Selbstbestimmung der die Datenverarbeitungseinrichtungen besitzenden Personen oder Rechtspersonen - die Initiative bei den einzelnen Klienten. Die Push-Technologie erlaubt aber den Versand von Informationen an den Klienten über einen Informationskanal, der nach vorheriger Abstimmung mit dem Klienten, insbesondere dessen "Anmeldung" für den Empfang von "push"-Informationen über diesen Informationskanal zugestimmt hat.

Erfindungsgemäß ist die Kommunikationseinrichtung des Laborgeräts, vorzugsweise auch jene der systemzugehörigen externen Datenverarbeitungseinrichtung dazu eingerichtet, aufgrund der Kommunikationsanfrage der -insbesondere zweiten- externen Datenverarbeitungseinrichtung eine Datenfernverbindung zu erlauben, die die - insbesondere zweite- externe Datenverarbeitungseinrichtung mit der Kommunikationseinrichtung herstellt. Dabei bildet die von der -insbesondere zweitenexternen Datenverarbeitungseinrichtung initiierte Datenfernverbindung einen Informationskanal, über den die -insbesondere zweite- externe Datenverarbeitungseinrichtung zweite Daten an die Kommunikationseinrichtung sendet. Die zweiten Daten können von den ersten Daten abhängig sein, können sich insbesondere auf eine Menge mindestens eines verbrauchten Verbrauchsartikels beziehen. Die zweiten Daten können Produktinformationen repräsentieren, insbesondere Informationen über Laborgeräte oder Verbrauchsartikel, oder Bestätigungsinformationen über begonnene Bestellvorgänge, die insbesondere in Abhängigkeit von ersten Daten begonnen wurden.

Erfindungsgemäß ist die Steuereinrichtung oder Datenverarbeitungseinrichtung des erfindungsgemäßen Laborgeräts und optional zusätzlich oder - nicht erfindungsgemäß ausschließlich - die systemzugehörige externe Steuereinrichtung dazu eingerichtet, die zweiten Daten automatisch zu verarbeiten.

Erfindungsgemäß weist das Laborgerät eine Benutzerschnittstelleneinrichtung mit einer Anzeigeneinrichtung auf. Erfindungsgemäß ist die Steuereinrichtung des erfindungsgemäßen Laborgeräts mittels Implementierung durch Programmcode dazu eingerichtet, die zweiten Daten automatisch zu verarbeiten, indem daraus Push-Informationen gewonnen und diese Push-Informationen dem Benutzer automatisch auf einer Anzeigeneinrichtung einer Benutzerschnittstelleneinrichtung des Laborgeräts angezeigt werden.

Vorzugsweise ist in einer zweiten Gestaltung die Steuereinrichtung des erfindungsgemäßen Laborgeräts dazu eingerichtet, die Kommunikationsanfrage der - insbesondere zweiten- externen Datenverarbeitungseinrichtung in Abhängigkeit von den ersten Daten und/oder in Abhängigkeit von anderen Bedingungen zu steuern, insbesondere die Datenfernverbindung zu erlauben.

Vorzugsweise weist in einer dritten Gestaltung des erfindungsgemäßen Laborgeräts das erfindungsgemäße Laborgerät eine Zugriffssteuerung auf, die dazu ausgebildet ist, den Zugriff, insbesondere die Kommunikationsanfrage, der -insbesondere zweiten- externen Datenverarbeitungseinrichtung zu steuern, insbesondere Zugriffe zu erlauben oder zu verweigern.

Vorzugsweise ist in einer vierten Gestaltung des erfindungsgemäßen Laborgeräts, das insbesondere die zweite und dritte Gestaltung aufweisen kann, die Steuereinrichtung des erfindungsgemäßen Laborgeräts und optional oder - dann nicht erfindungsgemäß - ausschließlich die systemzugehörige externe Datenverarbeitungseinrichtung des erfindungsgemäßen Systems oder die Datenverarbeitungseinrichtung des Laborgeräts dazu eingerichtet, bei mindestens einer Behandlung mindestens eine erste Information über die Größe X in Abhängigkeit von Y zu erfassen, insbesondere durch Zählen oder Messen, wobei die Werte X und Y mindestens einen Verbrauchsartikel Y der Menge X charakterisieren, und diese mindestens eine erste Information in den ersten Daten zu erfassen. Die Menge X kann z.B. eine Anzahl repräsentieren, und/oder ein Volumen und/oder eine Masse.

Vorzugsweise weist in einer fünften Gestaltung des erfindungsgemäßen Laborgeräts, das insbesondere die zweite bis vierte Gestaltung aufweisen kann, das erfindungsgemäße Laborgerät und optional oder - dann nicht erfindungsgemäß - ausschließlich das System eine Benutzerschnittstelleneinrichtung und eine Zugriffssteuerung auf, die dazu eingerichtet ist, den Zugriff mindestens eines über die Benutzerschnittstelleneinrichtung zugreifenden Benutzers, also z.B. eines Individuums, einer Gruppe, eines Administrators, eines Einkäufers, oder eines anderen Rechteinhabers, zu steuern, insbesondere zu erlauben, und diesen Benutzer dabei zu identifizieren. Dabei ist die Steuereinrichtung des Laborgeräts oder die systemzugehörige externe Datenverarbeitungseinrichtung dazu eingerichtet, die ersten Daten in Abhängigkeit vom identifizierten Benutzer zu erfassen und/oder die Information über den identifizierten Benutzer, insbesondere Benutzerdaten, als Bestandteil der ersten Daten zu erfassen.

Vorzugsweise ist in einer sechsten Gestaltung des erfindungsgemäßen Laborgeräts, das insbesondere die zweite bis fünfte Gestaltung aufweisen kann, die Steuereinrichtung des erfindungsgemäßen Laborgeräts dazu eingerichtet, die ersten Daten in Abhängigkeit von mindestens einer geplanten Behandlung zu ermitteln, insbesondere zu berechnen, insbesondere in Abhängigkeit von mindestens einer gemäß den Reservierungsdaten einer Reservierungsdatenbank geplanten mindestens einen Behandlung.

Die Erfindung betrifft ferner ein **System,** aufweisend mindestens ein erfindungsgemäßes Laborgerät und mindestens eine systemzugehörige externe Datenverarbeitungseinrichtung, insbesondere einen Server, die insbesondere zum Austausch von Daten, insbesondere Konfigurationsdaten, miteinander verbunden sind. Dabei ist vorzugsweise die Kommunikationseinrichtung des mindestens einen Laborgeräts zum Herstellen einer Datenverbindung mit der mindestens einen systemzugehörigen externen Datenverarbeitungseinrichtung eingerichtet, um diese wenigstens einigen ersten Daten an die systemzugehörige externe Datenverarbeitungseinrichtung zu übermitteln. Zu diesem Zweck weist die systemzugehörige externe Datenverarbeitungseinrichtung vorzugsweise ebenfalls eine Kommunikationseinrichtung auf. Das System ist insbesondere dazu eingerichtet, dass zwischen der mindestens einen systemzugehörigen externen Datenverarbeitungseinrichtung und dem mindestens einen Laborgerät eine Datenverbindung herstellbar ist, über die die ersten Daten und/oder die zweiten Daten austauschbar sind. Ein derartiges System ermöglicht es, zumindest die ersten Daten im System zu sammeln, insbesondere über einen Zeitraum zu sammeln. Die gesammelten ersten Daten können dann weiter verarbeitet werden, insbesondere können die ersten Daten und/oder die in den ersten Daten enthaltene Information an eine zweite externe Datenverarbeitungseinrichtung übersandt werden. Beispielsweise kann das System in einem Labor angeordnet sein, indem mittels des Systems die ersten Daten, insbesondere Verbrauchsinformationen, von einem oder mehreren Laborgeräten des Systems gesammelt werden. Die zweite externe Datenverarbeitungseinrichtung kann ein Server außerhalb des Labors sein, an den das System z.B. über eine das Internet nutzende Datenfernverbindung die ersten Daten und/oder die darin enthaltene Information übersendet.

Vorzugsweise weist in einer ersten Gestaltung des Systems die systemzugehörige externe Datenverarbeitungseinrichtung eine Speichereinrichtung auf und ist insbesondere dazu eingerichtet, die ersten Daten und/oder zweiten Daten zu verarbeiten, insbesondere in dieser Speichereinrichtung zu speichern.

Vorzugsweise weist das System, das insbesondere die erste Gestaltung aufweisen kann, oder die systemzugehörige externe Datenverarbeitungseinrichtung, in einer zweiten Gestaltung des Systems eine Kommunikationseinrichtung zum Herstellen einer Datenfernverbindung mit mindestens einer zweiten externen Datenverarbeitungseinrichtung auf, um mittels dieser Datenfernverbindung diese wenigstens einigen ersten Daten an die zweite externe Datenverarbeitungseinrichtung zu übermitteln. Die zweite externe Datenverarbeitungseinrichtung ist vorzugsweise nicht Bestandteil des Systems.

Die genannten Gestaltungen des erfindungsgemäßen Laborgeräts und/oder des erfindungsgemäßen Systems können mit anderen Merkmalen kombiniert werden, die im Rahmen der Beschreibung der vorliegenden Erfindung genannt sind.

Die **Zugriffssteuerung** erlaubt es bei einem Laborgerät, den Zugriff eines oder mehrerer weiterer Benutzer am Laborgerät zu steuern, während ein erster Benutzer bereits angemeldet ist und dessen Sitzung am Laborgerät noch läuft, also während des Zugriffs des weiteren Benutzers noch aktiv ist. Durch diese Ausgestaltung lässt sich das Laborgerät effizienter nutzen und die Produktivität des Labors verbessern.

Eine **Steuereinrichtung** weist im Rahmen der vorliegenden Erfindung generell insbesondere eine Datenverarbeitungseinrichtung, insbesondere eine Recheneinheit (CPU) zum Verarbeiten von Daten und/oder einen Mikroprozessor auf oder ist eine Datenverarbeitungseinrichtung. Die Recheneinheit der Steuereinrichtung eines Laborgeräts ist vorzugsweise auch zum Steuern des Behandlungsprozesses und/oder der individuellen Behandlungen eingerichtet.

Die Steuereinrichtung des Laborgeräts und/oder die optionale Zugriffssteuerung und/oder die optionale Benutzerschnittstelleneinrichtung können -insbesondere alle- in einer physikalischen Geräteinheit integriert sein, können aber jeweils auch eine eigene physikalischen Geräteinheit sein. Eine physikalische Geräteinheit kann insbesondere ein Modul sein, das mit dem Laborgerät verbunden ist oder verbindbar ist. Die Steuereinrichtung des Laborgeräts und/oder die optionale Zugriffssteuerung und/oder die optionale Benutzerschnittstelleneinrichtung oder Bestandteile dieser Komponenten können auch durch Softwarefunktionen implementiert sein, oder können insbesondere als Programmcode vorliegen. Ein Laborgerät kann z.B. einen Computer aufweisen, der in Kombination mit Softwarefunktionen die Steuereinrichtung des Laborgeräts und/oder die optionale Zugriffssteuerung und/oder die optionale Benutzerschnittstelleneinrichtung jeweils zumindest teilweise implementiert. Ist z.B. die Zugriffssteuerung in das Laborgerät integriert, kann die Zugriffssteuerung selber Teil der Steuereinrichtung des Laborgeräts sein bzw. mittels der Steuereinrichtung implementiert sein, insbesondere durch Softwarefunktionen, insbesondere zumindest teilweise als ausführbarer Programmcode.

Ein **Modul** kann insbesondere die Zugriffssteuerung und/oder die Konfigurationssteuerung und/oder eine Benutzerschnittstelleneinrichtung aufweisen. Ein Modul ist ein von anderen Geräten getrenntes, und/oder ein vom anderen Gerät, insbesondere Laborgerät, abtrennbares Gerät. Ein Laborgerät kann eine Verbindungseinrichtung aufweisen, durch die das Modul mit dem Laborgerät verbindbar ist, insbesondere mittels einer vom Benutzer lösbaren Verbindung. Ein Modul kann portabel sein, also von einem Benutzer transportierbar. Das Modul kann auch fest mit dem Laborgerät verbunden sein. Die modulare Bauweise bietet Vorteile bei der Herstellung von Laborgeräten. Ein portables Modul bietet eine größere Flexibilität bei der Benutzung eines Laborgeräts.

Vorzugsweise weist die systemzugehörige externe Datenverarbeitungseinrichtung eine Recheneinheit auf.

Bevorzugte Ausgestaltungen der Zugriffssteuerung und des Laborgeräts mit dieser Zugriffssteuerung werden im Rahmen der Beschreibung der vorliegenden Erfindung genannt oder lassen sich dort entnehmen.

Die **Zugriffssteuerung** ist vorzugsweise dazu eingerichtet, während der Sitzung des ersten Benutzers die Anfrage des mindestens einen weiteren Benutzers nach einer Anmeldung an der Zugriffssteuerung, insbesondere den Zugriff auf mindestens eine Funktion des Laborgeräts zu steuern, insbesondere der Anfrage stattzugeben (Zugriff erlaubt) oder die Anfrage abzulehnen (Zugriff verweigert).

Die Zugriffssteuerung ist eine zur Datenverarbeitung ausgebildete Einrichtung, die vorliegend auch als "access control device" bezeichnet wird. Sie dient der Zugriffskontrolle (englisch: "access control"). Die Zugriffssteuerung weist eine Steuereinrichtung auf. Die Steuereinrichtung ist zur Datenverarbeitung ausgebildet. Die Steuereinrichtung ist insbesondere eine elektronische Steuereinrichtung. Sie weist vorzugsweise eine Datenverarbeitungseinrichtung auf, die insbesondere elektronisch ist.

Die **Datenverarbeitungseinrichtung** weist vorzugsweise eine Recheneinheit auf, insbesondere eine CPU, ferner vorzugsweise mindestens eine Datenspeichereinrichtung, insbesondere zur flüchtigen und/oder dauerhaften Speicherung von Daten. Die Datenverarbeitungseinrichtung ist vorzugsweise dazu ausgebildet, über die erste Schnittstelleneinrichtung eine oder mehrere erste Datenverbindungen mit einer oder mehreren Benutzerschnittstelleneinrichtungen herzustellen, die insbesondere Bestandteile der Zugriffssteuerung oder des Laborgerätes sein können; vorzugsweise über die zweite Schnittstelleneinrichtung eine zweite Datenverbindung mit dem Laborgerät herzustellen; und vorzugsweise Zugriffsrechte für den Zugriff von Benutzern über die Benutzerschnittstelleneinrichtungen und die ersten und zweiten Datenverbindungen auf Funktionen des Laborgerätes zu steuern; wobei vorzugsweise die Zugriffsrechte so gesteuert werden können, dass ein gleichzeitiger Zugriff (Eingeloggt sein) eines ersten und wenigstens eines weiteren Benutzers mit jeweils getrennt zugewiesenen Zugriffsrechten auf Funktionen des Laborgerätes, erfolgt.

Eine Datenverbindung verbindet insbesondere zwei datenverarbeitende Einheiten, insbesondere zwei Datenverarbeitungseinrichtungen, in der Weise, dass Daten zwischen den Einheiten ausgetauscht werden können, entweder unidirektional oder bidirektional. Die Datenverbindung kann leitungsgebunden realisiert sein, oder drahtlos, insbesondere als Funkverbindung. Eine Datenfernverbindung verbindet insbesondere zwei datenverarbeitende Einheiten, insbesondere zwei Datenverarbeitungseinrichtungen, die entfernt voneinander angeordnet sind, die also insbesondere nicht Bestandteil desselben Geräts, insbesondere derselben Konfigurationssteuerung, Zugriffssteuerung, Benutzerschnittstelleneinrichtung oder desselben Laborgeräts sind, falls die genannten Geräte als separate Geräte ausgeführt sind. Eine Datenverbindung, insbesondere eine Datenfernverbindung, eines Geräts mit einem anderen Gerät wird vorzugsweise über eine direkte Verbindung der beiden Geräte realisiert, oder eine mittelbare Verbindung der beiden Geräte, so dass ein drittes Gerät zwischen die beiden Geräte geschaltet ist, um die Daten weiterzuvermitteln. Eine Datenfernverbindung kann insbesondere über ein Netzwerk aus Computern realisiert sein, bei denen die über die Datenfernverbindung verbundenen Geräte über das Netzwerk verbunden sind. Das Netzwerk kann ein beschränktes Netzwerk sein, z.B. ein Intranet, oder kann ein weltweites Netzwerk sein, insbesondere das Internet.

Die Zugriffssteuerung ist vorzugsweise dazu eingerichtet, die Zugriffsrechte zu steuern, indem die Steuereinrichtung eine Datenverbindung mit einer **Datenbank für Zugriffsrechte** verwendet. Die Datenbank für Zugriffsrechte ist vorzugsweise in mindestens einer, vorzugsweise in genau einer, Speichereinrichtung für Zugriffsrechte gespeichert. Die mindestens eine Speichereinrichtung für Zugriffsrechte kann in der Zugriffssteuerung angeordnet sein, und/oder kann in einer externen Datenverarbeitungseinrichtung angeordnet sein. Extern bedeutet, dass das Gerät, hier die Datenverarbeitungseinrichtung, nicht Bestandteil der in Rede stehenden Vorrichtung, hier der Zugriffssteuerung ist. Die Datenbank für Zugriffsrechte kann zentral gespeichert sein, kann aber auch in mehreren Speichereinrichtungen gespeichert sein, die jeweils Teile der Daten der Datenbank aufweisen können oder eine Kopie der Daten der Datenbank aufweisen können.

Eine -insbesondere zweite- **externe Datenverarbeitungseinrichtung** kann ein Computer sein, insbesondere ein Server sein, der insbesondere zur Herstellung einer Datenverbindung zu mehr als einer Zugriffssteuerung und/oder mehr als einem Laborgerät eingerichtet ist. Eine -insbesondere zweite- externe Datenverarbeitungseinrichtung kann einen Computer oder Mikroprozessor aufweisen. Ein Server ist insbesondere ein Computer, dessen Hardware vorzugsweise auf Serveranwendungen abgestimmt ist. Eine externe Datenverarbeitungseinrichtung kann ein Server sein, der mit dem erfindungsgemäßen System oder Laborgerät insbesondere über das Internet kommuniziert. Eine externe Datenverarbeitungseinrichtung kann eine mobile Datenverarbeitungseinrichtung sein, die zur Herstellung einer drahtlosen Datenverbindung eingerichtet ist, insbesondere einer Datenverbindung über ein begrenztes, insbesondere ein Intranet, oder weltweites Rechnernetz, insbesondere das Internet. Ein Rechnernetz ist ein Zusammenschluss verschiedener technischer, primär selbstständiger elektronischer Systeme (insbesondere Computer, aber auch Sensoren, Aktoren, Agenten und sonstiger funktechnischer Komponenten usw.), der die Kommunikation der einzelnen Systeme untereinander ermöglicht.

Das Laborgerät, die Steuereinrichtung des Laborgeräts oder die die Steuereinrichtung der optionalen Zugriffssteuerung kann eine **Kommunikationseinrichtung** zur Herstellung einer Datenverbindung mit einer externen Datenverarbeitungseinrichtung, insbesondere über eine Schnittstelleneinrichtung des Laborgeräts aufweisen. Die Zugriffssteuerung ist vorzugsweise dazu ausgebildet, die Zugriffsrechte unter Verwendung der Datenverbindung zu der externen Datenverarbeitungseinrichtung herzustellen, insbesondere über eine Schnittstelleneinrichtung der Zugriffssteuerung. Die systemzugehörige externe Datenverarbeitungseinrichtung weist vorzugsweise die Datenbank für Zugriffsrechte zumindest teilweise oder vollständig auf.

Die Zugriffssteuerung, insbesondere eine Steuereinrichtung der Zugriffssteuerung, ist vorzugsweise dazu eingerichtet, Berechtigungen und/oder Zugriffsrechte für den Zugriff von Benutzern über die Benutzerschnittstelleneinrichtungen und die ersten und zweiten Datenverbindungen auf Funktionen des Laborgeräts zu steuern. Dadurch wird eine benutzerabhängige Nutzung des Laborgeräts möglich, die in Abhängigkeit von den jeweils vergebenen Zugriffsrechten gesteuert wird. Insbesondere wird eine gleichzeitige Nutzung des Laborgeräts durch mindestens einen ersten und mindestens einen zweiten Benutzer möglich.

Die Zugriffssteuerung ("access control device") führt die Zugriffskontrolle ("access control") aus. Der Begriff "Zugriffskontrolle" bezeichnet insbesondere Verfahren zur Verwaltung der Anfrage (englisch: "requests") nach Ressourcen und/oder Daten, die durch ein Informationsverwaltungssystem verwaltet werden, und zur Verwaltung der Entscheidungen, wie die Anfrage gehandhabt wird, insbesondere ob Zugriff gewährt wird oder nicht, und/oder in welcher Weise Zugriff gewährt wird oder nicht. Das Informationsverwaltungssystem kann insbesondere eine Betriebssystem sein, das auf der Zugriffssteuerung ausgeführt wird. Wenn der Benutzer eines Informationsverwaltungssystems eine bestimmte Operation auf einer bestimmten Ressource und/oder bestimmten Daten durchführen möchte, entscheidet die Zugriffskontrolle, ob diese Anfrage tatsächlich zugelassen oder abgelehnt werden soll. Eine Zugriffskontrollentscheidung (ja/nein) bezieht sich insbesondere auf ein Zugangssteuerungstriplett ("access control triple") bestehend aus "Subjekt", "Objekt" und "Operation" .

Als Subjekt wird insbesondere eine aktive Entität eines Systems bezeichnet, die eine bestimmte Operation auf einem bestimmten Objekt durchführen möchte. Eine Entität bezeichnet in diesem Zusammenhang eine eindeutig bestimmbare Einheit, über die Informationen gespeichert und/oder verarbeitet werden sollen. Die Einheit kann materiell oder immateriell, konkret oder abstrakt sein. Subjekte sind insbesondere menschliche Benutzer eines Informationsverwaltungssystems oder Computerprogramme, die von menschlichen Nutzern zur Erfüllung von Aufgaben eingesetzt werden. Ein Subjekt kann auch eine Gruppe von Nutzern sein, z.B. Laborarbeiter, Servicetechniker, Administrator. Die Gruppe fasst demnach mehrere individuelle Subjekte zusammen.

Ein Benutzer kann ein Individuum repräsentieren, oder eine Gruppe von mehreren Individuen, oder eine Klasse von Individuen, die gemäß einer Klassenregel oder Rollenregel ausgewählt wurden.

Die Zugriffssteuerung kann vorzugsweise den mindestens einen ersten Benutzer und den mindestens einen zweiten Benutzer voneinander unterscheiden. Ein Benutzer wird von der Zugriffssteuerung vorzugsweise eindeutig identifiziert. Dazu verarbeitet die Zugriffssteuerung vorzugsweise Identifizierungsdaten. Vorzugsweise ist die Zugriffssteuerung dazu ausgebildet, den anfragenden Benutzer zu **authentifizieren,** das heißt, ein Nachweisverfahren durchzuführen, mit dem die Authentizität des anfragenden Benutzers überprüft wird und der Benutzer authentisiert wird, falls der Nachweis positiv ist. Authentifizierungsdaten enthalten zum Beispiel einen Logintext und einen Passworttext, oder einen Datensatz zur Gesichtserkennung oder zum Iris-Scan oder zum Fingerabdruckscan etc.. Die Authentifizierung kann ferner mittels RFID Chips oder NFC Chip, oder über Gestenerkennung erfolgen. Eine Authentifizierung kann insbesondere per Direktzugriff auf das Laborgerät bzw. dessen Zugriffssteuerung vor Ort erfolgen oder per Fernzugriff.

Die Zugriffssteuerung weist vorzugsweise ein **Informationsverwaltungssystem** auf, mit dem die Zugriffskontrolle realisiert wird. Das Informationsverwaltungssystem ist vorzugsweise ein Betriebssystem eines Laborgeräts und/oder ein Betriebssystem der Zugriffssteuerung eines Laborgeräts, mit dem die Zugriffssteuerung und/oder das Laborgerät betrieben wird.

Die Zugriffssteuerung ist vorzugsweise dazu ausgebildet, den **anfragenden Benutzer,** insbesondere mehrere anfragende Benutzer, insbesondere den mindestens einen ersten Benutzer und den mindestens einen zweiten Benutzer, an der Zugriffssteuerung, insbesondere am Informationsverwaltungssystem der Zugriffssteuerung anzumelden. Der Anmeldevorgang wird auch als Einloggen bezeichnet. Vorzugsweise erhält der erfolgreich angemeldete Benutzer vorbestimmte Berechtigungen und/oder Zugriffsrechte. Die Anmeldung kann vom Benutzer selber aufgehoben werden oder aufgrund anderer Bedingungen aufgehoben werden, z.B. durch das gerätegesteuerte Abmelden des Benutzers, insbesondere bei Überschreiten einer maximalen Anmeldezeit, in der der Benutzer ohne Unterbrechung über die Zugriffssteuerung eingeloggt war, oder nach einer vorbestimmten Zeit der Inaktivität, oder in Abhängigkeit vom Zeitpunkt des Endes einer vom Benutzer ausgeführten Behandlung, oder aufgrund einer individuellen Methodenprogrammierung. Das Aufheben der Anmeldung bedeutet vorzugsweise, dass die bei der Anmeldung gewährte Autorisierung wieder entzogen wird.

Das Einloggen in das Informationsverwaltungssystem erfolgt vorzugsweise dadurch, dass der Benutzer authentifiziert wird. Nach erfolgter Authentifizierung erhält der Benutzer zum Einloggen einen personalisierten Zugang zu dem Informationsverwaltungssystem, mit Berechtigungen und/oder Zugriffsrechten, die mittels der Datenbank für Zugriffsrechte ermittelt werden. Mit dem Login beginnt eine Sitzung, die durch ein Logout, auch bezeichnet als Abmelden, beendet wird.

Die Zugriffssteuerung ist vorzugsweise dazu ausgebildet, die Verwendung der Berechtigungen, Operationen und Objekte an dem Laborgerät, bzw. die Funktionen und Dienste des Laborgeräts, das die Zugriffssteuerung aufweist, in Abhängigkeit von den vorbestimmten Zugriffsrechten freizugeben, d.h. den authentifizierten Benutzer zu autorisieren. Die Zugriffssteuerung ist vorzugsweise Software-gesteuert, insbesondere programmgesteuert. Vorzugsweise wird als Anwendungsprotokoll bei der Implementierung der Softwarefunktionen LDAP eingesetzt (Lightweight Directory Access Protocol).

Als Objekt wird bei einem Zugriff bzw. bei einem Zugriffsversuch insbesondere eine passive Entität bezeichnet, auf der eine Operation durchgeführt werden soll. Objekte werden auch als "Ressourcen" bezeichnet. Objekte können z.B. sein: Daten oder Datensammlungen, d.h. Dateien, Datenobjekte in Datenbanken, z.B. Tabellen oder Spalten), Dienste oder Funktionen, insbesondere solche Dienste oder Funktionen, die mit der Zugriffssteuerung und/oder dem Laborgerät durchführbar sind. Solche Dienste können z.B. bezeichnen das Verfügbarmachen einer Kalenderdatenbank, wobei diese Verwendung die Anzeige von Kalenderdaten, die Lese- und/oder Schreibrechte an der Kalenderdatenbank vorsehen kann. Solche Dienste und Funktionen können z.B. bezeichnen eine Nachrichtenfunktion, mittels der Benutzern Nachrichten übersandt werden können, die insbesondere Informationen über die Verfügbarkeit des Laborgeräts in einem bestimmten Kalenderzeitraum enthalten können. Eine solche Funktion wäre insbesondere auch das Verfügbarmachen der Ausführung einer Behandlung, was insbesondere beinhalten kann, die dafür notwendigen Zugriffsrechte zu erteilen. Eine Funktion kann z.B. das Einschalten einer UV-Beleuchtung des Laborgeräts sein oder das Öffnen einer Gehäusetüre eines Laborgerätegehäuses.

Als Operationen werden Vorgänge bezeichnet, die auf einem Objekt ausgeführt werden. Operationen können insbesondere Funktionen sein, insbesondere Funktionen der Zugriffssteuerung oder des Laborgeräts. Mehrere Funktionen können auf einem Objekt ausgeführt werden. Ist das Objekt eine Datei, sind mögliche Operationen das Schreiben, Lesen, Hinzufügen, Verändern, Kopieren oder Löschen von Daten. Ist das Objekt ein Dienst oder eine Funktion, so kann ausführen die einzige mögliche Operation sein. Die Menge der möglichen Operationen hängt von der Art des Objekts ab. Die Mengen der Operationen, die von einzelnen Subjekten auf dem gleichen Objekt durchgeführt werden können, können unterschiedlich sein.

Ein bestimmtes Objekt in Kombination mit einer bestimmten Operation wird insbesondere als Berechtigung bezeichnet. Als "Leseberechtigung" lässt sich z.B. die Kombination aus der Operation "Lesen" mit dem Objekt "Datei" verstehen, als "Ausführungsberechtigung" lässt sich z.B. die Operation "Ausführen" mit dem Objekt "Funktion" verstehen.

Die Zugriffskontrolle kann insbesondere als Erlaubnisfunktion formuliert werden, formal beschrieben durch

$$\textit{Erlaubnis\_für(Subjekt, Objekt, Operation)} \rightarrow \textit{(ja, nein).}$$

Wird dieser Funktion das Triple an Parametern (Subjekt, Objekt, Operation) übergeben, so liefert die Erlaubnisfunktion entweder "ja" (Zugriff gewährt) oder "nein" (Zugriff verweigert) zurück.

Es ist auch möglich, bei dieser Erlaubnisfunktion einen weiteren Eingabeparameter vorzusehen, der eine weitere Bedingung für die Zugriffsentscheidung liefert. Diese Bedingung kann z.B. den Zweck bezeichnen, zu dem ein bestimmter Zugriff erfolgen soll. Ferner ist es möglich, dass die Erlaubnisfunktion nicht -oder nicht nur- die ja/nein-Entscheidung über die Zugriffserlaubnis zurückliefert, sondern eine Auflage (auch "Obligation") zurückliefert, in Abhängigkeit von der über die Zugriffserlaubnis entschieden wird. Dadurch kann insbesondere eine "Erlaubnis unter Vorbehalt" definiert werden. Eine solche Obligation ist insbesondere bereits vor dem Zugriff bzw. Zugriffsversuch erfüllt, kann aber auch während -oder nach- dem Zugriff oder der zu erlaubenden Operation erfüllt werden.

Die Zugriffskontrolle kann gemäß einem oder mehrerer spezieller Datenmodelle erfolgen. Ein solches spezielles Datenmodell ist insbesondere das Zugriffskontrollmodell (ZKM, Access Control Model (ACM)). Die Zugriffskontrolle kann insbesondere einen sogenannten Referenz-Monitor aufweisen. Diese Komponente ist insbesondere als funktioneller Kern der Zugriffssteuerung zu verstehen. Der Referenz-Monitor erfüllt die Funktion der Entscheidung, ob der von einem Subjekt gewünschte Zugriff auf ein Objekt gewährt wird. Vorzugsweise ist von der Zugriffssteuerung kein Zugriff auf eine Ressource des Laborgeräts freigebbar, ohne dass der Referenz-Monitor benutzt wird. Der Referenz-Monitor erfüllt vorzugsweise auch die Funktion, die erfolgten Zugriffsversuche aufzuzeichnen.

Die Datenbank über Zugriffsrechte enthält vorzugsweise Informationen in Form von Daten darüber, welche Operationen, insbesondere abhängig von einem bestimmten Zeitpunkt oder Zeitraum, für ein Objekt verfügbar sind. Dadurch kann insbesondere festgelegt werden, ob für einen Benutzer zu einem bestimmten Zeitpunkt und/oder in einem bestimmten Zeitraum der Zugriff auf die mindestens eine Behandlungseinrichtung erlaubt wird, insbesondere ob zu einem bestimmten Zeitpunkt und/oder in einem bestimmten Zeitraum das Recht vergeben wird, eine Behandlung am Laborgerät zu starten oder zu ändern, wobei das Laborgerät mit der Zugriffssteuerung über die zweite Datenverbindung verbindbar ist und/oder verbunden ist.

Die Datenbank über Zugriffsrechte enthält vorzugsweise Informationen in Form von Daten darüber, welche Berechtigungen an den anfragenden Benutzer vergeben werden können, insbesondere in Abhängigkeit von möglichen Rechten aufgrund einer Gruppenzugehörigkeit und/oder Rollenzugehörigkeit.

Die Zugriffskontrolle ist vorzugsweise gemäß einem oder auch gemäß mehrerer der bekannten Grundformen DAC ("Discretionary Access Control"), MAC ("Mandatory Access Control") oder RBAC ("Role-Based Access Control") ausgestaltet, wobei RBAC besonders bevorzugt ist.

Das RBAC-Modell sieht vor, dass einzelnen Subjekten nicht direkt Rechte zugewiesen werden sondern indirekt über sogenannte "Rollen". Ein möglicher, im Rahmen der Gestaltung der Zugriffssteuerung anwendbarer Standard des RBAC Modell ist detailliert beschrieben im US Standard ANSI INCITS 359-2004. Die Zugriffssteuerung kann zumindest teilweise als RBAC-Modell ausgebildet sein, insbesondere zumindest teilweise gemäß dem genannten US Standard.

Vorzugsweise sieht die Zugriffskontrolle den Einsatz mindestens einer Rolle vor, vorzugsweise mehrerer Rollen, wobei insbesondere in der Rolle jeweils Rechte zusammengefasst sind. Die mindestens eine Rolle ist vorzugsweise in der Datenbank für Zugriffsrechte gespeichert. Eine Rolle ist insbesondere geeignet angepasst an eine Zuständigkeit oder eine Aufgabenbeschreibung im Rahmen des Einsatzes eines Laborgeräts, insbesondere innerhalb des Unternehmens, welches das Laborgerät einsetzt, und/oder beim Unternehmen, dass einen Wartungsvertrag über das Laborgerät erfüllt, indem er z.B. Diagnosefunktionen am Laborgerät ausführt, und/oder beim Hersteller des Laborgeräts, der z.B. Firmware-Aktualisierungen, Kalibrierungen oder Informationen über das Laborgerät und/oder dessen Zubehör über die Zugriffssteuerung direkt an das Laborgerät übersendet. Solche Rollen können insbesondere Rechte zusammenfassen. Anstatt für jeden Benutzer einen Satz an Einzelrechten zu speichern, kann ihm mindestens eine Rolle zugeordnet werden. Die Rollenzuweisung ist besonders zuverlässig in der Umsetzung und erfordert relativ wenig Aufwand, insbesondere Verwaltungsaufwand beim Ermitteln und Speichern der Rechte.

Vorzugsweise sieht die Zugriffskontrolle mindestens zwei, vorzugsweise mehrere **Rollen** vor. Mögliche Rollen sind insbesondere Administrator ("Admin"), Wartungsdienst (Maintenance), normaler Labor-Benutzer ("LabUser"), unerfahrener Labor-Benutzer ("Inexperienced"), Manager. Durch solche Rollen wird eine sichere und effiziente Zugriffskontrolle ermöglicht. Die Benutzung eines mit der Zugriffssteuerung versehenen Laborgeräts ist sicher und effizient. Es wird auf einfache Weise verhindert, dass ein Benutzer z.B. wegen mangelnder Qualifikation bestimmte Operationen am Laborgerät durchführt, die gegebenenfalls zu Beschädigungen oder ineffizienter Nutzung des Laborgeräts bzw. zu Mehrkosten im Betrieb führen, z.B. durch überflüssigen Verbrauch an Verbrauchsmaterial, das für eine Behandlung verwendet wird.

Vorzugsweise sieht die Zugriffskontrolle mindestens eine Rolle oder mehr als eine Rolle vor, denen ein Benutzer gleichzeitig zugeordnet sein kann. Ein Individuum kann somit z.B. als Administrator oder als normaler Labor-Benutzer Zugriff erhalten, in Abhängigkeit von einer weiteren Bedingung. Vorzugsweise kann der Benutzer selber entscheiden, in welcher Rolle er am Laborgerät Zugriff erhält. Es ist aber auch möglich dass der Benutzer dies nicht selber entscheidet sondern dies die Zugriffssteuerung entscheidet. Diese Bedingung kann der verwendete Datensatz zur Authentifizierung sein, insbesondere das verwendete Passwort, oder kann von einem Parameter des Laborgeräts abhängen, insbesondere von einem Betriebsparameter des Laborgeräts, z.B. einem Betriebsparameter, der den Fehlerstatus des Laborgeräts kennzeichnet.

Vorzugsweise weist die Steuereinrichtung der Zugriffssteuerung und/oder die Steuereinrichtung des Laborgeräts einen Zeitgeber, insbesondere eine Uhr, und/oder insbesondere eine **Reservierungseinrichtung** auf, die eine Speichereinrichtung aufweist, in der Reservierungsdaten gespeichert sind, die insbesondere mindestens einen Reservierungsdatensatz oder eine Vielzahl von Reservierungsdatensätzen enthalten, die zumindest einen Reservierungszeitplan ("booking schedule"), insbesondere für jede Behandlungseinrichtung einzeln, beschreiben.

Ein Reservierungsdatensatz enthält insbesondere mindestens eine der Informationen, insbesondere welcher Benutzer, insbesondere zu welchem Zeitpunkt, insbesondere welche Behandlung von Proben, insbesondere mittels welches Laborgerätes, durchführt, bzw. durchgeführt hat, bzw. durchführen wird. Die Reservierungsdaten enthalten vorzugsweise Informationen über die von der Reservierungseinrichtung akzeptierten Reservierungen, die nach einem Vergleich mit den im Reservierungszeitplan vorhandenen freien Kapazitäten tatsächlich bestätigt und in den Reservierungszeitplan aufgenommen wurden. Die Reservierungsdaten können aber auch Reservierungsanträge ("booking requests") enthalten, die die Reservierungseinrichtung insbesondere auch zu einem späteren Zeitpunkt nach der Antragstellung erneut prüfen kann und gegebenenfalls später akzeptieren kann, falls z.B. ein früherer Eintrag in der Reservierungszeitplan nachträglich storniert wurde. Der Reservierungsdatensatz enthält vorzugsweise auch die Information darüber, welche Art der Behandlung an einem Laborgerät jeweils geplant ist, welcher konkrete Zeitraum, oder welche Dauer der Belegung des Laborgeräts dabei vorgesehen ist, und/oder Informationen über das zu verwendende Methodenprogramm, und enthält vorzugsweise insbesondere mindestens einen Programmparameter oder Steuerparameter.

Vorzugsweise ist die Zugriffssteuerung dazu ausgebildet, einem Benutzer auf Anfrage mindestens eine Information über den Reservierungszeitplan zu übersenden, insbesondere den Reservierungszeitplan teilweise oder vollständig zu übersenden oder mindestens eine Änderung des Reservierungszeitplans zu übersenden. Vorzugsweise ist die Zugriffssteuerung dazu ausgebildet, einem Benutzer automatisch eine Nachricht in Abhängigkeit von mindestens einer Bedingung zu übersenden. Diese Bedingung könnte die Änderung des Reservierungszeitplans eines Laborgeräts sein, insbesondere betreffend die Verfügbarkeit eines Termins zur Durchführung einer Behandlung, insbesondere das Freiwerden oder Stornieren eines Termins.

Die "Art der Behandlung" ist insbesondere durch die Programmparameter vorgegeben, die eine Behandlung kennzeichnen. Solche Programmparameter werden insbesondere von der Steuereinrichtung dazu verwendet, ein Methodenprogramm zu erzeugen. Ein Methodenprogramm ist insbesondere ein Steuerungscode zur Steuerung der Behandlung mittels Steuerparameter. Die Steuerparameter werden insbesondere von der Steuereinrichtung, insbesondere von einem in der Steuereinrichtung ablaufenden Steuerprogramm, z.B. einem Betriebssystem, unter Verwendung der Programmparameter erzeugt. Die Behandlung einer Probe wird insbesondere durchgeführt, indem von der Steuereinrichtung ein Methodenprogramm ausgeführt wird.

Eine "Art der Behandlung" meint eine Methode, nämlich eine Anwendungsart (z.B. "MagSep Blood gDNA", "Compose Mastermix" etc.). Der Benutzer wählt bei einer bevorzugten Ausgestaltung des Laborgeräts als Laborautomat zunächst eine gewünschte Anwendung, also eine "Art der Behandlung", indem er insbesondere am Touchscreen eines Geräts eine Anwendung auswählt. Diese Anwendung, auch bezeichnet als "Methode", ist insbesondere einem Programmmodul zugeordnet, das insbesondere ein Bestandteil des Steuerprogramms sein kann. Insbesondere mittels des Programmmoduls wird vom Benutzer mindestens ein Programmparameter abgefragt. Ein Programmmodul erzeugt insbesondere ein Methodenprogramm auf Basis des mindestens einen vom Benutzer gewählten Programmparameters.

Vorzugsweise ist die Steuereinrichtung der Zugriffssteuerung und/oder die Steuereinrichtung des Laborgeräts dazu ausgebildet, Reservierungsdaten in der Speichereinrichtung der Reservierungseinrichtung abzuspeichern.

Vorzugsweise ist die Steuereinrichtung der Zugriffssteuerung und/oder die Steuereinrichtung des Laborgeräts dazu ausgebildet, den von einem Benutzer in das Laborgerät, insbesondere mittels der Benutzerschnittstelleneinrichtung oder einer portablen bzw. mobilen Benutzerschnittstelleneinrichtung eingegebenen Reservierungsdatensatz aufzunehmen.

Vorzugsweise ist die Steuereinrichtung der Zugriffssteuerung und/oder die Steuereinrichtung des Laborgeräts dazu ausgebildet, den von einem Benutzer eingegebenen Reservierungsdatensatz mit bereits in der Speichereinrichtung der Benutzerschnittstelleneinrichtung gespeicherten Reservierungsdaten zu vergleichen.

Vorzugsweise ist die Steuereinrichtung der Zugriffssteuerung und/oder die Steuereinrichtung des Laborgeräts dazu ausgebildet, mindestens einen, einige oder alle von mindestens einem Benutzer eingegebenen Reservierungsdatensätze in der Speichereinrichtung zu speichern.

Vorzugsweise ist die Steuereinrichtung der Zugriffssteuerung und/oder die Steuereinrichtung des Laborgeräts dazu ausgebildet, einige oder alle von mindestens einem Benutzer eingegebenen und in der Speichereinrichtung gespeicherten Reservierungsdatensätze gemäß einem in der Steuereinrichtung abgelegten Auswerteverfahren auszuwerten, und nach mindestens einem Kriterium den Zeitplan zu erstellen, indem der/die Reservierungsdatensätze gemäß dem mindestens einen Kriterium eines in der Steuereinrichtung abgelegten Sortierverfahrens sortiert werden.

Vorzugsweise ist die Steuereinrichtung der Zugriffssteuerung und/oder die Steuereinrichtung des Laborgeräts dazu ausgebildet, das Auswerteverfahren dem mindestens einen Reservierungsdatensatz eine Priorität zuordnet, die gemäß mindestens eines Kriteriums ermittelt wird.

Das Kriterium kann insbesondere durch eine in der Steuereinrichtung gespeicherten Datentabelle repräsentiert sein, in der z.B. die Priorität zu mindestens einem anderen Parameter in Bezug gesetzt wird, wobei dieser andere Parameter z.B. den Benutzer oder eine Benutzergruppe, oder die Klassifizierung einer Behandlung gemäß einer Relevanzliste (z.B. von wichtig bis unwichtig, kostspielig bis kostengünstig, etc.) charakterisieren kann.

Vorzugsweise ist die Steuereinrichtung der Zugriffssteuerung und/oder die Steuereinrichtung des Laborgeräts dazu ausgebildet, dass das Sortierverfahren mindestens zwei Reservierungsdatensätze gemäß mindestens eines Kriteriums sortiert, um insbesondere einen Zeitplan zu erstellen, der andere Zeitdaten verwendet, als dies in den Reservierungsdatensätzen der Benutzer vorgesehen ist.

Das Kriterium kann entsprechend der Definitionen beim Auswerteverfahren gewählt sein. Vorzugsweise ist die Steuereinrichtung zur Realisierung eines bevorzugten Kriteriums dazu ausgebildet, die Reservierungsdatensätze unter dem Aspekt zu sortieren, dass eine Ressource optimiert wird.

Diese Ressource kann z.B. die Zeit sein, insbesondere kann eine Minimierung der Wartezeiten angestrebt werden, die ein Benutzer jeweils als Differenz zwischen der von ihm gewünschten Startzeit und der vom Laborgerät nach Auswertung und Sortierung zugewiesenen Startzeit für sein Experiment, also die von ihm gewünschte Behandlung, erhält. Es kann auch die Minimierung der Passivitätszeit angestrebt werden, während der ein Laborgerät nicht genutzt wird. Insbesondere können auch zwischenzeitliche Wartungs-, Reinigungs- und/oder Sterilisierungsvorgänge mit eingeplant werden, während der z.B. mindestens ein Arbeitsbereich mindestens eines Laborgeräts bzw. Laborautomaten vorbereitet wird, insbesondere manuell und/oder automatisch vorbereitet, und/oder gereinigt und/oder sterilisiert wird.

Diese Ressource kann auch die Energie sein, die möglicherweise über verschieden und nacheinander ablaufende Behandlungen in Abhängigkeit von deren Reihenfolge unterschiedlich stark verbraucht wird.

Die Ressource kann ein Verbrauchsmaterial sein, insbesondere eine Substanz, z.B. ein Reinigungsmittel, oder bestimmte Transportbehälter, z.B. Pipettenspitzen, oder Lagerbehälter, z.B. Mikrotiterplatten, die über verschieden und nacheinander ablaufende Behandlungen in Abhängigkeit von deren Reihenfolge unterschiedlich stark verbraucht werden. Möglicherweise werden bei von unterschiedlichen Benutzern geplanten Behandlungen die gleichen Methoden verwendet, so dass es effizient sein kann, die Reservierungen anhand der Methoden zu sortieren. Es ist z.B. denkbar, dass eine bestimmte Substanz und/oder ein bestimmtes Verbrauchsmaterial und/oder ein bestimmtes Werkzeug bei mehreren von unterschiedlichen (oder denselben Benutzern) geplanten Methoden eingesetzt werden. Dann kann es insbesondere effizient sein, diese Substanz bzw. dieses Verbrauchsmaterial bzw. dieses Werkzeug im Laborgerät zu lagern, so dass einige Transportvorgänge überflüssig werden, wodurch Zeit gespart wird, und ggf. auch die Ressource selbst, die oftmals unter sterilen Bedingungen gehalten werden muss. Es wäre z.B. auch möglich, dass zwei im Reservierungszeitplan zeitlich nacheinander vorgesehene Behandlungen sich bestimmte Verbrauchsmaterialien teilen können. Beispielsweise könnte ein und derselbe Lagerbehälter in beiden Behandlungen verwendbar sein, so dass es effizient ist, den Lagerbehälter nach Beendigung der ersten Behandlung für die zweite Behandlung zu verwenden, anstatt zum Ende der ersten Behandlung den ersten Lagerbehälter zu entsorgen und zu Beginn der zweiten Behandlung einen weiteren Lagerbehälter einzusetzen. Darüber hinaus wäre es z.B. auch möglich, zwei separate Reservierungen für eine identische Behandlung zusammen zu legen und in einer Behandlung in einem einzigen Verbrauchsmaterial gemeinsam (Mikrotiterplatte) abzuarbeiten. Dadurch kann in vielen Situationen Material und Zeit gespart werden.

Die Ressource kann auch die Mehrzahl von Laborgeräten sein, auf die die in einem Reservierungszeitraum anfallenden Reservierungen gemäß der mehreren Reservierungsdatensätze mehrerer Benutzer automatisch verteilt werden sollen, um eine optimale Ausnutzung des in einem Labor verfügbaren Parks von Laborgeräten erhalten. Insbesondere kann es Experimente geben, die den zeitlich abgestimmten Einsatz von mehr als einem Laborgerät erfordern. Die Ressource kann deshalb darin bestehen, eine Mehrzahl von Laborgeräten zeitlich optimal zu nutzen, insbesondere unter Berücksichtigung mindestens eines Experiments oder mehrerer Experimente, die jeweils unterschiedliche Laborgeräten benötigen können.

Der Begriff **"Behandlung"** bedeutet, dass eine Laborprobe, die zumeist flüssig ist, bewegt, transportiert, und/oder untersucht und/oder verändert wird, insbesondere in ihrer Zusammensetzung, physikalisch, chemisch, biochemisch oder auf andere Weise verändert wird.

Der Begriff **"gerätegesteuerte Behandlung"** bedeutet, dass die Behandlung der mindestens einen Laborprobe zumindest teilweise vom Laborgerät gesteuert wird, insbesondere durchgeführt wird. Soweit die Behandlung vom Laborgerät gesteuert und/oder durchgeführt wird, wird diese insbesondere insofern nicht vom Benutzer gesteuert und/oder durchgeführt, insbesondere nicht manuell vom Benutzer gesteuert und/oder durchgeführt.

Unter einer gerätegesteuerten Behandlung wird ferner vorzugsweise verstanden, dass die Behandlung in Abhängigkeit von mindestens einer Benutzereingabe zumindest teilweise vom Laborgerät gesteuert wird, insbesondere durchgeführt wird. Die Benutzereingabe kann vor dem Start der Behandlung erfolgen und/oder während der Behandlung. Die Benutzereingabe erfolgt vorzugsweise über eine Benutzerschnittstelleneinrichtung, die vorzugsweise ein Bestandteil des Laborgeräts ist, oder die separat vom Laborgerät vorgesehen ist und mit der Steuereinrichtung des Laborgeräts und/oder der Steuereinrichtung der Zugriffssteuerung signalverbunden ist. Die Benutzereingabe dient insbesondere der Eingabe mindestens eines Parameters, dessen Wert die Behandlung beeinflusst und/oder steuert. Dieser Parameter kann insbesondere ein Programmparameter sein.

Die "gerätegesteuerte Behandlung" bezeichnet insbesondere die zumindest teilautomatisierte Behandlung. Bei einer teilautomatisierten Behandlung ist es insbesondere möglich, dass die Behandlung so durchgeführt wird, dass nach dem Starten der Behandlung und vor dem Beenden der Behandlung mindestens eine Benutzereingabe erfolgt, mit dem der Benutzer die laufende Behandlung beeinflussen kann, insbesondere indem er z.B. eine über eine Benutzerschnittstelleneinrichtung des Laborgeräts erfolgende automatische Abfrage beantwortet, insbesondere durch eine Eingabe bestätigt oder verneint oder andere Eingaben vornimmt. Bei der teilautomatisierten Behandlung ist es insbesondere möglich, dass die Behandlung mehrere Behandlungsschritte aufweist, die insbesondere zeitlich nacheinander automatisch durchgeführt werden, und mindestens einen Behandlungsschritt aufweist, der eine, insbesondere über eine Benutzerschnittstelleneinrichtung erfolgende, Benutzereingabe erfordert.

Eine gerätegesteuerte Behandlung ist vorzugsweise eine **programmgesteuerte Behandlung,** also eine durch ein Programm gesteuerte Behandlung. Unter einer programmgesteuerten Behandlung einer Probe ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mithilfe einer digitalen Datenverarbeitungseinrichtung, die insbesondere Bestandteil der Steuereinrichtung des Laborgeräts sein kann. Die digitale Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich.

Unter einem **Programmparameter** wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Ein Programmparameter kann ein benutzerseitig erforderlicher Programmparameter sein. Ein benutzerseitig erforderlicher Programmparameter zeichnet sich dadurch aus, dass er für die Ausführung einer Behandlung, insbesondere zur Ausführung eines Methodenprogramms, erforderlich ist. Andere Programmparameter, die nicht benutzerseitig erforderlich sind, können aus den benutzerseitig erforderlichen Programmparametern abgeleitet werden oder anderweitig verfügbar gemacht werden, insbesondere wahlweise vom Benutzer eingestellt werden. Die Einstellung eines Programmparameters durch einen Benutzer erfolgt insbesondere durch Anzeige einer Auswahl von möglichen vorgegebenen Werten aus einer im Laborgerät gespeicherten Liste von vorgegebenen Werten, wobei der Benutzer aus dieser Liste den gewünschten Parameter auswählt und somit einstellt. Es ist auch möglich, dass dieser Programmparameter eingestellt wird, indem der Benutzer den Wert eingibt, indem er z.B. über einen Ziffernblock eine numerische Zahl eingibt, die dem gewünschten Wert entspricht oder indem der Benutzer einen Wert kontinuierlich oder in Inkrementen erhöht bzw. erniedrigt, bis dieser dem gewünschten Wert entspricht, und den Wert so einstellt.

Unter einem **Programm** wird insbesondere ein Computerprogramm verstanden. Ein Programm ist eine Folge von Anweisungen, insbesondere bestehend aus Deklarationen und Instruktionen, um auf einer digitalen Datenverarbeitungsanlage eine bestimmte Funktionalität, Aufgaben- oder Problemstellung bearbeiten und/oder lösen zu können. Ein Programm liegt in der Regel als Software vor, die mit einer digitalen Datenverarbeitungsanlage verwendet wird. Das Programm kann insbesondere als Firmware vorliegen, im Fall der vorliegenden Erfindung insbesondere als Firmware der Steuereinrichtung des Laborgeräts und/oder der Zugriffssteuerung. Das Programm liegt meist auf einem Datenträger als ausführbare Programmdatei, häufig im sogenannten Maschinencode vor, die zur Ausführung in den Arbeitsspeicher des Rechners der digitalen Datenverarbeitungsanlage geladen wird. Das Programm wird als Abfolge von Maschinen-, d. h. Prozessorbefehlen von dem/den Prozessoren des Computers verarbeitet und damit ausgeführt. Unter "Computerprogramm" wird insbesondere auch der Quelltext des Programms verstanden, aus dem im Verlauf der Steuerung des Laborgerätes der ausführbare Code entstehen kann.

Als **Anweisung** wird in üblicher Weise ein zentrales Element einer Programmiersprache bezeichnet. Die Programme derartiger Sprachen setzen sich primär aus einer oder mehreren Anweisungen zusammen. Eine Anweisung stellt eine in der Syntax einer Programmiersprache formulierte einzelne Vorschrift dar, die im Rahmen der Abarbeitung des Programms auszuführen ist. Wie eine Anweisung syntaktisch auszusehen hat, wird durch die jeweilige Programmiersprache bzw. deren Spezifikation festgelegt. In der maschinennahen Programmierung werden Anweisungen häufig auch als Befehl bezeichnet. Anweisungen sind üblicherweise Zuweisungen, Kontrollanweisungen (wie Sprünge, Schleifen und bedingte Anweisungen) und Prozeduraufrufe. Abhängig von der Programmiersprache sind teilweise auch Zusicherungen, Deklarationen, Klassen- und Funktionsdefinitionen Anweisungen. Die Anweisungen des Steuerprogramms können so in übliche Weise ausgestaltet sein.

Unter einem **Programmmodul** wird in üblicher Weise eine abgeschlossene funktionale Einheit einer Software verstanden, bestehend aus einer Folge von Verarbeitungsschritten und Datenstrukturen. Dabei können insbesondere folgende Definitionen zutreffen: Der Inhalt eines Moduls ist häufig eine wiederkehrende Berechnung oder Bearbeitung von Daten, die mehrfach durchgeführt werden muss. Module bieten eine Kapselung durch die Trennung von Schnittstelle und Implementierung: Die Schnittstelle eines Moduls definiert die Datenelemente, die als Eingabe und Ergebnis der Verarbeitung durch das Modul benötigt werden. Die Implementierung enthält den tatsächlichen Programmcode. Ein Modul wird zum Beispiel als Funktion oder Unterprogramm aufgerufen, führt eine Reihe von Verarbeitungsschritten durch und liefert als Ergebnis Daten zurück an das aufrufende Programm. Ein Modul kann selbst weitere Module aufrufen - so ist eine Hierarchie von Programmaufrufen möglich. Die in Modulen festgelegten Datenstrukturen und Methoden können gegebenenfalls vererbt und von anderen Modulen geerbt werden. Module sind daher ein wesentliches Element in der strukturierten und objektorientierten Programmierung.

Unter einem **Steuerprogramm** wird ein ausführbares Computerprogramm verstanden, das vorzugsweise die gewünschte Behandlung der mindestens einen Probe steuert und/oder durchführt, insbesondere in Abhängigkeit von mindestens einem Programmparameter. Dieser Programmparameter kann ein vom Benutzer beeinflusster und/oder eingestellter Programmparameter sein. Die Behandlung kann insbesondere gesteuert werden, indem die Steuereinrichtung in Abhängigkeit von den Programmparametern einen oder mehrere Steuerparameter erzeugt, mittels derer die mindestens eine Behandlungseinrichtung gesteuert wird. Vorzugsweise weist das Laborgerät ein Betriebssystem auf, das ein Steuerprogramm sein kann oder aufweisen kann. Das Steuerprogramm kann insbesondere ein Betriebssystem des Laborgeräts bezeichnen oder einen Bestandteil des Betriebssystems. Das Betriebssystem steuert die Behandlung und weitere Betriebsfunktionen des Laborgeräts.

Das Steuerprogramm kann insbesondere mit der Zugriffssteuerung signalverbunden sein, und/oder kann die Zugriffssteuerung steuern. Die Steuereinrichtung der Zugriffssteuerung kann in die Steuereinrichtung des Laborgeräts integriert sein, oder kann getrennt von dieser Steuereinrichtung ausgebildet sein. Die Zugriffssteuerung kann in die Steuereinrichtung des Laborgeräts integriert sein. Die Steuerung der Zugriffssteuerung kann in die Steuerung des Laborgeräts integriert sein, kann vom Steuerprogramm steuerbar sein und/oder kann insbesondere in das Steuerungsprogramm integriert sein. Das Steuerprogramm kann weitere vorzugsweise vorgesehene Funktionen des Laborgeräts steuern, zum Beispiel eine Energiesparfunktion des Laborgeräts oder eine Kommunikationsfunktion zur Kommunikation mit externen Datenverarbeitungseinrichtungen, die insbesondere separat zum Laborgerät vorgesehen sind und insbesondere nicht ein Bestandteil des Laborgeräts sind.

Unter einem **Methodenprogramm** wird ein Programm verstanden, das den konkreten Ablauf einer Behandlung bestimmt, insbesondere gemäß einer vorbestimmten Behandlungsart und/oder gemäß einer benutzerseitig festgelegten Weise.

Die Erfindung betrifft ferner ein **Laborgerät** zur gerätegesteuerten Behandlung mindestens einer Laborprobe, das mindestens eine Behandlungseinrichtung zur Durchführung der Behandlung der mindestens einen Laborprobe und eine erfindungsgemäße Zugriffssteuerung aufweist.

Vorzugsweise weist das Laborgerät eine Kommunikationseinrichtung zur Herstellung einer Datenfernverbindung für den Datenaustausch mit einem externen Gerät auf, das ebenfalls eine geeignete Kommunikationseinrichtung zur Herstellung einer Fernverbindung für den Datenaustausch mit dem Laborgerät aufweist. Eine solche Kommunikationseinrichtung kann zur Ausbildung einer Funkverbindung ausgebildet sein, insbesondere einer Mobilfunkverbindung. Vorzugsweise ist die Kommunikationseinrichtung dazu eingerichtet, den Fernzugriff eines Benutzers auf das Laborgerät, insbesondere die Auswahl oder Einstellung mindestens eines Parameters zu ermöglichen, insbesondere eines Parameters der eine Funktion des Laborgeräts zu steuern, insbesondere die Funktion der Ausführung einer Behandlung.

Vorzugsweise ist die Steuereinrichtung der Zugriffssteuerung oder des Laborgeräts dazu ausgebildet ist, **Synchronisationsdaten** bereitzustellen. Vorzugsweise ist die Zugriffssteuerung, insbesondere die Steuereinrichtung der Zugriffssteuerung, so eingerichtet, dass, wenn mindestens eine Bedingung erfüllt ist, über die Schnittstelleneinrichtung Informationen über den Betriebszustand des Laborgeräts, Messwerte oder durch Benutzer beeinflussbare Einstellungen oder Programmierungen des Laborgeräts an die zweite Benutzerschnittstelleneinrichtung übermittelt werden. Durch die Informationsübermittlung kann das Laborgerät, insbesondere eine dort laufende Behandlung, mittels der zweiten Benutzerschnittstelleneinrichtung weiter beobachtet und/oder gesteuert werden. Insbesondere kann der Verwendungszustand der ersten Benutzerschnittstelleneinrichtung teilweise oder vollständig in die zweite Benutzerschnittstelleneinrichtung kopiert, bzw. geklont werden. Die Informationsübermittlung kann insbesondere ein Synchronisierungsvorgang sein. Die erste und zweite Benutzerschnittstelle können insbesondere auf diese Weise synchronisiert werden. Die mindestens eine andere Bedingung kann sein, dass der Zugriff des zugreifenden Benutzers über eine Datenfernverbindung via einer (mobilen) Benutzerschnittstelleneinrichtung und der Antrag des Benutzers nach einer Synchronisierung erfolgt. Die mindestens eine andere Bedingung kann zudem die Bedingung a) oder b) sein, nämlich die Antwort auf die Prüfung, ob der anmeldende Benutzer vorausgehend bereits über eine erste Benutzerschnittstelleneinrichtung a) eine oder mehrere gerade ausgeführte Funktionen des Laborgeräts aktiviert hatte oder b) schon angemeldet ist. In den Fällen a) und b) würde die Synchronisierung nur einem Benutzer mit aktiver Sitzung und/oder gerade am Laborgerät aktivierten Funktionen, insbesondere laufende Behandlungen, die vom Benutzer initiiert wurden, erlaubt werden. Es ist aber auch möglich und bevorzugt, dass es einem weiteren Benutzer erlaubt werden kann, eine Synchronisierung durchzuführen, z.B. um eine Fernsteuerung zum Zwecke der Assistenz bei einer laufenden Sitzung oder Behandlung durchzuführen, oder Wartungsarbeiten etc.

Vorzugsweise ist die Steuereinrichtung der Zugriffseinrichtung dazu eingerichtet, diese Synchronisationsdaten an eine -insbesondere mobile- Benutzerschnittstelleneinrichtung zu übertragen. Vorzugsweise sind diese Synchronisationsdaten dazu geeignet, die in der Anzeige der Benutzerschnittstelleneinrichtung angezeigten Informationen zumindest teilweise identisch auf der Anzeige der-insbesondere mobilen- Benutzerschnittstelleneinrichtung anzuzeigen.

Der Begriff **Laborgerät** bezeichnet insbesondere ein Gerät, das zur gerätegesteuerten Behandlung mindestens einer Laborprobe ausgebildet ist und das zur Verwendung in einem Labor ausgebildet ist. Bei diesem Labor kann es sich insbesondere um ein chemisches, biologisches, biochemisches, medizinisches oder forensisches Laboratorium handeln. Solche Labors dienen der Forschung und/oder der Analyse von Laborproben, können aber auch zur Herstellung von Produkten mittels Laborproben oder der Herstellung von Laborproben dienen.

Ein Laborgerät ist vorzugsweise eines der folgenden **Laborgeräte** und/oder ist vorzugsweise als mindestens eines der folgenden Laborgeräte ausgebildet: Laborzentrifuge, im Rahmen der Beschreibung der vorliegenden Erfindung auch bezeichnet als "Zentrifuge" oder"centrifuge"; Thermocycler, im Rahmen der Beschreibung der vorliegenden Erfindung auch bezeichnet als "Cycler"; Labor-Spektralphotometer, im Rahmen der Beschreibung der vorliegenden Erfindung auch bezeichnet als "Biospektrometer"; Zellenzählgerät, im Rahmen der Beschreibung der vorliegenden Erfindung auch bezeichnet als "cellcounter", insbesondere optische Zählgeräte; Labor-Inkubator, im Rahmen der Beschreibung der vorliegenden Erfindung auch bezeichnet als "incubator"; Labor-Schüttelgerät, im Rahmen der Beschreibung der vorliegenden Erfindung auch bezeichnet als "shaker"; Labor-Mischer, auch bezeichnet als "mixing device"; Labor-Gefriergerät, im Rahmen der Beschreibung der vorliegenden Erfindung auch bezeichnet als "freezer"; Bioreaktor, im Rahmen der Beschreibung der vorliegenden Erfindung auch bezeichnet als Fermenter; Sicherheitswerkbank, insbesondere Biosicherheitswerkbank, im Rahmen der Beschreibung der vorliegenden Erfindung auch bezeichnet als "biosafety cabinet"; Probenplatten-Lesegerät, im Rahmen der Beschreibung der vorliegenden Erfindung auch bezeichnet als "plate reader", insbesondere "microplate reader"; Laborautomat zur Behandlung von fluiden Proben, insbesondere Pipettierautomat;.

Eine **Laborzentrifuge** ist ein Gerät, das unter Ausnutzung der Massenträgheit arbeitet. Die Laborzentrifuge, insbesondere die Behandlungseinrichtung der Laborzentrifuge, weist insbesondere mindestens einen Rotor auf, indem die mindestens eine Laborprobe anordenbar ist. Der mindestens eine Rotor ist rotierbar in mindestens einem Zentrifugenkessel angeordnet. Die Laborzentrifuge, insbesondere die Behandlungseinrichtung der Laborzentrifuge, weist mindestens eine Antriebseinrichtung auf, mittels der die Rotation angetrieben und/oder gebremst wird. Die Proben sind in dem mindestens einen Rotor anordenbar, vorzugsweise in Laborbehältern, z.B. Probenröhrchen, die in geeigneten Halterungen im Rotor angeordnet werden. Vorzugsweise weist die Laborzentrifuge, insbesondere die Behandlungseinrichtung der Laborzentrifuge, mindestens eine Heiz-/Kühleinrichtung auf, mit der die Temperatur der mindestens einen im Rotor angeordneten Probe gesteuert und/oder geregelt werden kann. Vorzugsweise weist die Laborzentrifuge, insbesondere die Behandlungseinrichtung der Laborzentrifuge, eine Zeitgebereinrichtung auf, mit der zeitliche Parameter der Rotation oder Temperatureinstellung steuerbar sind. Die Funktionsweise beruht auf der Zentrifugalkraft, die aufgrund einer gleichförmigen Kreisbewegung der zu zentrifugierenden Proben zustande kommt. Die Zentrifugalkraft wird zur Stofftrennung von Stoffen unterschiedlicher Dichte genutzt, die in einer Probe enthalten sind. Eine Zentrifuge kann ein Trennverfahren durchführen, bei dem insbesondere die Bestandteile von Suspensionen, Emulsionen und/oder Gasgemischen getrennt werden. Die gerätegesteuerte Behandlung der mindestens einen Laborprobe entspricht bei einer Laborzentrifuge einer Rotationsbehandlung, der die mindestens eine Probe unterzogen wird. Mögliche Parameter, insbesondere Programmparameter, insbesondere Nutzerparameter, die zur Beeinflussung einer Rotationsbehandlung verwendet werden, definieren insbesondere eine Temperatur der Laborzentrifuge, eine Rotationsgeschwindigkeit der Laborzentrifuge, einen zeitlichen Parameter der Rotation oder Temperatureinstellung, und/oder mindestens einen Ablaufparameter, der den Ablauf, insbesondere die Reihenfolge, eines aus mehreren Rotationsschritten bestehenden Rotationsprogramms beeinflusst oder definiert. Die Temperatur der Laborzentrifuge kann insbesondere mindestens eine Temperatur im Inneren des mindestens einen Rotors sein, insbesondere mindestens eine Temperatur mindestens einer Probe.

Ein **Thermocycler** ist ein Gerät, das in der Lage ist, die Temperatur mindestens einer Probe zeitlich nacheinander auf eine vorbestimmte Temperatur einzustellen und für eine vorgegebene Dauer auf dieser Temperaturstufe zu halten. Der Ablauf dieser Temperatursteuerung ist zyklisch. Das heißt ein vorbestimmter Temperaturzyklus, also eine Abfolge von mindestens zwei Temperaturstufen, wird wiederholt durchgeführt. Dieses Verfahren dient insbesondere der Durchführung einer Polymerase-Kettenreaktion (PCR). In diesem Zusammenhang bezeichnet man einen Thermocycler auch manchmal als PCR-Block. Ein Thermocycler, insbesondere die Behandlungseinrichtung des Thermocyclers, weist vorzugsweise einen Thermoblock auf. Ein Thermoblock ist ein Probenhalter aus einem wärmeleitenden Material, meistens ein metallhaltiges Material oder ein Metall, insbesondere Aluminium oder Silber. Der Probenhalter weist eine Kontaktierseite auf, die durch mindestens eine Heiz-/Kühleinrichtung des Thermocyclers, insbesondere ein Peltierelement, kontaktiert wird. Der Thermocycler, insbesondere die Behandlungseinrichtung des Thermocyclers, weist eine Regeleinrichtung mit mindestens einem Regelkreis auf, dem als Stellglied die mindestens eine Heiz-/Kühleinrichtung und als Messglied mindestens eine Temperaturmesseinrichtung zugeordnet sind. Mittels der Regeleinrichtung wird die Temperatur einer Temperaturstufe geregelt. Ein Kühlkörper des Thermocyclers, insbesondere der Behandlungseinrichtung des Thermocyclers, dient zur Kühlung von Abschnitten des Thermocyclers, insbesondere der Kühlung der Peltierelemente. Der Thermocycler, insbesondere die Behandlungseinrichtung des Thermocyclers, kann weitere Heizund/oder Kühlelemente aufweisen. Vorzugsweise weist der Thermocycler, insbesondere die Behandlungseinrichtung des Thermocyclers, eine Zeitgebereinrichtung auf, mit der zeitliche Parameter der Einstellung des Temperaturzyklus steuerbar sind. Die gerätegesteuerte Behandlung der mindestens einen Laborprobe entspricht bei einem Thermocycler einer Temperaturzyklusbehandlung, der die mindestens eine Probe unterzogen wird. Mögliche Parameter, insbesondere Programmparameter, insbesondere Nutzerparameter, die zur Beeinflussung einer Temperaturzyklusbehandlung verwendet werden, definieren insbesondere die Temperatur einer Temperaturstufe, die Dauer einer Temperaturstufe, die Steuerung weiterer Heiz- und/oder Kühlelemente, und/oder die Anzahl von Temperaturstufen oder Zyklen, und/oder mindestens einen Ablaufparameter, der den Ablauf, insbesondere die Reihenfolge, eines aus mehreren Schritten bestehenden Temperaturkontrollprogramms beeinflusst oder definiert.

Ein **Labor-Spektralphotometer** ist ein Gerät, das durch das Beleuchten mindestens eines Messvolumens mindestens einer Laborprobe meist über das gesamte Spektrum von infrarot bis ultraviolett des sichtbaren Lichtes die Remissionswerte ermittelt. Die Remission bezeichnet die Situation, dass ein Messvolumen einen Teil des Lichtspektrums absorbiert und einen Teil des Spektrums transmittiert (transparente Medien) bzw. reflektiert (undurchsichtige Medien). Mit dem Labor-Spektralphotometer wird insbesondere das Absorptionsvermögen einer Probe in Abhängigkeit von der Lichtwellenlänge gemessen. Darüber hinaus besteht insbesondere die Möglichkeit, den Anwendungsbereich des Labor-Spektralphotometers durch verschiedene Module zu erweitern. So ist z.B. die Anordnung eines Fluoreszenz-Moduls zur Messung von Fluoreszenz oder eines Temperiermoduls zur Temperierung der Probe im Spektrometer denkbar. Das gemessene Absorptionsspektrum enthält insbesondere die bei bestimmten Wellenlängen gemessenen Lichtintensitäten. Das Absorptionsspektrum ist charakteristisch für die Laborprobe bzw. den darin enthaltenen Stoff oder die Stoffe. Dies kann zur qualitativen Analyse der Laborprobe genutzt werden. Ist die flüssige Probe bzw. der darin gelöste Stoff bekannt, kann durch Messung der Absorption die Konzentration des gelösten Stoffes ermittelt werden. Dies kann zur quantitativen Analyse der Laborprobe genutzt werden. Das Labor-Spektralphotometer, insbesondere die Behandlungseinrichtung des Labor-Spektralphotometers, weist vorzugsweise mindestens eine Lichtquelle auf, vorzugsweise mindestens einen Zeitgeber, vorzugsweise mindestens einen Photodetektor. Die gerätegesteuerte Behandlung der mindestens einen Laborprobe entspricht bei einem Labor-Spektralphotometer einer Licht- und Messbehandlung, der die mindestens eine Probe unterzogen wird. Mögliche Parameter, insbesondere Programmparameter, insbesondere Nutzerparameter, die zur Beeinflussung einer Licht- und Messbehandlung verwendet werden, definieren insbesondere das optische Lichtspektrum, mit dem die mindestens eine Probe bestrahlt wird und/oder mindestens einen Ablaufparameter, der den Ablauf, insbesondere die Reihenfolge, eines aus mehreren Schritten bestehenden Licht- und Messbehandlungsprogramms beeinflusst oder definiert.

Ein **Zellenzählgerät** dient dem Zählen von biologischen Zellen, bzw. Partikeln, die in einer Laborprobe enthalten sind. Es gibt verschiedene physikalische Prinzipien, die zum Zählen von Zellen in Frage kommen, insbesondere optische Verfahren, bei denen die zu messende Laborprobe in einer Zählkammer angeordnet wird, beleuchtet wird und ein Bild der in der Zählkammer angeordneten Zellen bzw. Partikel erfasst wird und ausgewertet wird. Ein als Coulter Counter ausgeführtes Zellenzählgerät leitet die die Zellen enthaltende Laborprobe durch eine Messschleuse. Jeder Durchtritt einer Zelle durch die Messschleuse wird als zählbares Ereignis elektrisch detektiert. Das Zellenzählgerät, insbesondere die Behandlungseinrichtung des Zellenzählgeräts, weist je nach Ausführung vorzugsweise mindestens eine Lichtquelle auf, mindestens eine Bilderfassungseinrichtung und mindestens eine Bildauswerteeinrichtung, oder eine Kammereinrichtung mit Messschleuse, eine Pumpeneinrichtung und eine elektrische Messeinrichtung. Die gerätegesteuerte Behandlung der mindestens einen Laborprobe entspricht bei einem Zellenzählgerät z.B. einer Licht- und Messbehandlung, oder einer Pump- und Messbehandlung, der die mindestens eine Probe unterzogen wird. Mögliche Parameter, insbesondere Programmparameter, insbesondere Nutzerparameter, die zur Beeinflussung einer Licht- und Messbehandlung oder der Pump- und Messbehandlung verwendet werden, definieren insbesondere die Lichtintensität der Lichtquelle, mit dem die mindestens eine Probe bestrahlt wird und/oder mindestens einen Ablaufparameter, der den Ablauf, insbesondere die Reihenfolge, eines aus mehreren Schritten bestehenden Licht- und Messbehandlungsprogramms oder Pump- und Messbehandlungsprogramms beeinflusst oder definiert.

Ein **Zellenzählgerät** dient dem Zählen von biologischen Zellen, bzw. Partikeln, die in einer Laborprobe enthalten sind. Es gibt verschiedene physikalische Prinzipien, die zum Zählen von Zellen in Frage kommen, insbesondere optische Verfahren, bei denen die zu messende Laborprobe in einer Zählkammer angeordnet wird, insbesondere bei automatisch arbeitenden zusätzlich beleuchtet wird und ein Bild der in der Zählkammer angeordneten Zellen bzw. Partikel erfasst wird und ausgewertet wird. Ein weiteres etabliertes Verfahren ist die Impedanzmessung: ein als Coulter Counter ausgeführtes Zellenzählgerät leitet die die Zellen enthaltende Laborprobe durch eine Apertur ("Messschleuse"). Jeder Durchtritt einer Zelle durch die Apertur wird als zählbares Ereignis elektrisch detektiert. Optische Zellenzählgeräte, insbesondere die Behandlungseinrichtung des Zellenzählgeräts, weisen je nach Ausführung vorzugsweise mindestens eine Lichtquelle auf, mindestens eine Bilderfassungseinrichtung und mindestens eine Bildauswerteeinrichtung, zusätzlich u.U. eine Positioniereinrichtung. Die gerätegesteuerte Behandlung der mindestens einen Laborprobe entspricht bei einem optischen Zellenzählgerät z.B. einer Licht- und Messbehandlung, bei einem nach Coulter Prinzip arbeitenden Gerät einer Pump- und Messbehandlung, der die mindestens eine Probe unterzogen wird. Mögliche Parameter, insbesondere Programmparameter, insbesondere Nutzerparameter, die zur Beeinflussung einer Licht- und Messbehandlung oder der Pump- und Messbehandlung verwendet werden, definieren insbesondere die Lichtintensität der Lichtquelle, mit dem die mindestens eine Probe bestrahlt wird und/oder mindestens einen Ablaufparameter, der den Ablauf, insbesondere die Reihenfolge, eines aus mehreren Schritten bestehenden Licht- und Messbehandlungsprogramms oder Pump- und Messbehandlungsprogramms beeinflusst oder definiert. Bei optischen Zählgeräten sind außerdem die für die Bildauswertung notwendigen Algorithmen, deren Reihenfolge und Parametrierung entscheidend für die Aussagekraft des Messergebnisses. Optische Messgeräte, aber auch Coulter Counter, nutzen oft Zählkammern zum einmaligen Gebrauch ("Consumables)", dies sind den herkömmlichen Neubauerzählkammern nachempfundene Kunststoffartikel, bzw., bei den Coulter Countern, "Lab-on-a-Chip"- ähnliche Einmalzählkammern. Es gibt aber auch Geräte, die ohne diese Verbrauchsmaterialien arbeiten (z.B. "CASY").

Ein **Labor-Inkubator** ist ein Gerät, mit dem kontrollierte Klimabedingungen für verschiedene biologische Entwicklungs- und Wachstumsprozesse geschaffen und erhalten werden können. Er dient der Schaffung und Erhaltung eines Mikroklimas mit geregelten Gas-, und/oder Luftfeuchtigkeits- und/oder Temperatur-Bedingungen in einem Inkubatorraum, wobei diese Behandlung zeitabhängig sein kann. Der Labor-Inkubator, insbesondere die Behandlungseinrichtung des Labor-Inkubators, weist insbesondere einen Zeitgeber auf, insbesondere eine Zeitschaltuhr, eine Heiz-/Kühleinrichtung und vorzugsweise eine Einstellung für die Regelung eines dem Inkubatorraum zugeführten Austauschgases, insbesondere Frischluft, eine Einstelleinrichtung für die Zusammensetzung des Gases im Inkubatorraum des Labor-Inkubators, insbesondere zur Einstellung des $CO_2$ und/oder des $O_2$ Gehalts des Gases und/oder eine Einstelleinrichtung zur Einstellung der Luftfeuchtigkeit im Inkubatorraum des Labor-Inkubators. Der Labor-Inkubator, insbesondere die Behandlungseinrichtung des Labor-Inkubators, weist insbesondere den Inkubatorraum auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. $CO_2$-Inkubatoren dienen insbesondere der Kultivierung tierischer bzw. humaner Zellen. Inkubatoren können Wendevorrichtungen zum Wenden der mindestens einen Laborprobe und/oder eine Schütteleinrichtung zum Schütteln bzw. Bewegen der der mindestens einen Laborprobe aufweisen. Die gerätegesteuerte Behandlung der mindestens einen Laborprobe entspricht bei einem Labor-Inkubator einer Klimabehandlung, der die mindestens eine Probe unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, den $O_2$- und/oder $CO_2$-Partialdruck im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Ein **Labor-Schüttelgerät** dient der Bewegung einer Laborprobe, insbesondere zum Mischen einer mehrere Bestandteile aufweisenden Laborprobe. Labor-Schüttelgeräte gibt es in verschiedenen Ausführungen, insbesondere als Überkopf-Schüttelgeräte oder als Flachbett-Schüttelgeräte. Labor-Schüttelgeräte können eine Temperierfunktion zum Temperieren der mindestens einen Laborprobe aufweisen, und können insbesondere eine Inkubatorfunktion zum Inkubieren der mindestens einen Laborprobe bei kontrollierten Klimabedingungen aufweisen. Labor-Schüttelgeräte, insbesondere deren Behandlungseinrichtung, können insbesondere zur Durchführung einer oszillierenden Bewegung eingerichtet sein. Labor-Schüttelgeräte, insbesondere deren Behandlungseinrichtung, weisen insbesondere einen Antrieb zum Antreiben der Bewegung auf, weisen insbesondere eine Zeitgebereinrichtung auf, mit der zeitliche Parameter der Einstellung der Schüttelbehandlung steuerbar sind und weisen insbesondere mindestens eine Heiz-/Kühleinrichtung und mindestens eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Die gerätegesteuerte Behandlung der mindestens einen Laborprobe entspricht bei einem Labor-Schüttelgerät einer Schüttelbehandlung, der die mindestens eine Probe unterzogen wird. Mögliche Parameter, insbesondere Programmparameter, insbesondere Nutzerparameter, die zur Beeinflussung einer Schüttelbehandlung verwendet werden, definieren insbesondere die Bewegungsintensität, insbesondere die Bewegungsfrequenz bei einem oszillierenden Antrieb, einer Zeitperiode bei der Schüttelbehandlung, und/oder mindestens einen Ablaufparameter, der den Ablauf, insbesondere die Reihenfolge, eines aus mehreren Schritten bestehenden Schüttelbehandlungsprogramms beeinflusst oder definiert.

Ein **Labor-Mischer,** auch bezeichnet als "mixing device", dient wie das Labor-Schüttelgerät der Bewegung einer Laborprobe, insbesondere zum Mischen einer mehrere Bestandteile aufweisenden Laborprobe. Im Vergleich zu einem Labor-Schüttelgerät ermöglicht ein Labor-Mischer Bewegungen mit höheren Frequenzen, insbesondere höheren Drehzahlen. Labor-Mischer, insbesondere deren Behandlungseinrichtung, können insbesondere zur Durchführung einer oszillierenden Bewegung eingerichtet sein. Labor-Mischer, insbesondere deren Behandlungseinrichtung, weisen insbesondere einen Antrieb zum Antreiben der Bewegung auf, weisen insbesondere eine Zeitgebereinrichtung auf, mit der zeitliche Parameter der Einstellung der Mischbehandlung steuerbar sind und weisen insbesondere mindestens eine Heiz-/Kühleinrichtung und mindestens eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Die gerätegesteuerte Behandlung der mindestens einen Laborprobe entspricht bei einem Labor-Mischer einer Mischbehandlung, der die mindestens eine Probe unterzogen wird. Mögliche Parameter, insbesondere Programmparameter, insbesondere Nutzerparameter, die zur Beeinflussung einer Mischbehandlung verwendet werden, definieren insbesondere die Bewegungsintensität, insbesondere die Bewegungsfrequenz bei einem oszillierenden Antrieb, einer Zeitperiode bei der Mischbehandlung, und/oder mindestens einen Ablaufparameter, der den Ablauf, insbesondere die Reihenfolge, eines aus mehreren Schritten bestehenden Mischbehandlungsprogramms beeinflusst oder definiert.

Ein **Labor-Gefriergerät** dient der Lagerung mindestens einer Laborprobe in einem Gefrierraum bei geregelten Temperaturen insbesondere im Tiefkühlbereich von -18° C bis -50 °C oder im Ultratiefkühlbereich von -50° C bis - 90° C. Ein Labor-Gefriergerät ist insbesondere kein Kühlschrank, der zum Kühlen bei Temperaturen insbesondere im Bereich von 0° C bis 10° C oder von - 10° C bis 10 °C verwendbar ist. Ein Labor-Gefriergerät, insbesondere die Behandlungseinrichtung des Labor-Gefriergeräts, weist insbesondere mindestens eine Kühleinrichtung und mindestens eine Regeleinrichtung mit mindestens einem Regelkreis, dem als Stellglied die mindestens eine Kühleinrichtung und als Messglied mindestens eine Temperaturmesseinrichtung zugeordnet sind. Ein Labor-Gefriergerät, insbesondere die Behandlungseinrichtung des Labor-Gefriergeräts, weist insbesondere ein Kontrollmessgerät zur Temperaturmessung und/oder insbesondere eine Alarmeinrichtung auf, mit der ein Alarmsignal ausgegeben wird, wenn die im Gefrierraum gemessene Temperatur einen erlaubten Temperaturbereich verlässt. Ein Labor-Gefriergerät, insbesondere die Behandlungseinrichtung des Labor-Gefriergeräts, kann insbesondere eine Informationsleseeinrichtung zum Lesen der Information aufweisen. Diese Information kann auf einem Informationsträger enthalten sein, der mit einem Artikel verbunden sein kann. Dieser Artikel kann insbesondere ein Probenbehälter, insbesondere ein Aufbewahrungsbehälter, sein, der mindestens eine Laborprobe enthalten kann. Der Informationsträger kann insbesondere einen RFID-Chip oder andere Identifikationsmerkmale aufweisen, wie z.B. einen Barcode, einen Datamatrix Code, einen QR-Code, die mit geeigneten Verfahren lesbar sind. Die gerätegesteuerte Behandlung der mindestens einen Laborprobe entspricht bei einem Labor-Gefriergerät einer Tieftemperaturbehandlung, der die mindestens eine Probe unterzogen wird. Mögliche Parameter, insbesondere Programmparameter, insbesondere Nutzerparameter, die zur Beeinflussung einer Tieftemperaturbehandlung verwendet werden, definieren insbesondere die Temperatur des Gefrierraums, in dem die mindestens eine Probe tiefgekühlt wird und/oder den Informationslesevorgang, der vorzugsweise durchgeführt wird, wenn ein mit einem Informationsträger versehener Artikel von einem Benutzer in das Labor-Gefriergerät überführt wird. Es ist auch möglich und bevorzugt, dass das erfindungsgemäße Laborgerät kein Labor-Gefriergerät ist.

Ein **Bioreaktor** weist einen Behälter auf, in dem bestimmte Mikroorganismen, Zellen, Algen oder Pflanzen, zB. Moose, unter möglichst optimalen Bedingungen kultiviert (auch: fermentiert) werden. Der Betrieb eines Bioreaktors ist somit eine Anwendung der Biotechnologie, die biologische Prozesse, insbesondere Biokonversion oder Biokatalyse, in technischen Einrichtungen nutzt bzw. nutzbar macht. Faktoren, die in den meisten Bioreaktoren insbesondere durch Einstellung entsprechender Parameter steuerbar oder kontrollierbar sind, sind die Zusammensetzung des Nährmediums, die Sauerstoffzufuhr, Temperatur, pH-Wert, Sterilität und/oder andere Faktoren. Zweck der Kultivierung in einem Bioreaktor kann die Gewinnung der Zellen oder von Bestandteilen der Zellen oder die Gewinnung von Stoffwechselprodukten sein. Diese können z. B. als Wirkstoff in der pharmazeutischen oder als Grundchemikalie in der chemischen Industrie verwendet werden. Auch der Abbau von chemischen Verbindungen kann in Bioreaktoren stattfinden, wie z. B. bei der Abwasserreinigung in Kläranlagen. Die Herstellung von Bier, Wein und anderen derartigen Produkten findet ebenfalls in Bioreaktoren statt. In Bioreaktoren werden unterschiedlichste Organismen für verschiedene Zwecke kultiviert. Ein Bioreaktor kann deshalb unterschiedlich ausgeführt sein. Er kann als Rührkesselreaktor aus Metall ausgeführt sein, der ein Volumen von wenigen Millilitern bis hunderten Litern haben kann und mit Nährlösung gefüllt werden kann. Er kann auch als Festbettreaktor, Photobioreaktor verwendet werden bzw. ausgebildet sein. Ein Bioreaktor kann insbesondere auch als Einweg-Bioreaktor ausgebildet sein, bei dem der Reaktorbehälter, insbesondere inklusive Rührer, aus Kunststoff besteht, beispielsweise Celligen® BLU Einweg-Bioreaktor, Eppendorf AG. Ein Bioreaktor kann Teil eines Bioreaktor-Systems sein, vorzugsweise eines parallelen Bioreaktor-Systems. In einem solchen parallelen Bioreaktor-System wird eine Vielzahl von Bioreaktoren parallel betrieben und mit höherer Präzision kontrolliert. Ein Bioreaktor, insbesondere dessen Behandlungseinrichtung, weist insbesondere eine Rühreinrichtung auf zum Rühren der im Reaktorbehälter enthaltenen Probe, insbesondere des Nährmediums. Ein Bioreaktor, insbesondere dessen Behandlungseinrichtung, weist insbesondere eine Pumpeinrichtung zum Pumpen der vorzugsweise als Nährmedium ausgeführten Laborprobe auf. Ein Bioreaktor, insbesondere dessen Behandlungseinrichtung, weist insbesondere eine Einstelleinrichtung für die Einstellung eines Gasgehalts im Reaktorbehälter, insbesondere des Gehalts an $CO_2$ und/oder $O_2$ bzw. an Gelöstsauerstoff (DO) auf. Ein Bioreaktor, insbesondere dessen Behandlungseinrichtung, weist insbesondere eine Einstelleinrichtung für die Einstellung, insbesondere Regelung, eines pH-Werts in der Probe im Reaktorbehälter auf. Die gerätegesteuerte Behandlung der mindestens einen Laborprobe entspricht bei einem Bioreaktor insbesondere einer Nährmediumsbehandlung, der die mindestens eine vorzugsweise als Nährmedium ausgeführte Probe unterzogen wird. Mögliche Parameter, insbesondere Programmparameter, insbesondere Nutzerparameter, die zur Beeinflussung einer Nährmediumsbehandlung verwendet werden, definieren insbesondere die Temperatur des Nährmediums im Reaktorbehälter, und/oder die Geschwindigkeit der Rühreinrichtung, insbesondere Rotationsgeschwindigkeit, und/oder die Pumpgeschwindigkeit bzw. Dosiergeschwindigkeit, und/oder einen Gasgehalt im Nährmedium, insbesondere $CO_2$ und/oder $O_2$ bzw. Gelöstsauerstoff (DO), und/oder den pH-Wert des Nährmediums, und/oder mindestens einen Ablaufparameter, der den Ablauf, insbesondere die Reihenfolge, eines aus mehreren Schritten bestehenden Nährmediumsbehandlungsprogramms beeinflusst oder definiert.

Eine **Sicherheitswerkbank** dient zur sicheren Lagerung oder Aufbewahrung von Gefahrenstoffen, insbesondere zur Erfüllung einer biologischen Schutzstufe. Diese Stufen sind insbesondere in der EU-Richtlinie 2000/54/EG über den Schutz der Arbeitnehmer gegen Gefährdung durch biologische Arbeitsstoffe bei der Arbeit normiert und in der Biostoffverordnung in Deutschland. Eine Sicherheitswerkbank soll verhindern, dass im Falle der Gefahrenentstehung bei in einer Sicherheitswerkbank gelagerten Laborproben die Umwelt gefährdet wird. Die Sicherheit wird insbesondere dadurch gewährleistet, dass die im Aufnahmebereich der Sicherheitswerkbank enthaltene Atmosphäre ausgetauscht und insbesondere gefiltert wird. Dabei wird insbesondere diese Atmosphäre von einer Fördereinrichtung durch den Aufnahmebereich gefördert und durch einen Filter bewegt, der die Atmosphäre filtert, insbesondere von Gefahrstoffen reinigt. Die Sicherheitswerkbank, insbesondere deren Behandlungseinrichtung, weist insbesondere eine Fördereinrichtung zum Befördern von Atmosphärengas auf, weist insbesondere eine Zeitgebereinrichtung zur Messung einer Filterbetriebsdauer und einer Lüfterbetriebsdauer auf und/oder weist insbesondere eine Messeinrichtung zur Messung einer geförderten Menge an Atmosphärengas auf. Die gerätegesteuerte Behandlung der mindestens einen Laborprobe entspricht bei einer Sicherheitswerkbank insbesondere einer Atmosphärengasbehandlung zur Behandlung des Atmosphärengases, in dem die mindestens eine Probe gelagert ist. Mögliche Parameter, insbesondere Programmparameter, insbesondere Nutzerparameter, die zur Beeinflussung einer Atmosphärengasbehandlung verwendet werden, definieren insbesondere die Temperatur des Atmosphärengases im Aufnahmebereich, und/oder die Strömungsgeschwindigkeit des von der Fördereinrichtung beförderten Atmosphärengases, die geförderte Luftmenge, die Filterbetriebsdauer, und/oder die Lüfterbetriebsdauer.

Ein **Probenplatten-Lesegerät,** auch bezeichnet als "plate reader" oder "microplate reader", ist ein Laborgerät zum Nachweis biologischer, chemischer oder physikalischer Ereignisse von Proben in Mikrotiterplatten. Sie sind weit verbreitet in der Forschung verwendet, Wirkstoffforschung, Bioassay Validierung, Qualitätskontrolle und Fertigungsprozesse in der pharmazeutischen und biotechnologischen Industrie und akademischen Organisationen. Das Probenplatten-Lesegerät kann insbesondere mindestens eine Lichtquelle oder Strahlungsquelle aufweisen, kann mindestens einen Photodetektor aufweisen, kann eine Temperaturkontrolleinrichtung zu Temperierung der Proben bzw. der Probenplatten aufweisen, kann einen Zeitgeber aufweisen. Probenreaktionen können in 6-1536 Well-Format Mikrotiterplatten getestet werden. Das häufigste Format für Probenplatten, insbesondere Mikrotiterplatten, das in akademischen Forschungslabors oder klinisch-diagnostische Laboratorien verwendet wird, ist eine 96-well plate (eine 8 mal 12 Matrix) mit einem typischen Einzelvolumen zwischen 100 und 200 $\mu$l pro Well. Mikrotiterplatten mit höherer Dichte (384 - oder 1536-Well-Mikroplatten) werden typischerweise für Screening-Anwendungen verwendet, wenn Durchsatz (Anzahl der pro Tag verarbeiteten Proben) und Assay-Kosten pro Probe zu kritischen Parametern werden, mit einem typischen Assay-Volumen zwischen 5 und 50 $\mu$l pro Well. Die Behandlung ist insbesondere eine optische Messung der Mikrotiterplatte, insbesondere die Messung einer Absorption, Fluoreszenz-Intensität, Lumineszenz, zeitaufgelöste Fluoreszenz, und/oder Fluoreszenzpolarisation. Mögliche Parameter, insbesondere Programmparameter, insbesondere Nutzerparameter, die zur Beeinflussung einer Messung verwendet werden, definieren z.B. die Intensität der Lichtquelle, die Sensitivität eines Photodetektors, eine Zeitdauer und/oder eine Temperatur.

Ein **Laborautomat zur Behandlung von fluiden Proben,** insbesondere Pipettierautomat, dient der programmgesteuerten Behandlung dieser Proben. Ein Laborautomat kann ein Laborgerät sein oder mindestens ein Laborgerät der vorgenannten Art aufweisen, und/oder kann zur Durchführung mindestens einer, mehrerer oder aller der von diesem vorgenannten Laborgerät ausführbaren Behandlungen ausgebildet sein. Ein Laborautomat weist die Behandlungseinrichtung zur automatischen, programmgesteuerten Behandlung der mindestens einen Laborprobe auf, wobei die Behandlung unter Verwendung von mehreren Programmparametern, die zumindest teilweise vom Benutzer gewählt werden, gesteuert wird. Dabei kann die Probe vom Laborautomaten, bzw. einer Behandlungseinrichtung des Laborautomaten, beispielsweise bewegt und/oder transportiert werden. Die Bewegung kann durch Transport in beweglichen Probenbehältern oder durch die Leitung in Schlauchsystemen, Kapillaren oder Pipettenspitzen erfolgen. Dabei werden flüssige Proben insbesondere durch Ansaugen, also Pipettieren, oder allgemeiner, durch Anlegen von Druckdifferenzen transportiert. Durch eine Behandlung der Probe kann eine Probe z.B. geteilt oder verdünnt werden. Die Inhaltsstoffe einer Probe können analysiert werden oder es können, z.B. durch eine chemische Reaktion, neue Inhaltsstoffe hergestellt werden, insbesondere unter Verwendung der Probe. Insbesondere im Zusammenhang mit der Bearbeitung und Analyse von DNA oder RNA oder deren Bestandteilen sind Laborautomaten hilfreich, um eine Fülle von Informationen innerhalb einer geeigneten Zeitspanne zu gewinnen oder viele solcher Proben zu analysieren. Diese Behandlungseinrichtung eines Laborautomaten weist meist eine Arbeitsfläche mit Arbeitsstationen auf, an denen Proben in verschiedener Weise bearbeitet oder gelagert werden können. Für den Transport von z.B. flüssigen Proben zwischen verschiedenen Positionen, insbesondere Probenbehältern, weist die Behandlungseinrichtung meist eine gerätegesteuerte Bewegungsvorrichtung und eine gerätegesteuerte Fluid-Transfer-Einrichtung, die z.B. ein Pipettiersystem aufweisen kann. Sowohl der Transport der Proben als auch deren Behandlung an den verschiedenen Stationen lässt sich gerätegesteuert durchführen, insbesondere programmgesteuert durchführen. Die Behandlung erfolgt dann vorzugsweise zumindest teilweise oder vollständig automatisiert.

Vorzugsweise kann der Benutzer des Laborautomaten die Art der Behandlung der Probe festlegen. Eine solche Behandlungsart kann insbesondere dienen:

der Nukleinsäureaufreinigung, insbesondere

- "MagSep Blood gDNA": Aufreinigung von genomischer DNA aus Vollblut, insbesondere unter Verwendung des Eppendorf ® MagSep Blood gDNA Kits;
- "MagSep Tissue gDNA": Aufreinigung von genomischer DNA aus lebendem Gewebe, insbesondere unter Verwendung des Eppendorf ® MagSep Tissue gDNA Kits;
- "MagSep Viral DANN/RNA": Aufreinigung von viraler RNA oder DNA aus zellfreien Körperflüssigkeiten, insbesondere unter Verwendung des Eppendorf ® MagSep Viral DNA/RNA Kits;

und PCR-Anwendungen, insbesondere

- "Compose Mastermix";
- "Normalize Concentrations";
- "Create Dilution Series";
- "Setup Reactions".

Ein **Laborgerät,** insbesondere der Laborautomat, ist vorzugsweise so ausgebildet, dass die Steuerung der Behandlung der mindestens einen Laborprobe automatisch unter Verwendung der erfassten Programmparameter erfolgen kann. Das Laborgerät, insbesondere der Laborautomat,, insbesondere dessen Steuerungsprogramm ist vorzugsweise so ausgebildet, dass die vom Benutzer vorgenommenen Eingaben, insbesondere der mindestens eine Wert mindestens eines Programmparameters, dazu verwendet werden, um gegebenenfalls weitere erforderliche Programmparameter automatisch zu ermitteln, insbesondere durch Berechnung oder durch Vergleich mit Daten in einer Datenbank des Laborgerätes. Insbesondere werden vorzugsweise die zur Durchführung der Behandlung im Einzelnen vorzugsweise zu verwendenden Steuerparameter automatisch bestimmt. Durch diese Maßnahmen wird die Bedienung des Laborgerätes komfortabel, der Benutzer erspart sich insbesondere das Entwerfen eines Programmcodes, da diese Schritte insbesondere automatisch vom Laborgerät, insbesondere Laborautomat, durchgeführt werden. In einer bevorzugten Ausgestaltung werden vom Benutzer nur die Eingaben abgefordert, die direkt mit der durchzuführenden Behandlung der Proben zu tun haben. Das sind häufig dieselben Angaben, die auch für eine manuelle Durchführung der Behandlung notwendig wären und dem Benutzer geläufig sind. Dagegen müssen solche Parameter, die die Steuerung des Laborgerätes, insbesondere des Laborautomaten, betreffen, insbesondere die Steuerparameter, nicht im Einzelnen festgelegt werden, da diese vorzugsweise automatisch festgelegt werden. Steuerparameter sind die zur Steuerung der technischen Bestandteile der Behandlungseinrichtung im Einzelnen erforderlichen Parameter. Steuerparameter können Programmparameter sein oder können daraus für die technische Umsetzung abgeleitete Parameter sein, insbesondere automatisch bestimmte Parameter sein.

Vorzugsweise wählt ein Laborgerät, insbesondere der Laborautomat, ausgehend von der vom Benutzer gewählten Behandlungsart automatisch den passenden Satz von Programmparametern aus, dessen benutzerseitig erforderliche Programmparameter dann in den Schritten (b) und (c) vom Benutzer abgefragt wird. Der Programmparametersatz kann einerseits die benutzerseitig erforderlichen Programmparameter enthalten, und kann andererseits weitere Programmparameter enthalten. Diese weiteren Programmparameter können in Abhängigkeit von der gewählten Behandlungsart automatisch festgelegt werden, oder können in Abhängigkeit von mindestens einem, oder allen, vom Benutzer eingegebenen Programmparametern automatisch festgelegt werden, und/oder können in der Speichereinrichtung gespeichert sein. Die gespeicherten Pararametersätze sind -oder werden vom Laborgerät, insbesondere Laborautomat, - vorzugsweise für die Behandlungsart optimiert, so dass der Benutzer vorzugsweise kein Spezialwissen zur Optimierung der Parameter benötigt. Aus dem Programmparametersatz werden die Steuerparameter abgeleitet, die zur Durchführung der konkreten Behandlung mittels der Behandlungseinrichtung notwendig sind.

Für eine Behandlungsart ist vorzugsweise ein Programmparametersatz von für diese Behandlungsart spezifischen Programmparametern definiert. Die Programmparameter dieses Programmparametersatzes können insbesondere die für die Behandlung zu verwendenden Zubehörteile, z.B. Probenbehältnis, Transportbehältnis, und/oder die zu verwendenden Tools, und/oder weiteres Verbrauchsmaterial definieren.

Die Zuordnung von Programmparametersatz und Behandlungsart ist in der Speichereinrichtung des Laborgerätes, insbesondere des Laborautomaten, gespeichert. Vorzugsweise ist das ein Laborgerät, insbesondere der Laborautomat, dazu ausgebildet, dass der Benutzer weiterer solcher Zuordnungen im ein Laborgerät, insbesondere Laborautomat, speichern und/oder verwenden kann. Durch diese Zuordnung, in Kombination mit der übersichtlichen und gut strukturierten Abfrage der Programmparameter wird die Bedienung des Laborgeräts besonders effizient. Diese Zuordnung erfolgt vorzugsweise durch die Verwendung eines oder mehrere Programmmodule, wobei jeweils ein Programmmodul auf eine bestimmte Anwendung zugeschnitten ist:

Die Behandlung einer Laborprobe(n) kann einen oder mehrere der nachfolgend genannten Vorgänge, insbesondere gleichzeitig oder nacheinander, beinhalten:

- Transport der Laborprobe, insbesondere durch eine Transporteinrichtung, unter Wirkung der Gravitation und/oder einer durch das Laborgerät, insbesondere den Laborautomaten, bewirkten Kraft;

- berührungslose (nicht-invasive) physikalische Behandlung der Probe, insbesondere thermische Behandlung, insbesondere Erwärmen und/oder Kühlen, insbesondere geregeltes Temperieren der Probe; oder Gefrieren oder Auftauen der Probe, oder sonstiges thermisches Herbeiführen einer Phasenänderung der Probe, z.B. Verdampfen, Kondensieren etc; magnetische Behandlung der Probe; optische Behandlung der Probe, insbesondere Bestrahlen der Probe mit Strahlung, insbesondere Licht, insbesondere sichtbare Licht. Infrarotlicht oder UV-Licht, oder Detektion von solcher Strahlung, insbesondere Fluoreszenzlicht, aus dieser Probe; magnetische Behandlung einer Probe mit magnetischen Bestandteilen, insbesondere magnetische Separation von magnetischen Bestandteilen, insbesondere "magnetic beads", von einer fluiden Phase der Probe; Bewegen der Probe, also Durchführen einer mechanischen Behandlung der Probe, insbesondere Schütteln, rotieren, oszillieren, vibrieren, zentrifugieren, akustische Behandlung, insbesondere mit Ultraschall, jeweils z.B. zum Zwecke des Mischens der Probe oder des Separierens von Bestandteilen innerhalb der Probe oder zum Transportieren der magnetischen Bestanteile aus der Probe heraus oder in die Probe hinein;

- invasive physikalische Behandlung der Probe, also Durchführen einer mechanischen Behandlung der Probe: Einbringen von Rührwerkzeug, z.B. Rührstab oder magnetischer Rührfisch in die Probe und Rühren, Einbringen einer Sonde für akustische oder Ultraschalbehandlung, Einbringen von Transportmitteln, insbesondere Transportbehältnissen in die Probe, z.B. Dispenserspitze oder Pipettenspitze, oder Hohlnadel oder Schlauch; Zugabe von anderen Hilfsmitteln in die Probe;

- chemische, biochemische oder biomedizinische Behandlung der Probe: Zugabe von chemischen (z.B. Reaktant, Reagenz, Solvens, Solut) biochemischen (z.B. biochemische Makromoleküle, z.B. DNA, DNA-Bestandteile; pharmazeutische Wirkstoffe), oder biomedizinischen (Blut, Serum, Zellmedium) Stoffen;

- Lagerung der Probe, insbesondere für einen programmgesteuert definierten Zeitraum, insbesondere unter bestimmten physikalische Bedingungen, z.B. bei bestimmter Temperatur, Temperaturen, oder Temperaturwechseln, insbesondere wiederholten Temperaturwechseln, z.B. zyklisch und/oder periodisch wiederholten Temperaturwechseln, und/oder Einstellen eines Umgebungsdruck, z.B. Anlegen eines Überdrucks oder eines Unterdrucks, insbesondere eines Vakuums, und/oder Einstellen einer definierten Umgebungsatmosphäre, z.B. ein Schutzgas oder eine bestimmte Luftfeuchtigkeit, unter bestimmten Strahlungsbedingungen, z.B. abgeschirmt gegen sichtbares Licht, im Dunkeln, oder unter definierter Bestrahlung;

- Messung oder Analyse der Probe, insbesondere Analyse durch eine nicht-invasive und/oder invasive Behandlung der Probe, insbesondere um mindestens eine oder mehrere chemische, physikalische, biochemische und/oder medizinische Eigenschaften der Probe zu messen; insbesondere Zählen von Zellen mittels Cell-Counter;

- Bearbeitung der Probe, insbesondere Änderungen mindestens einer Eigenschaft der Probe, insbesondere mittels nicht-invasiver und/oder invasiver Behandlung der Probe.

Diese Behandlung erfolgt insbesondere programmgesteuert, unter Verwendung mindestens eines Programmparameters.

Diese Behandlung erfolgt insbesondere gemäß mindestens einem Steuerparameter, der die Behandlung der Laborprobe mittels der Behandlungseinrichtung bestimmt. Ein Steuerparameter kann einen Zeitraum festlegen, einen Zeitpunkt, ein bestimmtes Probenvolumen und/oder Dosiervolumen, eine bestimmte Probentemperatur, etc. Ein Steuerparameter kann die automatische Verwendung eines bestimmten Transportkopfes, eines bestimmten Typs eines Transportbehältnisses, eines bestimmten Typs von Probebehältnissen, einer oder mehrerer individuelle Proben oder von bestimmten Positionen dieser Komponenten im Arbeitsbereich betreffen. Ein Steuerparameter kann die Behandlung einer individuellen Probe betreffen oder die Behandlung mehrerer oder vieler Proben.

Vorzugsweise wird ein Steuerparameter in Abhängigkeit von mindestens einem Programmparameter automatisch vom Laborgerät, insbesondere Laborautomat, ausgewählt, insbesondere automatisch in Abhängigkeit von den vom Benutzer ausgewählten Programmparametern automatisch ausgewählt. Dadurch hat der Benutzer den Vorteil, dass er nicht alle Steuerparameter einzeln bestimmen muss. Der Benutzer muss keine Kenntnisse über die Programmierung des Laborgeräts besitzen. Vielmehr erfolgt die Auswahl der für die Behandlung notwendigen Steuerparameter mittels der vom Benutzer eingegebenen Porgrammparameter. Dadurch ist die Verwendung des Laborgeräts besonders komfortabel.

Ein Steuerparameter kann auch einem Programmparameter entsprechen.

Der Transport einer Probe kann ein Transport aus einem Probenbehältnis in ein Transportbehältnis und/oder aus dem Transportbehältnis in ein Probenbehältnis oder einen sonstigen Zielort sein. Dieser Transport erfolgt insbesondere programmgesteuert, unter Verwendung mindestens eines Programmparameters.

Das **Transportbehältnis** kann z.B. ein Dispenserbehältnis sein, das einen beweglichen Kolben und eine Einlass-/Auslassöffnung aufweist. Der Kolben erzeugt einen Unterdruck bzw. Überdruck im Dispenserbehältnis und saugt so die Probe in das Behältnis hinein oder gibt sie wieder ab. Dieser Vorgang folgt dem Verdrängerprinzip, d.h. die zu bewegende, meist flüssige und somit inkompressible Probe wird zwangsbewegt, indem das zuvor von der Probe eingenommene Volumen vom Kolben bewegt wird. Der Kolben wird in der Regel von einer Bewegungseinrichtung bewegt, insbesondere programmgesteuert bewegt, die dem Laborautomaten zugeordnet ist.

Das Transportbehältnis kann ferner eine Pipettenspitze sein. Eine Pipettenspitze weist eine Einlass-/Auslassöffnung auf, und eine zweite Öffnung. Die zweite Öffnung wird an eine Ansaugeinrichtung gekoppelt, so dass durch einen Unterdruck eine flüssige Probe aus einem Probenbehältnis in das Transportbehältnis gesaugt (pipettiert) werden kann. Die Abgabe der Probe erfolgt durch Ventilieren des Ansaugbereichs, mittels Gravitation und/oder über einen Überdruck, der z. B. über die zweite Öffnung im Pipettenspitze erzeugt wird.

Das Transportbehältnis besteht vorzugsweise teilweise oder vollständig aus Kunststoff. Es ist vorzugsweise ein Verbrauchsartikel, der typischer Weise nur für eine Behandlung oder eine geringe Zahl von Behandlungsschritten der Probe verwendet wird. Das Transportbehältnis kann aber auch teilweise oder vollständig aus einem anderen Material bestehen.

Der Transport einer Probe kann ein Transport der Probe von einer Ausgangsposition in eine Zielposition sein. Die Ausgangsposition kann vorliegen, wenn die Probe in einem ersten Probenbehältnis angeordnet ist und die Zielposition dieser Probe kann ihre Position in einem zweiten Probenbehältnis sein, in das die Probe übertragen wird. Diese Art des Transports wird vorliegend auch als Probentransfer oder Transfer bezeichnet. Ein Probentransfer wird in der Praxis meist durchgeführt, um eine Probe aus einem Vorratsbehältnis, in dem z.B. die Probe gelagert war und/oder das z.B. eine größere Menge der Probe enthalten kann, in ein zweites Probenbehältnis zu übertragen, in dem die Probe weiter behandelt wird. Dieser Transport erfolgt insbesondere programmgesteuert, unter Verwendung mindestens eines Programmparameters.

Das Transportbehältnis ist vorzugsweise mit einer Transporteinrichtung des Laborautomaten verbunden oder verbindbar.

Ein **Probenbehältnis** kann ein Einzelbehältnis sein, in dem nur eine einzige Probe enthalten ist, oder kann ein Mehrfachbehältnis sein, in dem mehrere Einzelbehältnisse miteinander verbunden angeordnet sind.

Ein Einzelbehältnis kann ein offenes Behältnis oder ein verschließbares Behältnis sein. Bei einem verschließbaren Behältnis kann ein Deckelelement, insbesondere eine Verschlusskappe, vorgesehen sein. Das Deckelelement kann fest mit dem Behältnis verbunden sein, z.B. als Klappdeckel oder Klappverschlusskappe, oder kann als separate Komponente verwendet werden.

Vorzugsweise sind bei einem Mehrfachbehältnis die mehreren Einzelbehältnisse in festen Positionen zueinander angeordnet, insbesondere entsprechend den Kreuzungspunkten eines Gittermusters angeordnet. Die vereinfacht die automatisierte Ansteuerung der Positionen und insbesondere die individuellen Adressierung von Proben. Ein Mehrfachbehältnis kann als Plattenelement ausgebildet sein, bei dem die Einzelbehältnisse so verbunden sind, dass sie eine plattenförmige Anordnung bilden. Die Einzelbehältnisse können als Vertiefungen in einer Platte ausgebildet sein oder können über Stegelemente miteinander verbunden sein. Das Plattenelement kann ein Rahmenelement aufweisen, in dem die Einzelbehältnisse gehalten werden. Diese Verbindungen von Komponenten können integrale Verbindungen sein, d.h. stoffschlüssige Verbindungen und/oder durch einen gemeinsamen Spritzgussprozess erzeugte Verbindungen sein, oder können kraftschlüssig (englisch "force-fit") und/oder formschlüssig (englisch "form-fit") erzeugt sein. Das Plattenelement kann insbesondere eine Mikrotiterplatte sein.

Mehrfachbehältnisse können eine Mehrzahl (von 2 bis 10) Einzelbehältnissen aufweisen. Sie können ferner eine Vielzahl (größer 10) aufweisen, typischer Weise 12, 16, 24, 32, 48, 64, 96, 384, 1536 Einzelbehältnisse. Das Mehrfachbehältnis kann insbesondere eine Mikrotiterplatte sein. Eine Mikrotiterplatte kann gemäß einem oder mehreren Industrie-standard(s) ausgebildet sein, insbesondere der Industrienormen ANSI/SBS 1-2004, ANSI/SBS 2-2004, ANSI/SBS 3-2004, ANSI/SBS 4-2004.

Das maximale Probenvolumen, das von einem Transportbehältnis oder einem Probenbehältnis aufnehmbar ist, liegt typischer Weise zwischen 0,01 ml und 100 ml, insbesondere bei 10 — 100 $\mu$l, 100 - 500 $\mu$l, 0,5 - 5 ml, 5-25 ml, 25-50 ml, 50-100 ml, abhängig vom Typ des gewählten Transportbehältnis oder Probengefäßes.

Ein Probenbehältnis kann einen Informationsbereich aufweisen, der Informationen über das Probenbehältnis oder über dessen Inhalt aufweisen kann. Der Informationsbereich kann codierte Information aufweisen, z.B. einen Barcode oder QR-Code, oder einen RFID-Chip, oder eine anders codierte Information. Die Information kann Information zur Identifizierung der Probe und/oder eines Probenbehältnisses aufweisen. Der Laborautomat kann ein Informationsleseeinrichtung aufweisen, um diese Information zu lesen und, vorzugsweise der Steuereinrichtung zur Verfügung zu stellen.

Das Probenbehältnis besteht vorzugsweise teilweise oder vollständig aus Kunststoff. Es ist vorzugsweise ein Verbrauchsartikel, der typischer Weise nur für eine Behandlung oder eine geringe Zahl von Behandlungsschritten der Probe verwendet wird. Das Probenbehältnis kann aber auch teilweise oder vollständig aus einem anderen Material bestehen, z.B. kann ein Aufbewahrungsbehälter, insbesondere eine Aufbewahrungsbox, auch aus Pappe bestehen.

Das Probenbehältnis ist vorzugsweise mit einer Transporteinrichtung des Laborautomaten transportierbar.

Das Laborgerät, insbesondere der Laborautomat, ist vorzugsweise dazu ausgebildet, eine Vielzahl von Proben

nacheinander und/oder parallel zu behandeln. Insbesondere ist das Laborgerät vorzugsweise dazu ausgebildet, eine Vielzahl von Probengefäßen, insbesondere Einzelbehältnisse und/oder Mehrfachbehältnisse, programmgesteuert zu behandeln, insbesondere zu transportieren, zu entleeren und/oder zu befüllen.

Vorzugsweise weist das Laborgerät, insbesondere der Laborautomat, mindestens eine Halteeinrichtung für mindestens ein Probenbehältnis auf, insbesondere einen Halterahmen zum Halten mehrerer Einzelbehältnisse, bezeichnet als "Probengefäßrack". Die Halteeinrichtung ist vorzugsweise transportabel ausgebildet, um innerhalb des Arbeitsbereichs des Laborautomaten, oder zwischen unterschiedlichen Arbeitsbereichen beweglich zu sein. Durch die Halteeinrichtung können eine Mehrzahl oder Vielzahl von Einzelbehältnissen an einer vorbestimmten Position der Halteeinrichtung gehalten werden. Dadurch wird die individuelle programmgesteuerte Ansteuerung oder Adressierung von Probenbehältnissen oder Proben vereinfacht.

Das Laborgerät, insbesondere der Laborautomat, kann mehrere spezialisierte Laborgeräte aufweisen, insbesondere derart, dass es als Laborstraße ausgebildet ist, bei der mehrere Arbeitsbereiche so benachbart angeordnet sind, dass mittels eine Transportvorrichtung eine, mehrere oder eine Vielzahl von Proben, nacheinander und/oder parallel zwischen den Arbeitsbereichen transportiert werden können. Ein Arbeitsbereich einer Laborstraße ist vorzugsweise dazu ausgebildet, dass eine bestimmte Laboraufgabe, betreffend meistens die parallele und/oder sequentielle Behandlung einer Vielzahl von Proben, durchgeführt wird. An einem Arbeitsbereich ist vorzugsweise mindestens ein Laborgerät vorgesehen oder mindestens eine spezielle Behandlungseinrichtung. Durch diese Spezialisierung jedes Arbeitsbereichs wird ein hoher Arbeitsdurchsatz der Laborstrasse erreicht. Zur Durchführung einer solchen bestimmten Aufgabe kann vorgesehen sein, dass in jedem Arbeitsbereich nur eine Art der Behandlung mindestens einer Probe, oder nur wenige Arten der Behandlung, z.B. zwei bis zehn Behandlungsarten durchgeführt werden. An jeder Arbeitsstation kann zur Durchführung einer Behandlung eine Behandlungseinrichtung angeordnet sein, die für ein bestimmtes Laborgerät, wie im Rahmen der Beschreibung der Erfindung beschrieben, charakteristisch ist. Die Transportvorrichtung kann ein Schienensystem aufweisen und/oder eine Robotereinrichtung zum programmgesteuerten Bewegen von Proben bzw. Probenbehältnissen.

Ein Laborgerät, insbesondere Laborautomat, kann mit einem LIMS verbunden oder verbindbar sein. LIMS steht für Labor-Informations- und Management-System. Ein LIMS ist in bekannter Weise ein Softwaresystem, das die Datenverarbeitung im chemischen, physikalischen, biologischen, medizinischen automatischen oder teilautomatischen Labor betrifft. Solche Daten können aus Messungen der Proben stammen, und/oder können die Steuerung der Bearbeitung der Daten betreffen. Ein LIMS dient vorzugsweise der Messwerterfassung und der Messwertauswertung. LIMS wird dazu verwendet, um den Arbeitsdurchsatz in einem Labor zu steigern und/oder die Effizienz der Behandlung von Laborproben zu optimieren.

Ein Werkzeugelement kann z.B. ein Transportkopf für den Fluidtransfer sein, insbesondere ein Pipettierkopf, der einen Verbindungsabschnitt zur Verbindung von einer Pipettenspitze (Einkanalpipettierkopf) oder zur Verbindung von mehreren Pipettenspitzen (Mehrkanalpipettierkopf) mit dem Pipettierkopf aufweisen kann. Über mindestens einen druck- und gasdichten Kanal, der mit dem Pipettierkopf verbunden ist, lässt sich Flüssigkeit in die mindestens eine Pipettenspitze saugen, wenn diese mit dem dem Verbindungsabschnitt verbunden ist. Dieses Pipettieren erfolgt beim Laborautomaten insbesondere programmgesteuert, insbesondere beeinflusst durch mindestens einen Programmparameter. Der Transportkopf kann auch ein Dispensierkopf sein, der mindestens eine Bewegungseinrichtung zur Bewegung eines Kolbens der Dispenserspitze aufweist. Die Bewegung der Bewegungseinrichtung erfolgt beim Laborautomaten insbesondere programmgesteuert, insbesondere beeinflusst durch mindestens einen Programmparameter. Der Transportkopf kann zur Flüssigkeitsdosierung dienen, insbesondere für die Dosierung in unterschiedlichen Bereichen; ein Transportkopf kann zur Dosierung einer flüssigen Probe mit einem Volumen ausgebildet sein, das aus einem für diesen Transportkopf spezifischen Volumenbereich ausgewählt sein kann: z.B. 1 - 50 $\mu$L, oder 20 -300 $\mu$L, oder 50 -1000 $\mu$L, ("l" und "L" stehen jeweils als Abkürzung für Liter). Ein Transportkopf kann als Einkanalkopf ausgebildet sein, bei dem nur eine Probe transportiert wird, oder kann als Mehrkanal, insbesondere Achtkanal- oder 12-Kanal ausgebildet sein, bei dem mehrere Proben parallel bearbeitet bzw. transportiert werden. Es sind vorzugsweise spezifische Transportbehältnisse vorgesehen, die in Abhängigkeit vom jeweiligen Typ von Transportkopf, insbesondere gemäß dem entsprechenden Volumenbereich eingesetzt werden können.

Ein Werkzeugelement kann z.B. ein Transportkopf für den Transport von Gegenständen sein, z.B. eine Trage- und/oder Greifwerkzeug zum Tragen und /oder Greifen eines Gegenstands. Ein Tragewerkzeug kann einen Befestigungsabschnitt zum lösbaren Befestigen des Gegenstands am Tragewerkzeug aufweisen, z.B. durch kraftschlüssiges und/oder formschlüssiges und/oder magnetisches Verbinden des Gegenstands mit dem Tragewerkzeug. Auf diese Weise können innerhalb der Arbeitsfläche oder zwischen mehreren Arbeitsbereichen und/oder Arbeitsflächen.

Ein Werkzeugelement kann ferner eine Behandlungseinheit sein, z.B. zur Durchführung einer thermischen, akustischen, optischen und/oder mechanischen Behandlung mindestens einer Probe.

Eine Behandlungseinheit zur thermischen Behandlung kann eine Temperiereinrichtung aufweisen, um die mindestens eine Probe zeitgleich oder nacheinander, insbesondere innerhalb festgelegter Zeiträume, auf bestimmte Temperaturen einzustellen und/oder diese Temperaturen mit bestimmten Raten zu ändern. Diese thermische Behandlung erfolgt insbesondere programmgesteuert, unter Verwendung mindestens eines Programmparameters. Auf diese Weise lässt sich z.B. an einer, mehrerer oder einer Vielzahl von PCR-Proben eine PCR (Polymerasekettenreaktion) durchführen.

Der Laborautomat kann eine Transporteinrichtung zum Transport mindestens eines beweglichen Kopfabschnitts aufweisen. Der Kopfabschnitt kann ein Transportkopf zum Transportieren mindestens einer Probe und/oder zum Transportieren mindestens eines Werkzeugelements sein. Der Kopfabschnitt kann einen Verbindungsabschnitt zur Verbindung mindestens eines Transportbehältnisses mit der Transporteinrichtung aufweisen. Die **Transporteinrichtung** kann ferner eine programmgesteuerte Bewegungseinrichtung, auch bezeichnet als Robotereinrichtung, aufweisen, um den Kopfabschnitt und/oder den Verbindungsabschnitt und insbesondere gegebenenfalls das mindestens eine Transportbehältnis programmgesteuert zu bewegen, insbesondere gemäß einem durch die Steuereinrichtung, oder eine andere Steuereinrichtung, vorgegebenen Bewegungsablauf. An diesem Kopfabschnitt kann dieser Verbindungsabschnitt vorgesehen sein. Ferner kann an diesem Kopfabschnitt der Verbindungsabschnitt zum Verbinden eines Werkzeugelements mit dem Kopfabschnitt vorgesehen sein.

Die Robotereinrichtung kann eine oder mehrere Antriebseinrichtungen, z.B. elektrische Motoren, aufweisen, um die programmgesteuerte Bewegung von beweglichen Komponenten zu ermöglichen, z.B. eines beweglichen Kopfabschnitts, insbesondere eines Halte- oder Greifwerkzeugs oder eines anderen beweglichen Werkzeugelementes, z. B. einer beweglichen Behandlungseinheit. Die Robotereinrichtung kann einen ein- oder mehrgliedrigen Bewegungsarm aufweisen, mittels dem der Transport des Kopfabschnitts, und insbesondere der Transport von Proben, zwischen verschiedenen, vorzugsweise frei wählbaren, Positionen des Arbeitsbereichs möglich ist. Die Robotereinrichtung kann ferner ein Schienensystem aufweisen, wobei der Kopfabschnitt in diesem Fall an einem beweglichen Gleit- oder Rollelement befestigt ist, dass über dieses Schienensystem an die Positionen bewegbar ist, die durch die Anordnung des Schienensystems vorgegeben sind. Die Bewegungen der Robotereinrichtung sind vorzugsweise programmgesteuert, und insbesondere gesteuert durch mindestens einen Programmparameter.

Das Laborgerät, insbesondere der Laborautomat, kann eine Informationsleseeinrichtung aufweisen, um eine Information über eine Probe und/oder ein Probenbehältnis und/oder eine Behandlungsanweisung für diese Probe und/oder dieses Probenbehältnis zu lesen und, vorzugsweise, der Steuereinrichtung des Laborautomaten zur Verfügung zu stellen.

Das Laborgerät, insbesondere der Laborautomat, weist vorzugsweise mindestens eine Zeitgebereinrichtung und/oder vorzugsweise eine Zeitnehmereinrichtung auf, um die zeitabhängige Behandlung der Proben zu ermöglichen. Die zeitabhängige Behandlung erfolgt vorzugsweise programmgesteuert, und insbesondere gesteuert durch mindestens einen Programmparameter.

In einer bevorzugten Gestaltung des erfindungsgemäßen Laborgerätes, insbesondere Laborautomaten, ist dieses dazu ausgebildet, in Abhängigkeit von der vom Benutzer gewählten Behandlungsart und der vom Benutzer eingegebenen Programmparameter automatisch eine oder mehrere der nachfolgend genannten Komponenten automatisch zur Verwendung bei der programmgesteuerten Behandlung auszuwählen:

- mindestens ein geeignetes Probenbehältnis, insbesondere geeignet zur Aufnahme mehrere Proben, die gemeinsam bearbeitet werden sollen, die z.B. gemischt werden sollen oder zwischen denen eine chemische Reaktion oder biochemische, biologische oder biomedizinische Wechselwirkung auftreten soll;
- mindestens ein geeignetes Transportbehältnis, insbesondere Pipettenspitze und/oder Dispenserspitze;
- mindestens einen geeigneten Transportkopf, mit dem das vorzugsweise automatisch gewählte Transportbehältnis verbindbar ist,
- mindestens ein geeignetes Werkzeugelement, das der Durchführung der gewünschten Behandlung dient.

Vorzugsweise ist das erfindungsgemäße Laborgerät, insbesondere der Laborautomat, dazu ausgebildet, in Abhängigkeit von der vom Benutzer gewählten Behandlungsart und der vom Benutzer eingegebenen Programmparameter automatisch einer oder mehrere der nachfolgend genannten Steuerparameter automatisch zur Verwendung bei der programmgesteuerten Behandlung auszuwählen:

- mindestens ein Zeitraum, zu dem ein bestimmter Arbeitsschritt der Behandlung durchgeführt wird;
- mindestens ein Probenvolumen und/oder Dosiervolumen;
- mindestens eine Arbeitsposition der mindestens einen Arbeitsfläche;
- Bewegungsparameter zur Festlegung des für die gewünschte Behandlung der Probe erforderlichen Bewegungsablauf der Robotereinrichtung des Laborautomaten.

Das Laborgerät weist vorzugsweise eine Benutzerschnittstelleneinrichtung zur manuellen Eingabe von Daten durch einen Benutzer auf, und zur Anzeige von Informationen, insbesondere von in diesen Daten enthaltenen Informationen, wobei die Benutzerschnittstelleneinrichtung eine Anzeigeneinrichtung, insbesondere ein Display, insbesondere ein Touch-Screen-Display aufweist.

Das erfindungsgemäße Laborgerät kann mehrere Behandlungseinrichtungen aufweisen. Die erfindungsgemäße Zugriffssteuerung kann mehreren erfindungsgemäßen Laborgeräten zugeordnet sein, insbesondere mit diesen über zweite Schnittstelleneinrichtung und insbesondere zweite Datenverbindungen verbindbar sein oder verbunden sein. Dadurch kann mit einer Zugriffssteuerung der Zugriff der Benutzer auf mehr als ein Laborgerät oder auf ein Laborgerät mit mehr als einer Behandlungseinrichtung ermöglicht sein.

Die Erfindung betrifft ferner ein **Verfahren** zur gerätegesteuerten Behandlung mindestens einer Laborprobe unter Verwendung mindestens eines Verbrauchsartikels bei einem erfindungsgemäßen Laborgerät, bei dem mittels der Steuereinrichtung des Laborgeräts und mittels Implementierung durch Programmcode folgende Schritte durchgeführt werden:

- Erfassen, insbesondere durch einen Zähl- oder Messvorgang, mindestens eine erste, von dieser Behandlung abhängige Information in ersten Daten, bei mindestens einer durch die Behandlungseinrichtung durchgeführten Behandlung; - Speichern wenigstens einiger der ersten Daten in der Speichereinrichtung; - Übersenden einer Kommunikationsanfrage an die mindestens eine externe Datenverarbeitungseinrichtung und Herstellen einer Datenfernverbindung über die Kommunikationseinrichtung mit einer externen Datenverarbeitungseinrichtung; - Übermittlung wenigstens einiger der ersten Daten über die Kommunikationseinrichtung an eine externe Datenverarbeitungseinrichtung, und Erfassen der ersten Daten zusätzlich in Abhängigkeit von einer Art der Behandlung; - Empfangen von zweiten Daten über die Kommunikationseinrichtung per Push-Technologie; - Verarbeiten der zweiten Daten, indem daraus Push-Informationen gewonnen werden und automatisches Anzeigen dieser aus den zweiten Daten gewonnenen Push-Informationen auf einer Anzeigeneinrichtung einer Benutzerschnittstelleneinrichtung.

Weitere mögliche bevorzugte Ausgestaltungen des erfindungsgemäßen Verfahrens lassen sich aus der Beschreibung des erfindungsgemäßen Laborgeräts und Systems und von deren bevorzugten Ausgestaltungen ableiten.

Weitere bevorzugte Ausgestaltungen des erfindungsgemäßen Laborgeräts, des Systems und des erfindungsgemäßen Verfahrens ergeben sich aus der nachfolgenden Beschreibung der Ausführungsbeispiele in Zusammenhang mit den Figuren und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:

Fig. 1 zeigt ein .Ausführungsbeispiel des erfindungsgemäßen Laborgeräts.

Fig. 2 zeigt ein Ausführungsbeispiel des erfindungsgemäßen Systems.

Fig. 3a zeigt ein Anwendungsbeispiel des erfindungsgemäßen Systems.

Fig. 3b zeigt ein weiteres Anwendungsbeispiel des erfindungsgemäßen Systems.

Fig. 4 zeigt ein Ausführungsbeispiel des erfindungsgemäßen Verfahrens.

Fig. 5 zeigt ein weiteres Ausführungsbeispiel des erfindungsgemäßen Laborgeräts, hier ein Thermocycler.

Fig. 6 zeigt ein weiteres Ausführungsbeispiel des erfindungsgemäßen Laborgeräts, hier ein Labor-Gefiergerät.

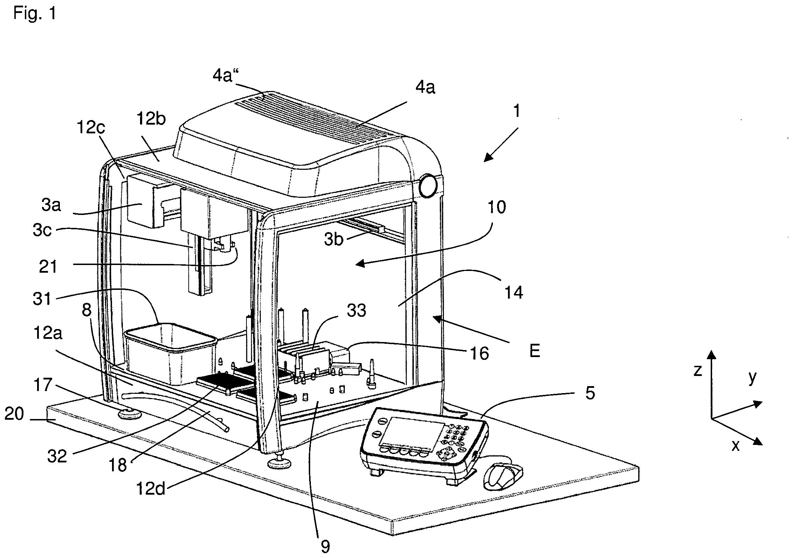

Fig. 1 zeigt das Laborgerät 1, das hier als Laborautomat 1 zur Behandlung von fluiden Proben, und zwar als Pipettierautomat ausgebildet ist. Der Laborautomat 1 dient der programmgesteuerten Behandlung dieser Proben.

Fig. 1 zeigt den Laborautomat 1 für die automatisierte Verarbeitung von flüssigen Proben, insbesondere für die programmgesteuerte Behandlung von flüssigen Proben. Der Laborautomat 1 ist ein Tischgerät ist und mit seinen vier Sockeln 17 auf dem Arbeitstisch 20 angeordnet. Es verfügt über eine elektronische Steuereinrichtung (nicht gezeigt), die geeignet ist, einen Programmcode für die programmgesteuerte Behandlung der flüssigen Proben zu verarbeiten. Die Steuereinrichtung ist in dem Steuerraum angebracht, der durch den Pfeil E bezeichnet ist und der von dem Arbeitsraum 10 durch eine vertikale Wand 14 getrennt. Der Steuerraum beherbergt auch die Spannungsversorgungskomponenten, die die geeignete Versorgungsspannung für die elektrischen Komponenten des Laborautomaten liefern. In die Steuereinrichtung ist vorliegend die Steuereinrichtung der Konfigurationssteuerung integriert.

Der Laborautomat 1 weist einen Behandlungsraum 10 zur Aufnahme der flüssigen zu behandelnden Proben auf, eine programmgesteuert steuerbare Probenbearbeitungseinrichtung 3, zum Durchführen von mindestens einem programmgesteuerten Behandlungsschritt an der mindestens einen Probe, die in dem Bearbeitungsraum angeordnet ist. Der Probenbearbeitungseinrichtung 3 sind die Komponenten 3a, 3b, 3c und 3d der Bewegungseinrichtung zugeordnet.

Der Laborautomat 1 weist ein Gehäuse 12 auf, das eine Vorderseite 12a aufweist, eine gegenüber der Vorderseite angeordnete Rückseite 12f (nicht gezeigt), eine Oberseite 12b, eine gegenüber der Oberseite angeordnete Unterseite 12e (nicht gezeigt) und gegenüberliegenden laterale Seiten 12c und 12d. Die Seiten 12a, 12b und 12c sind im Wesentlichen aus einem Material, das für sichtbares Licht transparent ist.

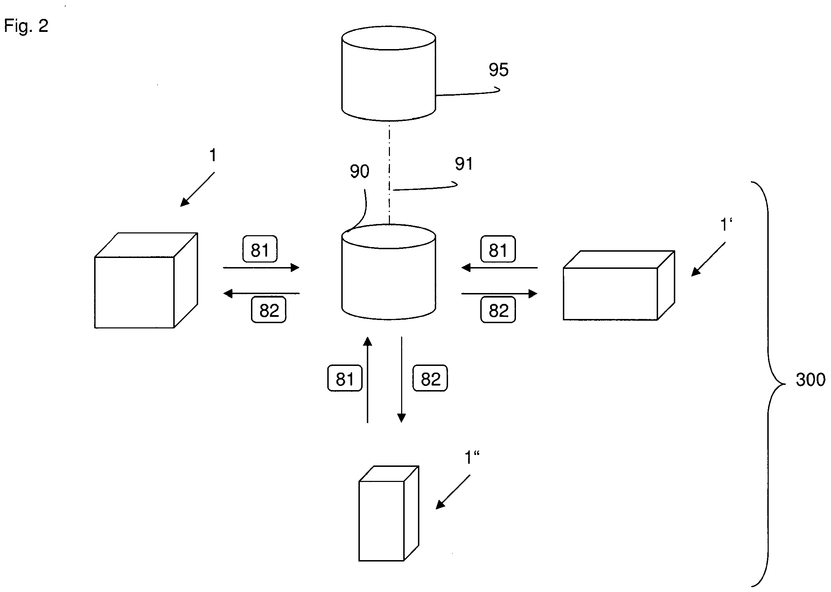

Die Vorderseite 12a, die im Wesentlichen wie eine Tür 12a, nämlich eine Schiebetür 12a ausgebildet ist, kann von Hand bewegt werden und/oder programmgesteuert bewegt werden und kann und sich nach unten im Wesentlichen entlang der z- Achse des kartesischen Koordinatensystems schließen. In Fig. 2a ist die geschlossene Position der Tür 12a gezeigt.

Der Behandlungsraum 10 ist durch die Vorderseite 12a und den beiden Seitenflächen 12c und 12d sowie die Wand 14 und der Arbeitsfläche 8, welche die obere Seite der Bodenplatte 9 bildet, beschränkt. Die Arbeitsfläche 8 stellt sechs Bearbeitungsstationen zur Verfügung. Die Bearbeitungsstationen sind im Wesentlichen ebene Flächen im Bearbeitungsbereich 8. Stifte dienen dazu, Lab-Ware, also Thermorack 33, Mikrotiterplatten 32 und Abfallbehälter 31 an der jeweiligen Bearbeitungsstation auszurichten. Die genaue Positionierung ermöglicht eine präzise robotergesteuerte Adressierung der Probenbehälter, insbesondere der Vertiefungen in den Mikrotiterplatten 32. Eine magnetische Trennvorrichtung 16 ist in der Nähe der Wand 14 angeordnet, wo ein Thermorack 33, d.h. eine temperaturgesteuerte Probengefäßhalterung angeordnet ist. Die magnetische Gabel (nicht gezeigt) der magnetischen Trennvorrichtung 16 fährt von der Seite in entsprechende Aufnahmekanäle des Thermoracks ein, um seitlich an den Laborgefäßen (Probenröhrchen) ihre magnetische Wirkung zu entfalten.

Der Laborautomat 1 weist zwei Dekontaminationseinrichtungen auf, eine elektronisch steuerbaren Luftreinigungsvorrichtung, für die Reinigung der Luft im Behandlungsraum, die elektronisch und digital durch die Steuereinrichtung gesteuert wird und die eine Belüftungsvorrichtung 4a, 4a'' aufweist. Die Belüftungsvorrichtung weist drei Ventilatoren auf (nicht dargestellt), der einen Luftstrom von außerhalb der Vorrichtung zu in den Behandlungsraum transportieren.

Die Steuereinrichtung weist ein Steuerprogramm auf. Der Laborautomat 1 weist eine Probenbearbeitungseinrichtung 3 auf, die eine Bewegungseinrichtung aufweist, mit drei Schienenelementen 3a, 3b, 3c , die Bewegungen entlang der y , x und z- Achse des kartesischen Koordinatensystems entsprechen. Zum Antrieb der Bewegung entlang der gewünschten Richtung sind elektronisch regelbare Linearmotoren vorgesehen. Auf diese Weise kann der Montagekopf 21 in jede gewünschte Position zugänglich in den Bearbeitungsraum 10 bewegt werden. Die Bewegungseinrichtung ist Teil eines Robotersystems der Probenbearbeitungseinrichtung 3. Mit dieser ist der Montagekopf 21 programmgesteuert transportierbar. Mit dem Montagekopf ist ein Werkzeuggerät verbindbar, z. B. ein Pipettierkopf oder ein Greifer. Die im Behandlungsraum angeordneten Bauteile, insbesondere die Probenbearbeitungseinrichtung 3, sind Bestandteil der Behandlungsvorrichtung des Laborautomaten.

Der Laborautomat ist dazu eingerichtet, die Bestückung des Pipettierkopfs mit Pipettierspitzen automatisch und programmgesteuert vorzunehmen. Der Benutzer kann dies durch die Programmierung eines entsprechenden Methodenprogramms, das den Ablauf der Behandlung steuert, zumindest teilweise beeinflussen. Somit wird der Verbrauch an Verbrauchsartikeln, in diesem Fall Pipettenspitzen, programmgesteuert erfolgen, wobei der Benutzer den Verbrauch durch seine Festlegung von Nutzerparametern beeinflussen kann. Der Benutzer kann z.B. gegebenenfalls in Abhängigkeit vom Zwischenschritt der Behandlung festlegen, dass zum Transport von Proben aus einer ersten Mikrotiterplatte in eine zweite Mikrotiterplatte bei jedem Transportvorgang eine frische Pipettenspitze verwendet wird oder dieselbe Pipettenspitze verwendet wird. Der Verbrauch an Verbrauchsartikeln ergibt sich also bei der Durchführung der Behandlung, insbesondere ab dem Zeitpunkt des Startens der Behandlung durch den Benutzer. Auch während der gestarteten Behandlung kann sich der Verbrauchswert noch ändern, etwa wenn nach der automatischen Messung einer Probenkonzentration eine noch größere Menge an Verbrauchsartikeln bei einer nachfolgenden Verdünnungsreihe erforderlich wird.

Während der Behandlung zählt die Steuereinrichtung des Laborautomaten bei jedem programmgesteuerten Abwurf von Pipettenspitzen in den Abfallbehälter 31 die verbrauchten Pipetten, und speichert diese Zahl als erste Daten in einer Speichereinrichtung der Steuereinrichtung. Am Ende der Behandlung steht die Menge an verbrauchten Verbrauchsartikel fest. Diese Menge an verbrauchten Verbrauchsartikeln wird in der Speichereinrichtung als erste Daten bereitgestellt, um über die Kommunikationseinrichtung (nicht gezeigt) des Laborgeräts, die Bestandteil der Steuereinrichtung sein kann, die ersten Daten an eine externe Datenverarbeitungseinrichtung zu übermitteln. Die externe Datenverarbeitungseinrichtung kann eine systemzugehörige externe Datenverarbeitungseinrichtung sein, insbesondere ein Computer im Labor sein, ein externer Computer in einem Intranet des Unternehmens oder ein externer Computer, der nicht Bestandteil des erfindungsgemäßen Systems ist und der über das Internet verbindbar ist.

Der Laborautomat weist eine Benutzerschnittstelleneinrichtung 5 auf, mit der ein Benutzer sich lokal am Laborautomat anmelden kann. Es ist auch möglich, dass er sich über die Benutzerschnittstelleneinrichtung 5 an einem Server 90 eines Intranets anmeldet. Er wird dann von der Zugriffssteuerung identifiziert, die Bestandteil des Servers 90 oder Bestandteil des Laborgeräts sein kann. Es kann somit vom System oder vom Laborgerät der Verbrauch an Verbrauchsartikeln benutzerabhängig erfasst werden, und zweite Daten können dem Benutzer benutzerabhängig an das Laborgerät übersandt werden, insbesondere als benutzerabhängige Produktinformationen.

Fig. 2 zeigt ein Ausführungsbeispiel 300 des erfindungsgemäßen Systems. Das System weist die systemzugehörige externe Datenverarbeitungseinrichtung 90 auf, die ein Server 90 ist, der über ein Intranet mit verschiedenen erfindungsgemäßen Laborgeräten 1, 1′ und 1″ verbunden ist oder verbindbar ist. Der Server 90 weist eine Kommunikationseinrichtung auf, mit der die Datenfernverbindung mit den Laborgeräten herstellbar ist und zudem die Datenfernverbindung 91 mit einem zweiten externen Server 95 realisiert oder herstellbar ist. Die Datenfernverbindung 91 mit dem zweiten externen Server 95 ist über das Internet realisiert. Der zweite externe Server 95 ist extern zum System 300, er ist hier nicht als Bestandteil des Systems 300 vorgesehen. Dies wäre aber ebenfalls möglich.

Jedes Laborgerät 1, 1′, 1″ des Systems 300 ist dazu eingerichtet, bei den durchgeführten Behandlungen erste Daten 81, 81′, 81″ über die verwendeten Verbrauchsartikel zu erfassen, zwischenzuspeichern und an den Server 90 zu übersenden, der diese Daten in seiner Speichereinrichtung speichern und verarbeiten kann. Der Server 90 kann diese Daten zu weiteren ersten Daten über die verwendeten Verbrauchsartikel verarbeiten, insbesondere kann er für mehrere verschiedene oder gleichartige Laborgeräte die ersten Daten über bestimmte Verbrauchsartikel zusammenfassen. Der erste Server 90 kann eine Datenverbindung zu einem zweiten externen Server 95 initiieren. Dieser Vorgang kann auch von einem weiteren externen Server 93 veranlasst werden, der Bestandteil des Systems 300 sein kann und der z.B. im Anwendungsfall des Systems 300 einer Einkaufsabteilung des Unternehmens zugeordnet sein kann, welche das System 300 betreibt.

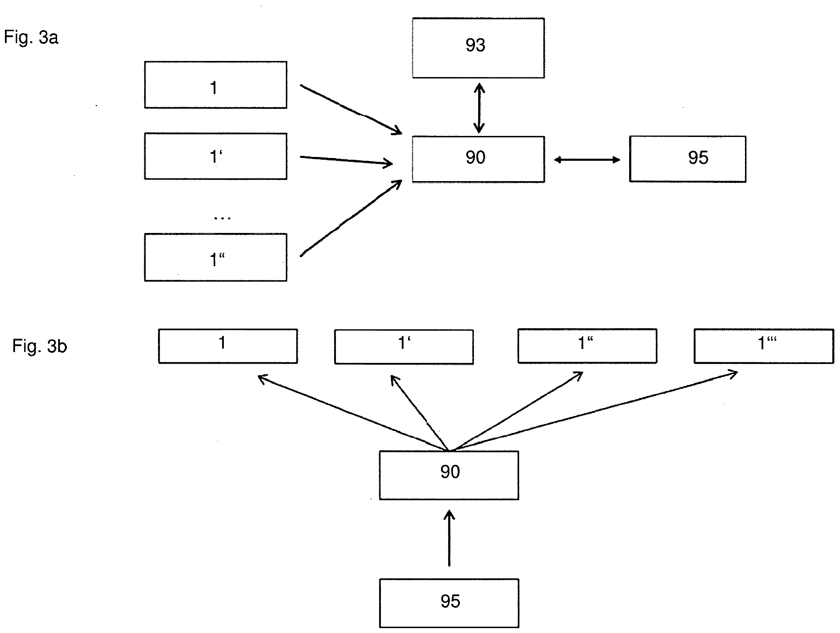

Das Anwendungsbeispiel in Fig. 3a zeigt schematisch die Anwendung des Systems 300 als automatisches Bestellsystem für Verbrauchsartikel.

Vorzugsweise weist hier der externe Server 90 des Systems eine Planungseinrichtung auf, um insbesondere mindestens eine statistische Größe über verwendete Verbrauchsartikel zu bestimmen. Diese statistische Größe kann eine Summe an Verbrauchsartikeln mit Bezug auf eine bestimmte Bezugsgröße sein, z.B. die Art der Behandlung. Die Planungseinrichtung kann Reservierungsdaten einer Reservierungsdatenbank berücksichtigen, die Bestandteil des Servers 90 sein kann, um erste Daten zu bestimmen und insbesondere diese ersten Daten an den Internet-Server 95 zu senden, der in einem Anwendungsbeispiel des Systems 300 einem Anbieter von Informationen und Produkten, insbesondere einem Verkäufer von Verbrauchsmaterial, zugeordnet sein kann. Der zweite externe Server 95 kann als zweite Daten z.B. eine Bestätigung an den Server 90 über den Erhalt der ersten Daten zurücksenden, die der Server 90 dann als zweite Daten empfängt. Zudem kann ein Laborgerät 1, 1′ und/oder 1″ dazu eingerichtet sein, über einen zuvor vereinbarten Informationskanal "push"-Informationen als zweite Daten 82, 82′ und/oder 82″ zu empfangen, die der Internet-Server 95 versendet.

Ein Laborgerät 1, 1′ und/oder 1″ zeigt diese Informationen insbesondere automatisch auf der Anzeigeneinrichtung der Benutzerschnittstelleneinrichtung 5 an, d.h. ohne dass das System, in welchem dieser Informationskanal freigegeben ist, einen weiteren Einfluss hat. Diese Anwendung des Systems 300 ist in Fig. 3b gezeigt. Es werden zweite Daten gemäß einer "push-technology" vom Server 95 jeweils über den geöffneten Informationskanal direkt an das Laborgerät übersandt, um einem jeweiligen Benutzer Informationen anzuzeigen. Neben Informationen über Verbrauchsartikel können auch Wartungsinformationen übersandt werden. Insbesondere können erste Daten auch Informationen über die Abnutzung von Bestandteilen des Laborgeräts enthalten, die als erste Daten an den externen Server 95 weitergeleitet werden, und auf die der Server 95 mit dem Übersenden der zweiten Daten reagiert, insbesondere mittels "push"-Datenübertragung.

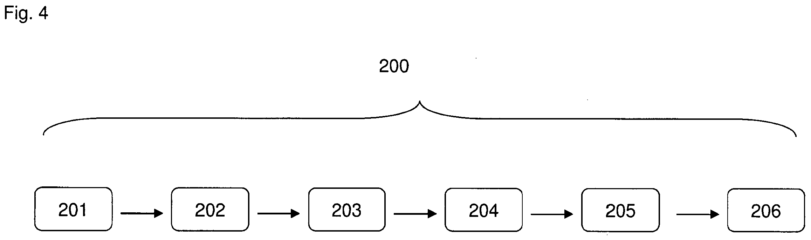

Ein in Figur 4 gezeigtes Ausführungsbeispiel des erfindungsgemäßen Verfahrens ist ein Verfahren zur gerätegesteuerten Behandlung mindestens einer Laborprobe unter Verwendung mindestens eines Verbrauchsartikels bei einem erfindungsgemäßen Laborgerät, bei dem mittels der Steuereinrichtung des Laborgeräts und mittels Implementierung durch Programmcode folgende Schritte durchgeführt werden:

- Erfassen, insbesondere durch einen Zähl- oder Messvorgang, mindestens einer ersten, von dieser Behandlung abhängigen Information in ersten Daten, bei mindestens einer durch die Behandlungseinrichtung durchgeführten Behandlung (Schritt 201);
- Speichern wenigstens einiger der ersten Daten in der Speichereinrichtung (202);
- Übersenden einer Kommunikationsanfrage an die mindestens eine externe Datenverarbeitungseinrichtung (203);
- Herstellen einer Datenfernverbindung über die Kommunikationseinrichtung mit einer externen Datenverarbeitungseinrichtung (204);
- Übermittlung wenigstens einiger der ersten Daten über die Kommunikationseinrichtung an eine externe Datenverarbeitungseinrichtung (204), die insbesondere Bestandteil des erfindungsgemäßen Systems sein kann;
- Empfangen von zweiten Daten über die Kommunikationseinrichtung per "pushtechnology" (205);
- Verarbeiten der zweiten Daten, indem daraus Push-Informationen gewonnen werden und automatisches Anzeigen dieser aus den zweiten Daten gewonnenen Push-Informationen auf einer Anzeigeneinrichtung einer Benutzerschnittstelleneinrichtung (206).

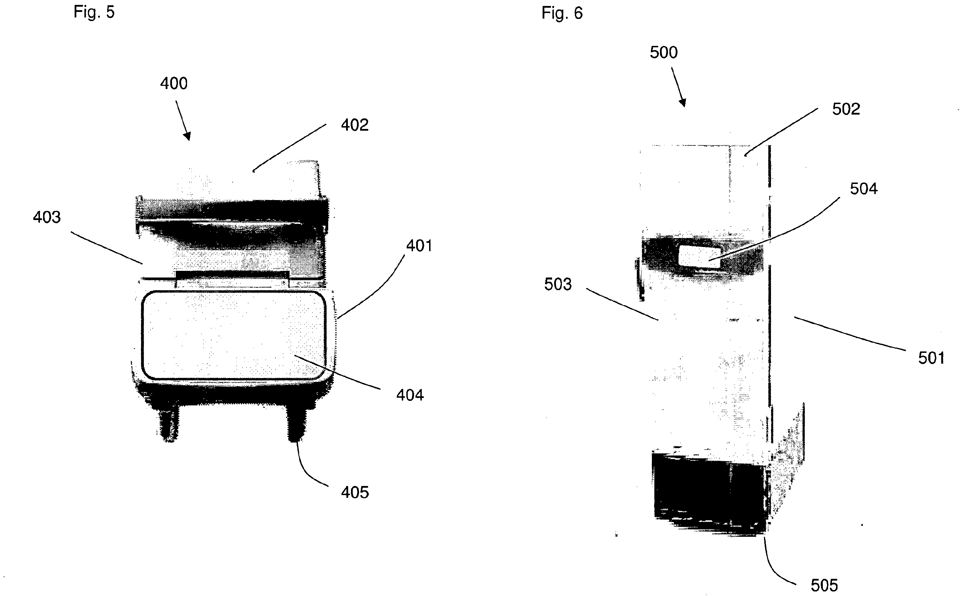

Fig. 5 zeigt ein Laborgerät 400, ein Thermoycler, ausgebildet für die automatisierte Verarbeitung von flüssigen Proben, insbesondere für die programmgesteuerte Temperierung von flüssigen Proben. Das Laborgerät 400 ist ein Tischgerät. Es verfügt über eine integrierte elektronische Steuereinrichtung 406 (nicht gezeigt), die geeignet ist, einen Programmcode für die programmgesteuerte Behandlung der flüssigen Proben zu verarbeiten. Die Steuereinrichtung 406 ist in dem Gehäuse 401 untergebracht. Das Gehäuse beherbergt auch die Spannungsversorgungskomponenten, die die geeignete Versorgungsspannung für die elektrischen Komponenten des Thermocyclers liefern.

Das Laborgerät 400 weist einen Behandlungsraum 403 zur Aufnahme der flüssigen zu behandelnden Proben auf, der mindestens eine programmgesteuert steuerbare Behandlungseinrichtung 408 (nicht gezeigt) aufnehmen kann, zum Durchführen von mindestens einem programmgesteuerten Behandlungsschritt an der mindestens einen Probe, die in der Behandlungseinrichtung angeordnet ist, welche in dem Bearbeitungsraum angeordnet ist. Der Behandlungsraum kann mit einem Deckel 402 verschlossen werden, um eine definierte Temperierungsumgebung herzustellen. In Fig. 5 ist das Laborgerät in geschlossenem Zustand dargestellt.

In die Steuereinrichtung 406 ist die Steuereinrichtung der Zugriffssteuerung integriert. Die Steuereinrichtung 406 weist ein Steuerprogramm auf.

Das Laborgerät weist eine Benutzerschnittstelleneinrichtung 404 auf, mit der ein Benutzer sich lokal am Laborgerät anmelden kann. Das Laborgerät 400 kann erste Daten über verwendetes Verbrauchsmaterial insbesondere in Abhängigkeit vom angemeldeten Benutzer speichern und mittels seiner Kommunikationseinrichtung an die externe Datenverarbeitungseinrichtung übersenden.

Die Steuereinrichtung 406 ist dazu eingerichtet, bei mindestens einer durch die Behandlungseinrichtung durchgeführten Behandlung mindestens eine erste, von dieser Behandlung abhängige Information in ersten Daten zu erfassen, insbesondere durch einen Zählvorgang, und, wenigstens einige der ersten Daten in der Speichereinrichtung zu speichern, wobei das Laborgerät eine Kommunikationseinrichtung zum Herstellen einer Datenverbindung mit mindestens einer externen Datenverarbeitungseinrichtung aufweist, wobei die Kommunikationseinrichtung dazu eingerichtet ist, diese wenigstens einigen ersten Daten an die -insbesondere zweite- externe Datenverarbeitungseinrichtung zu übermitteln. Der Zählvorgang kann das Entnehmen und/oder Einsetzen eines Mehrfachprobenbehältnisses, insbesondere Mikrotiterplatte, aus dem / in den Behandlungsraum 403 zählen. Jeder gezählte Vorgang kann dann als Verbrauch eines Mehrfachprobenbehältnisses gewertet werden, und diese Information kann gesammelt werden und/oder als erste Daten an die -insbesondere zweite- externe Kommunikationseinrichtung übersandt werden.

Fig. 6 zeigt in einem weiteren Ausführungsbeispiel der Erfindung ein Laborgerät 500, ein Labor-Gefriergerät, für die Lagerung von Laborproben, insbesondere bei Temperaturen unter -50°C. Das Laborgerät 500 ist ein Tischgerät. Es verfügt über eine integrierte elektronische Steuereinrichtung 506 (nicht gezeigt), die geeignet ist, die Temperatur in dem erforderten Bereich einzustellen, zu regeln und zu überwachen. Die Steuereinrichtung 506 ist in dem Gehäuse 501 untergebracht. Das Gehäuse beherbergt auch die Spannungsversorgungskomponenten, die die geeignete Versorgungsspannung für die elektrischen Komponenten des Labor-Gefriergeräts liefern.

Das Laborgerät 500 weist einen Behandlungsraum 503 zur Aufnahme der aufzubewahrenden Proben auf, der mindestens eine programmgesteuert steuerbare Behandlungseinrichtung 408 (nicht gezeigt) umfasst, der im Falle des Labor-Gefriergerätes einen abgeschlossenen Bereich mit einer definiert einstellbaren Temperatur entspricht. Der programmgesteuerte Behandlungsschritt entspricht dabei dem Einfrieren der mindestens einen Probe, die in der Behandlungseinrichtung angeordnet ist, welche in dem Bearbeitungsraum angeordnet ist. Der Behandlungsraum kann mit einer Tür 502 verschlossen werden, um eine definierte Temperierungsumgebung herzustellen. Im Falle von mehr als einer Behandlungseinrichtung sind auch mehrere Türen, ggf. angeordnet hinter der gemeinsamen Tür 502 denkbar. In Fig. 5 ist das Laborgerät in geschlossenem Zustand dargestellt.

In die Steuereinrichtung 506 des Laborgeräts 500 ist die Steuereinrichtung der Zugriffssteuerung integriert. Die Steuereinrichtung 406 weist ein Steuerprogramm auf.

Das Laborgerät weist eine Benutzerschnittstelleneinrichtung 504 auf, mit der ein Benutzer sich lokal am Laborgerät anmelden kann.

Die Steuereinrichtung 506 ist dazu eingerichtet, bei mindestens einer durch die Behandlungseinrichtung durchgeführten Behandlung mindestens eine erste, von dieser Behandlung abhängige Information in ersten Daten zu erfassen, insbesondere durch einen Zählvorgang, und, wenigstens einige der ersten Daten in der Speichereinrichtung zu speichern, wobei das Laborgerät eine Kommunikationseinrichtung zum Herstellen einer Datenverbindung mit mindestens einer externen Datenverarbeitungseinrichtung aufweist, wobei die Kommunikationseinrichtung dazu eingerichtet ist, diese wenigstens einigen ersten Daten an die -insbesondere zweite- externe Datenverarbeitungseinrichtung zu übermitteln. Der Zählvorgang kann das Entnehmen und/oder Einsetzen eines Mehrfachprobenbehältnisses, insbesondere Mikrotiterplatte, aus dem / in den Behandlungsraum 503 zählen. Jeder gezählte Vorgang kann dann als Verbrauch eines Mehrfachprobenbehältnisses gewertet werden, und diese Information kann gesammelt werden und/oder als erste Daten an die -insbesondere zweite- externe Kommunikationseinrichtung übersandt werden.

# EP 2 857 844 B1

**Patentansprüche**

1. Laborgerät (1; 1′; 1″; 400; 500) zur gerätegesteuerten Behandlung mindestens einer Laborprobe, aufweisend:

   - mindestens eine Behandlungseinrichtung (3; 408), die dazu eingerichtet ist, die Behandlung der mindestens einen Laborprobe unter Verwendung eines Pipettiervorgangs und einer Menge wenigstens eines Verbrauchsartikels (32) durchzuführen, der ein Proben- oder Transportbehältnis ist", und
   - eine Steuereinrichtung (2; 406; 506) zur Steuerung der Behandlung, die eine Datenverarbeitungseinrichtung und eine Speichereinrichtung zum Speichern von Daten aufweist,

   wobei die Steuereinrichtung (2; 406; 506) dazu eingerichtet ist,

   - bei mindestens einer durch eine Behandlungseinrichtung (3; 408) durchgeführten Behandlung mindestens eine erste, von dieser Behandlung abhängige Information über die Menge der Verbrauchsartikel und/oder die Anzahl der Verbrauchsartikel und/oder deren Verwendung und/oder deren Verbrauch in ersten Daten (81) zu erfassen, insbesondere durch einen Zähl-, Mess- oder Berechnungsvorgang, und die ersten Daten zusätzlich in Abhängigkeit von einer Art der Behandlung zu erfassen, welche diesen Pipettiervorgang und diesen mindestens einen Verbrauchsartikel verwendet, und
   - wenigstens einige der ersten Daten in der Speichereinrichtung zu speichern,

   wobei das Laborgerät eine Kommunikationseinrichtung zum Herstellen einer Datenverbindung mit mindestens einer externen Datenverarbeitungseinrichtung (95) aufweist, wobei die Kommunikationseinrichtung dazu eingerichtet ist, diese wenigstens einigen ersten Daten an die externe Datenverarbeitungseinrichtung zu übermitteln, **dadurch gekennzeichnet, dass**

   die Kommunikationseinrichtung dazu eingerichtet ist, eine Kommunikationsanfrage zu empfangen, die von der externen Datenverarbeitungseinrichtung (95) initiiert wurde und an die Kommunikationseinrichtung gesendet wurde,
   wobei die Kommunikationseinrichtung dazu eingerichtet ist, aufgrund der von der externen Datenverarbeitungseinrichtung (95) initiierten Kommunikationsanfrage eine Datenfernverbindung zu erlauben, die die externe Datenverarbeitungseinrichtung mit der Kommunikationseinrichtung herstellt,
   wobei die von der externen Datenverarbeitungseinrichtung (95) initiierte Datenfernverbindung eine Internet-basierte Form der Kommunikation zwischen der externen Datenverarbeitungseinrichtung und der Kommunikationseinrichtung ist, die gemäß einer vorausgegangenen Vereinbarung zwischen der externen Datenverarbeitungseinrichtung und der Kommunikationseinrichtung einen Informationskanal bildet, über den die externe Datenverarbeitungseinrichtung unter Verwendung von Push-Technologie Push-Informationen als zweite Daten (82) an die Kommunikationseinrichtung sendet,
   wobei die Steuereinrichtung (2; 406; 506) mittels Implementierung durch Programmcode dazu eingerichtet ist, die zweiten Daten automatisch zu verarbeiten, indem daraus Push-Informationen gewonnen und diese Push-Informationen dem Benutzer automatisch auf einer Anzeigeneinrichtung einer Benutzerschnittstelleneinrichtung (5) des Laborgeräts angezeigt werden.

2. Laborgerät gemäß Anspruch 1, wobei die Steuereinrichtung (2; 406; 506) dazu eingerichtet ist, die Kommunikationsanfrage der externen Datenverarbeitungseinrichtung (95) in Abhängigkeit von den ersten Daten und/oder in Abhängigkeit von anderen Bedingungen zu steuern, insbesondere die Datenfernverbindung zu erlauben.

3. Laborgerät gemäß einem der Ansprüche 1 bis 2, das eine Zugriffssteuerung (100) aufweist, die dazu ausgebildet ist, den Zugriff, insbesondere die Kommunikationsanfrage, der externen Datenverarbeitungseinrichtung (95) zu steuern.

4. Laborgerät gemäß einem der vorherigen Ansprüche,
   wobei die Steuereinrichtung (2; 406; 506) dazu eingerichtet ist, bei mindestens einer Behandlung mindestens eine erste Information über die Größe X in Abhängigkeit von Y zu erfassen, insbesondere durch Zählen oder Messen, wobei die Werte X und Y mindestens einen Verbrauchsartikel Y der Menge X charakterisieren, und diese mindestens eine erste Information in den ersten Daten zu erfassen.

5. Laborgerät gemäß einem der vorherigen Ansprüche, das eine Zugriffssteuerung aufweist, die dazu eingerichtet ist, den Zugriff mindestens eines über die Benutzerschnittstelleneinrichtung zugreifenden Benutzers zu steuern, insbesondere zu erlauben, und diesen Benutzer dabei zu identifizieren,
wobei die Steuereinrichtung dazu ausgebildet ist, die ersten Daten in Abhängigkeit vom identifizierten Benutzer zu erfassen und/oder die Information über den identifizierten Benutzer als Bestandteil der ersten Daten zu erfassen.

6. Laborgerät gemäß einem der vorherigen Ansprüche, wobei die Steuereinrichtung (2; 406; 506) dazu ausgebildet ist, die ersten Daten in Abhängigkeit von mindestens einer geplanten Behandlung zu ermitteln, insbesondere zu berechnen, insbesondere in Abhängigkeit von mindestens einer gemäß den Reservierungsdaten einer Reservierungsdatenbank geplanten mindestens einen Behandlung.

7. Laborgerät gemäß einem der vorherigen Ansprüche, das eine Planungseinrichtung zur Planung eines zu erwartenden Verbrauchs an Verbrauchsartikeln aufweist, wobei die Steuereinrichtung (2; 406; 506) dazu eingerichtet ist, diesen zu erwartenden Bedarf an Verbrauchsartikeln als erste Daten an die externe Datenverarbeitungseinrichtung zu übersenden.

8. System (300), aufweisend

    - mindestens ein Laborgerät (1; 1′; 1″) gemäß einem der vorangehenden Ansprüche
    - mindestens eine systemzugehörige externe Datenverarbeitungseinrichtung (95), wobei die Kommunikationseinrichtung des mindestens einen Laborgeräts zum Herstellen einer Datenverbindung mit der mindestens einen systemzugehörigen externen Datenverarbeitungseinrichtung eingerichtet ist, um diese wenigstens einigen ersten Daten an die systemzugehörige externe Datenverarbeitungseinrichtung zu übermitteln.

9. System gemäß Anspruch 8, wobei die mindestens eine, systemzugehörige externe Datenverarbeitungseinrichtung (95) eine Speichereinrichtung aufweist und dazu eingerichtet ist, die ersten Daten und/oder zweiten Daten zu verarbeiten, insbesondere in dieser Speichereinrichtung zu speichern.

10. System gemäß Anspruch 8 oder 9, wobei die systemzugehörige externe Datenverarbeitungseinrichtung (95) eine Kommunikationseinrichtung zum Herstellen einer Datenfernverbindung mit mindestens einer zweiten externen Datenverarbeitungseinrichtung aufweist, um mittels dieser Datenfernverbindung diese wenigstens einigen ersten Daten an die zweite externe Datenverarbeitungseinrichtung zu übermitteln.

11. Verfahren (200) zur gerätegesteuerten Behandlung mindestens einer Laborprobe unter Verwendung mindestens eines Verbrauchsartikels und eines Laborgeräts gemäß Anspruch 1, bei dem mittels der Steuereinrichtung des Laborgeräts und mittels Implementierung durch Programmcode folgende Schritte durchgeführt werden:

    - Erfassen, insbesondere durch einen Zähl- oder Messvorgang, mindestens einer ersten, von dieser Behandlung abhängigen Information in ersten Daten, bei mindestens einer durch die Behandlungseinrichtung durchgeführten Behandlung; (201)
    - Speichern wenigstens einiger der ersten Daten in der Speichereinrichtung; (202)
    - Übersenden einer Kommunikationsanfrage an die mindestens eine externe Datenverarbeitungseinrichtung; (203)
    - Herstellen einer Datenfernverbindung über die Kommunikationseinrichtung mit einer externen Datenverarbeitungseinrichtung; (204)
    - Übermittlung wenigstens einiger der ersten Daten über die Kommunikationseinrichtung an die externe Datenverarbeitungseinrichtung; (204)
    - Erfassen der ersten Daten zusätzlich in Abhängigkeit von einer Art der Behandlung;
    - Empfangen von zweiten Daten über die Kommunikationseinrichtung per Push-Technologie (205);
    - Verarbeiten der zweiten Daten, indem daraus Push-Informationen gewonnen und automatisches Anzeigen der aus den zweiten Daten gewonnenen Push-Informationen auf einer Anzeigeneinrichtung einer Benutzerschnittstelleneinrichtung. (206)

**Claims**

1. Laboratory apparatus (1; 1′; 1″; 400; 500) for the apparatus-controlled treatment of at least one laboratory sample, comprising:

    - at least one treatment device (3; 408) adapted to carry out the treatment of said at least one laboratory sample using a pipetting operation and a quantity of at least one consumable article (32) which is a sample or transport container, and
- one control device (2; 406; 506) for controlling the treatment, comprising a data processing device and a memory device for storing data,

wherein the control device (2; 406; 506) is adapted to,

- in the case of at least one treatment carried out by a treatment device (3; 408), to acquire at least one first item of information, dependent on this treatment, relating to the quantity of consumable articles and/or the number of consumable articles and/or their use and/or their consumption, in first data (81), particularly by a counting, measuring or calculating operation, and additionally acquiring the first data depending on the type of treatment using this pipetting operation and this at least one consumable article, and
- storing at least some of the first data in the memory device,

wherein the laboratory apparatus comprises a communication device for establishing a data connection with at least one external data processing device (95), the communication device being arranged to transmit said at least some first data to the external data processing device,
**characterized in that** the communication device is adapted to receive a communication request initiated by the external data processing device (95) and sent to the communication device,

wherein the communication device is arranged to permit, based on the communication request initiated by the external data processing device (95), a remote data connection to be established by the external data processing device with the communication device,
wherein the external data processing device (95) initiated by the external data processing device is an Internet-based form of communication between the external data processing device and the communication device, which, according to a prior agreement between the external data processing device and the communication device, forms an information channel through which the external data processing device sends push information as second data (82) to the communication device using push technology,
wherein the control device (2; 406; 506) is arranged, by implementation by program code, to automatically process the second data by extracting push information therefrom and automatically displaying that push information to the user on a display device of a user interface device (5) of the laboratory apparatus.

2. Laboratory apparatus according to claim 1, wherein the control device (2; 406; 506) is arranged to control the communication request of the external data processing device (95) in dependence on the first data and/or in dependence on other conditions, in particular to enable the remote data connection.

3. Laboratory apparatus according to any one of claims 1 to 2, comprising an access control (100) which is designed to control the access, in particular the communication request, of the external data processing device (95).

4. Laboratory apparatus according to any one of the preceding claims,
wherein the control device (2; 406; 506) is set up to detect at least a first piece of information about the variable X as a function of Y during at least one treatment, in particular by counting or measuring, wherein the values X and Y characterize at least one consumable article Y of quantity X, and to record this at least one first information in the first data.

5. Laboratory apparatus according to any one of the preceding claims, comprising an access control which is set up to control, in particular to enable, the access of at least one user accessing via the user interface device and identify that user in the process, wherein the control device is adapted to acquire the first data in dependence on the identified user and/or to acquire the information about the identified user as part of the first data.

6. Laboratory apparatus according to any one of the preceding claims, wherein the control device (2; 406; 506) is designed to determine, in particular to calculate, the first data as a function of at least one planned treatment, in particular as a function of at least one treatment planned according to the reservation data of a reservation database.

7. Laboratory apparatus according to any one of the preceding claims, comprising a planning device for planning an expected consumption of consumable articles, wherein the control device (2; 406; 506) is arranged to transmit this expected demand for consumable articles as first data to the external data processing device.

**8.** System (300) comprising

- at least one laboratory apparatus (1; 1′; 1″) according to any one of the preceding - claims,
- at least one system-associated external data processing device (95), wherein the communication device of the at least one laboratory apparatus is arranged to establish a data connection with the at least one system-associated external data processing device in order to transmit said at least some first data to the system-associated external data processing device.

**9.** System according to claim 8, wherein the at least one system-associated external data processing device (95) comprises a memory device and is set up to process the first data and/or second data, in particular to store them in this memory device.

**10.** System according to claim 8 or 9, wherein the system-associated external data processing device (95) comprises a communication device for establishing a remote data connection with at least a second external data processing device in order to transmit, by device of said remote data connection, said at least some first data to said second external data processing device.

**11.** Method (200) for the device-controlled treatment of at least one laboratory sample using at least one consumable article and a laboratory device according to claim 1, in which, by device of the control device of the laboratory device and with the following steps which are carried out by device of implementation through program code:

- acquiring, in particular by a counting or measuring operation, at least one first item of information dependent on this treatment, in first data, during at least one treatment carried out by the treatment device; (201)
- storing at least some of the first data in the storage device; (202)
- sending a communication request to the at least one external data processing device; (203)
- establishing a remote data link via the communication device with an external data processing device; (204)
- transmission of at least some of the first data via the communication device to the external data processing device; (204)
- acquiring the first data additionally depending on a type of treatment,
- receiving second data via the communication device using push technology (205);
- processing the second data by obtaining push information therefrom and automatically displaying the push information obtained from the second data on a display device of a user interface device. (206)

**Revendications**

**1.** Appareil de laboratoire (1 ; 1′ ; 1″ ; 400 ; 500) pour la manipulation commandée par appareil d'au moins un échantillon de laboratoire, présentant :

- au moins un dispositif de manipulation (3 ; 408), qui est conçu pour effectuer la manipulation du au moins un échantillon de laboratoire au moyen d'un processus de pipetage et d'une quantité d'au moins un consommable (32), qui est un récipient d'échantillon ou de transport, et
- un dispositif de commande (2 ; 406 ; 506) pour la commande de la manipulation, qui présente un dispositif de traitement de données et un dispositif mémoire pour la mise en mémoire de données,

dans lequel le dispositif de commande (2 ; 406 ; 506) est conçu

- lors d'au moins une manipulation effectuée par un dispositif de manipulation (3 ; 408), pour détecter au moins une première information dépendant de cette manipulation concernant la quantité des consommables et/ou le nombre des consommables et/ou leur utilisation et/ou leur consommation dans des premières données (81), en particulier par un processus de comptage, mesure ou calcul, et pour détecter les premières données en plus en fonction d'un type de la manipulation, lequel utilise ce processus de pipetage et ce au moins au consommable, et
- pour mettre en mémoire au moins certaines des premières données dans le dispositif mémoire,

dans lequel l'appareil de laboratoire présente un dispositif de communication pour l'établissement d'une liaison de données avec au moins un dispositif de traitement de données externe (95), dans lequel le dispositif de communication est conçu pour transmettre ces au moins certaines premières données au dispositif de traitement de données externe,
**caractérisé en ce que**

le dispositif de communication est conçu pour recevoir une demande de communication, qui a été initiée par le dispositif de traitement de données externe (95) et été envoyé au dispositif de communication, dans lequel le dispositif de communication est conçu pour permettre, sur la base de la demande de communication initiée par le dispositif de traitement de données externe (95), une liaison de données longue distance, que le dispositif de traitement de données externe établit avec le dispositif de communication, dans lequel la liaison de données longue distance initiée par le dispositif de traitement de données externe (95) est une forme basée sur Internet de la communication entre le dispositif de traitement de données externe et le dispositif de communication, qui forme conformément à un accord précédent entre le dispositif de traitement de données externe et le dispositif de communication un canal d'information, par l'intermédiaire duquel le dispositif de traitement de données externe envoie des informations de poussée en tant que deuxièmes données (82) au dispositif de communication au moyen d'une technologie de poussée, dans lequel le dispositif de commande (2 ; 406 ; 506) est conçu au moyen d'une implémentation par un code de programme pour traiter les deuxièmes données de manière automatique, du fait que les informations de poussée sont obtenues à partir de celles-ci et ces informations de poussée sont affichées à l'utilisateur de manière automatique sur un dispositif d'affichage d'un dispositif d'interface utilisateur (5) de l'appareil de laboratoire.

2. Appareil de laboratoire selon la revendication 1, dans lequel le dispositif de commande (2 ; 406 ; 506) est conçu pour commander la demande de communication du dispositif de traitement de données externe (95) en fonction des premières données et/ou en fonction d'autres conditions, en particulier pour permettre la liaison de données longue distance.

3. Appareil de laboratoire selon l'une quelconque des revendications 1 à 2, qui présente une commande d'accès (100), qui est réalisée pour commander l'accès, en particulier la demande de communication, du dispositif de traitement de données externe (95).

4. Appareil de laboratoire selon l'une quelconque des revendications précédentes, dans lequel le dispositif de commande (2 ; 406 ; 506) est conçu pour détecter lors d'au moins une manipulation au moins une première information concernant la taille X en fonction de Y, en particulier par comptage ou mesure, dans lequel les valeurs X et Y caractérisent au moins un consommable Y de la quantité X, et pour détecter cette au moins une première information dans les premières données.

5. Appareil de laboratoire selon l'une quelconque des revendications précédentes, qui présente une commande d'accès, qui est conçue pour commander, en particulier pour permettre, l'accès au moins d'un utilisateur accédant par l'intermédiaire du dispositif d'interface utilisateur, et ce faisant pour identifier cet utilisateur, dans lequel le dispositif de commande est réalisé pour détecter les premières données en fonction de l'utilisateur identifié et/ou pour détecter l'information concernant l'utilisateur identifié en tant que partie des premières données.

6. Appareil de laboratoire selon l'une quelconque des revendications précédentes, dans lequel le dispositif de commande (2 ; 406 ; 506) est réalisé pour déterminer, en particulier pour calculer les premières données en fonction d'au moins une manipulation planifiée, en particulier en fonction d'au moins une manipulation planifiée conformément aux données de réservation d'une banque de données de réservation.

7. Appareil de laboratoire selon l'une quelconque des revendications précédentes, qui présente un dispositif de planification pour la planification d'une consommation attendue de consommables, dans lequel le dispositif de commande (2 ; 406 ; 506) est conçu pour envoyer ce besoin attendu de consommables en tant que premières données au dispositif de traitement de données externe.

8. Système (300), présentant

- au moins un appareil de laboratoire (1 ; 1′ ; 1″) selon l'une quelconque des revendications précédentes,
- au moins un dispositif de traitement de données externe (95) faisant partie du système, dans lequel le dispositif de communication du au moins un appareil de laboratoire est conçu pour établir une liaison de données avec le au moins un dispositif de traitement de données externe faisant partie du système, afin de transmettre ces au moins certaines premières données au dispositif de traitement de données externe faisant partie du système.

**9.** Système selon la revendication 8, dans lequel le au moins un dispositif de traitement de données externe (95) faisant partie du système présente un dispositif mémoire et est conçu pour traiter les premières données et/ou deuxièmes données, en particulier pour les mettre en mémoire dans ce dispositif mémoire.

**10.** Système selon la revendication 8 ou 9, dans lequel le dispositif de traitement de données externe (95) faisant partie du système présente un dispositif de communication pour l'établissement d'une liaison de données longue distance avec au moins un deuxième dispositif de traitement de données externe, afin de transmettre ces au moins certaines premières données au dispositif de traitement de données externe au moyen de cette liaison de données longue distance.

**11.** Procédé (200) pour la manipulation commandée par appareil d'au moins un échantillon de laboratoire au moyen d'au moins un consommable et d'un appareil de laboratoire selon la revendication 1, selon lequel les étapes suivantes sont effectuées au moyen du dispositif de commande de l'appareil de laboratoire et au moyen d'une implémentation par un code de programme :

- la détection, en particulier par un processus de comptage ou de mesure, d'au moins une première information dépendant de cette manipulation dans des premières données, lors d'au moins une manipulation effectuée par le dispositif de manipulation ; (201)
- la mise en mémoire d'au moins certaines des premières données dans le dispositif mémoire ; (202)
- l'envoi d'une demande de communication à l'au moins un dispositif de traitement de données externe ; (203)
- l'établissement d'une liaison de données longue distance par l'intermédiaire du dispositif de communication avec un dispositif de traitement de données externe ; (204)
- la transmission d'au moins certaines des premières données par l'intermédiaire du dispositif de communication au dispositif de traitement de données externe ; (204)
- la détection des premières données en plus en fonction d'un type de la manipulation ;
- la réception de deuxièmes données par l'intermédiaire du dispositif de communication par technologie de poussée (205) ;
- le traitement des deuxièmes données, du fait des informations de poussée obtenues à partir de celles-ci et de l'affichage automatique des informations de poussée obtenues à partir des deuxièmes données sur un dispositif d'affichage d'un dispositif d'interface utilisateur (206).

Fig. 1

Fig. 2

Fig. 3a

Fig. 3b

Fig. 4

200

201 → 202 → 203 → 204 → 205 → 206

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2011246215 A
- US 2013159135 A
- US 2005014285 A
- JP 2006164295 A